# DEMANDE DE BREVET EUROPEEN

(11) **EP 1 233 015 A1**
(43) Date de publication de la demande: **21.08.2002**
(21) Numéro de dépôt: 02006541.3
(22) Date de dépôt: 27.02.1995
(51) Int. Cl.: C07D 233/84, C07D 233/68, C07D 233/90, A61K 31/415

(54) **Utilisation de dérivés de l'imidazole au traitement d'affections impliquant les récepteurs AT1 et AT2 de l'angiotensine, certains de ces produits, leur préparation, leur application à titre de médicaments et les compositions pharmaceutiques les renfermant**

(30) Priorité: 04.03.1994 FR 9402518
(62) Demande divisionnaire de: 95910603.0
(71) Demandeur: Aventis Pharma S.A., 92160 Antony (FR)
(72) Inventeur: Corbier, Alain, 91370 Verrieres le Buisson (FR); Fortin, Michel, 75018 Paris (FR); Heckmann, Bertrand, 91440 Bures sur Yvette (FR); Deprez, Pierre, 94320 Thiais (FR); Guillaume, Jacques, 93190 Livry Gargan (FR)
(74) Mandataire: Vieillefosse, Jean-Claude

(57) **Abrégé**

L'invention a pour objet l'utilisation pour la préparation de compositions pharmaceutiques destinées au traitement d'affections résultant d'une stimulation anormale du récepteur AT₁ de l'angiotensine II, de produits de formule (I) : 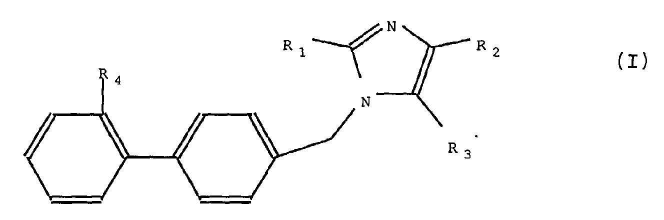 dans laquelle R₁ représente notamment alkyl, alkylthio ou alcoxy, R₂ représente notamment halogène, -S-R, -O-R ou -C(OH)(R)-COOH avec R représentent alkyle ou alkényle, R₃ représente notamment carboxy, acyle, halogène, alkyle, alkényle ou alkylthio
et R₄ représente notamment -(CH₂)ₘ₁-COOR₁₄, -(CH₂)ₘ₁-CONHR₁₄, -(CH₂)ₘ₁-CN, -SO₂-NH-SO₂-R₁₄, -NH-SO₂-R₁₄, -PO₃R₁₄, -NH-SO₂-CF₃ avec m1 représentent 0 à 4, et R₁₄ représentant hydrogène, alkyle ou alkényle.

## Description

La présente invention concerne une nouvelle utilisation de dérivés de l'imidazole au traitement d'affections impliquant les récepteurs AT₁ et AT₂ de l'Angiotensine, certains de ces produits, leur préparation, leur application à titre de médicaments et les compositions pharmaceutiques les renfermant.

Le document EP-A-560177 décrit des dérivés de l'imidazole comme antagonistes de récepteur de l'Angiotensine II.

Les documents EP-A-577023 et EP-A-577025 décrivent des dérivés hétérocycliques comme antagonistes de récepteur de l'Angiotensine II.

Les documents EP-A-505098 et EP-A-479479 décrivent des dérivés de l'imidazole comme antagonistes de l'Angiotensine II.

Le document EP-A-324377 décrit des dérivés de l'imidazole comme antagonistes de récepteur de l'Angiotensine II.

L'Article Bioorg. Med. Chem. Lett. 1994, Vol.4(1), 69-74 décrit des dérivés de l'imidazole comme antagonistes de l'Angiotensine II.

L'Angiotensine II est connue pour être une hormone circulante pouvant agir également comme un neuropeptide au niveau du système nerveux central ainsi que l'indiquent notamment les demandes de brevets européennes EP 0465368 et EP 0503162, ou encore internationale WO 91/14367.

Il a récemment été mis en évidence qu'il existe en fait deux sous-types de récepteurs à l'Angiotensine II :
le récepteur AT₁ et le récepteur AT₂.

La recherche s'était d'abord intéressée à la mise en évidence d'antagonistes au récepteur AT₁ comme substances à activité anti-hypertensives.

On vient de montrer que les produits de formule (I) de la présente invention ont une affinité non seulement pour le récepteur AT₁ mais également pour le récepteur AT₂.

Les produits de formule (I) de la présente invention peuvent ainsi tout particulièrement faire l'objet d'une nouvelle utilisation pour le traitement d'affections résultant d'une stimulation anormale des récepteurs AT₁ et AT₂ de l'Angiotensine II.

La présente invention a ainsi pour objet la nouvelle utilisation pour la préparation de compositions pharmaceutiques destinées au traitement d'affections résultant d'une stimulation anormale des récepteurs AT₁ et AT₂ de l'angio-tensine II, de produits de formule (I) : 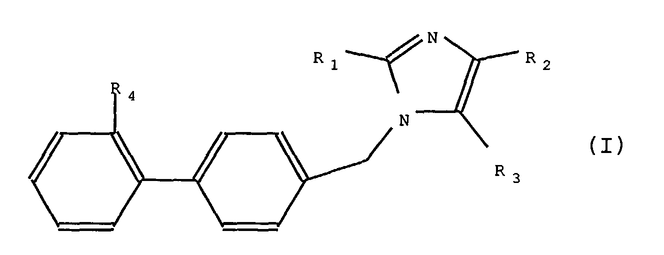 dans laquelle
R₁ représente un radical alkyl, alkylthio et alcoxy, linéaire ou ramifié renfermant au plus 6 atomes de carbone, aryle, arylthio, aryloxy, arylalkyle, dans lequel le radical alkyle est linéaire ou ramifié et renferme au plus 6 atomes de carbone,
R₂ représente :
**a)** les radicaux 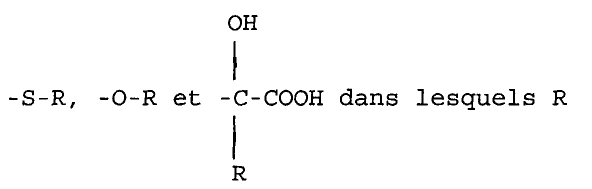 représente un radical alkyle ou alkényle, linéaire ou ramifié renfermant au plus 8 atomes de carbone, cycloalkyle renfermant au plus 6 atomes de carbone ou aryle, les radicaux alkyle, alkényle, cycloalkyle et aryle étant éventuellement substitués par un ou plusieurs radicaux choisis parmi les atomes d'halogène, le radical hydroxyle, alcoxy et alkylthio linéaire ou ramifié renfermant au plus 6 atomes de carbone et phényl lui-même éventuellement substitué par un ou plusieurs radicaux choisis parmi les atomes d'halogène, le radical hydroxyle et les radicaux alcoxy, linéaire ou ramifié renfermant au plus 6 atomes de carbone,
**b)** un atome d'halogène,
**c)** un radical 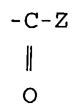 dans lequel Z représente un radical hydroxyle, alcoxy ou carboxy libre, salifié ou estérifié,
R₃ est choisi parmi
- un radical carboxy libre, salifié ou estérifié,
- un radical acyle, éventuellement substitué par un radical phényle, thiényle ou tétrazolyle,
- un atome d'halogène,
- un radical alkyle, alkényle ou alkylthio linéaire ou ramifié, renfermant au plus 8 atomes de carbone, éventuellement substitué par un ou plusieurs radicaux choisis parmi les atomes d'halogène, les radicaux
   . hydroxyle, alcoxy, acyle, aryle,
   . carboxy libre, salifié, estérifié ou amidifié,
   . les radicaux 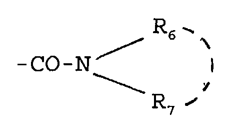 et 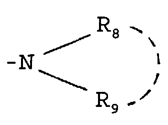
dans lesquels :
**ou bien** R₆ et R₇ ou R₈ et R₉, identiques ou différents, sont choisis parmi :
. l'atome d'hydrogène,
. les radicaux acyle, alkyle et alkényle renfermant au plus 6 atomes de carbone et alkylsulfonyle et arylsulfonyle, les radicaux étant éventuellement substitués par un ou plusieurs radicaux identiques ou différents choisis parmi les atomes d'halogène, le radical hydroxyle, les radicaux alcoxy renfermant au plus 6 atomes de carbone ou le radical carboxy libre, salifié ou estérifié,
. les radicaux aryle, arylalkyle et arylalkényle, dans lesquels les radicaux alkyle et alkényle linéaires ou ramifiés renferment au plus 6 atomes de carbone, ces radicaux aryle, arylalkyle et arylalkényle, étant éventuellement susbtitués par un ou plusieurs radicaux choisis parmi les atomes d'halogène, les radicaux hydroxyle, nitro, les radicaux alkyle, alkényle, haloalkyle, alcoxy et acyle renfermant au plus 6 atomes de carbone, le radical amino éventuellement substitué par un ou deux radicaux alkyles identiques ou différents renfermant au plus 6 atomes de carbone et les radicaux carboxy libre, salifié ou estérifié,
ou bien d'une part R₆ et R₇ et d'autre part R₈ et R₉ forment respectivement avec l'atome d'azote auxquels ils sont liés un radical hétérocyclique, choisis parmi les radicaux imidazolyle, pyrrolyle, pyrrolinyle, pyrrolidinyle, pipéridinyle, pyrimidinyle, pyridazinyle, pipérazinyle, phénylpipérazinyle, oxazolyle, morpholinyle et thiomorpholinyle, azépine, indolyle, ces radicaux étant éventuellement substitués par un ou plusieurs radicaux identiques ou différents choisis parmi les atomes d'halogène, les radicaux hydroxyle, trifluorométhyle, alkyle et alcoxy, ces radicaux renfermant au plus 6 atomes de carbone,
**ou bien** R₈ et R₉, identiques ou différents, représentent un acide aminé ou l'un de R₈ ou R₉ représente un radical carbamoyle, alcoxycarbonyle ou benzyloxycarbonyle ou R₈ et R₉ forment ensemble avec l'atome d'azote auquel ils sont liés un radical phtalimido ou succinimido,
R₄ représente
**a)** les radicaux - (CH₂)ₘ₁-COOR₁₄, - (CH₂)ₘ₁-CONHR₁₄, -(CH₂)ₘ₁-CN, dans lesquels ml représente un entier de 0 à 4, -SO₂-NH-SO₂-R₁₄, -NH-SO₂-R₁₄, -PO₃R₁₄, -NH-SO₂-CF₃ et 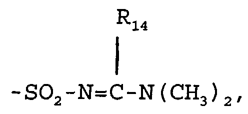 - (CH₂)ₘₗ-SO₃R₁₄, -CO-NH-OR₁₄, -CO-NH-NH-SO₂-CF₃, -CO-NH-SO₂-R₁₄, -CH₂SO₂NHCO-R₁₄, -CH₂-SO₂-NHR₁₄, -CH₂CONH-SO₂R₁₄, -NHSO₂NHCO-R₁₄, -NHCONHSO₂-R₁₄, NH-CH₂-SO₂-NHR₁₄, -CONHSO₂NR₁₄R₁₅, -SO₂NHCONR₁₄R₁₅, -SO₂N(R₁₄)OR₁₅, -SO₂NHPO(R₁₄)₂, -CONHPO(R₁₄)₂, -SO₂NHCN, - SO₂NHCOR₁₄, SO₂-NHCO₂R₁₄, - SO₂NHSO₂NR₁₄R₁₅, -SO₂NHSO₂N(-CH₂-CH₂-)₂D, -NHSO₂NHSO₂R₁₄, -NHSO₂NHPO(R₁₄)₂, -NR₁₄COCO₂H, -SO₂NHCO₂R₁₄, -SO₂-NH-CS-R₁₄, -SO₂-NH-CS-NH-R₁₄,
   avec D représente un atome d'oxygène ou de soufre,
b) le radical -SO₂-W-R₁₄ dans lequel W représente le radical - NR₁₅-, -NH-CO-, -NH-CO-O-, -N=CH-N-R₁₅- ou -NH-CO-NR₁₅-, radical dans lequel ou bien R₁₄ et R₁₅ identiques ou différents, sont choisis parmi l'atome d'hydrogène, le radical alkyle ou alkényle linéaire ou ramifié, renfermant au plus 8 atomes de carbone, cycloalkyle renfermant au plus 6 atomes de carbone et aryle, les radicaux alkyle, alkényle, cycloalkyle et aryle, étant éventuellement substitués par un ou plusieurs radicaux choisis parmi les atomes d'halogène, les radicaux hydroxyle, alcoxy renfermant au plus 4 atomes de carbone, nitro, cyano, amino, mono et dialkylamino, carboxy libre, salifié ou estérifié, haloalkyle, alkylthio, haloalkylthio, haloalcoxy, carbamoyle, acyle, acyloxy, cycloalkyle, cyclo-alkényle, aryle, phénylthio, pyridyle, tétrazolyle, thiényle, nitropyridyle, pyrimidyle, diazolyle, pipéridinyle, alkylpipéridinyle, thiazolyle, alkylthiazolyle, tétrahydrofuranyle et méthyltétrahydrofuranyle éventuellement substitués par un
ou plusieurs radicaux choisis parmi les atomes d'halogène et le radical hydroxyle ou alcoxyle renfermant au plus 4 atomes de carbone ;
ou bien R₁₄ et R₁₅ forment avec l'atome d'azote auquel ils sont liés un radical choisi parmi les radicaux pyrrolyle, pyrrolinyle, pyrrolidinyle, pipérazinyle, alkylpipérazinyle, phénylpipérazinyle, morpholinyle et indolinyle, étant entendu que les radicaux alkyle dans les produits de formule (I) sont éventuellement interrompus par un ou plusieurs hétéroatomes choisis parmi les atomes d'oxygène, soufre et azote et les atomes de soufre dans les produits de formule (I) peuvent éventuellement être oxydés sous forme de sulfone ou de sulfoxyde, lesdits produits de formule (I) étant sous toutes les formes isomères racémiques, énantiomères et diastéréoisomères possibles ainsi que les sels d'addition avec les acides minéraux et organiques ou avec les bases minérales et organiques desdits produits de formule (I).

Les produits de formule (I) tels que définis ci-dessus sont décrits dans les demandes de brevets européennes EP 465368 et EP 503162 et peuvent dont être préparés notamment ainsi qu'il est indiqué dans ces demandes de brevets européennes.

Dans les produits de formule (I) et dans ce qui suit :
- le terme radical alkyle linéaire ou ramifié désigne de préférence les radicaux méthyle, éthyle, propyle, isopropyle, butyle, isobutyle, sec-butyle et tert-butyle mais peut également représenter un radical pentyle ou hexyle et particulièrement isopentyle et isohexyle,
- le terme radical alkényle linéaire ou ramifié désigne de préférence un radical vinyle, allyle, 1-propényle, butényle et particulièrement 1-butényle, ou pentényle,
- le terme radical alkynyle linéaire ou ramifié désigne de préférence un radical éthynyle, propargyle, butynyle ou pentynyle.

Parmi les radicaux alkyles interrompus par un ou plusieurs hétéroatomes, on peut citer par exemple les radicaux méthoxyméthyle, méthoxyéthoxyméthyle, propylthiopropyle, propyloxypropyle, propylthioéthyle, méthylthiométhyle,
- le terme atome d'halogène désigne de préférence l'atome de chlore, mais peut aussi représenter un atome de fluor, de brome ou d'iode,
- le terme radical alcoxy linéaire ou ramifié désigne de préférence les radicaux méthoxy, éthoxy, propoxy ou isopropoxy, mais peut aussi représenter un radical butoxy linéaire, secondaire ou tertiaire,
- le terme radical acyle désigne de préférence un radical ayant de 1 à 6 atomes de carbone tel que par exemple le radical formyle, acétyle, propionyle, butyryle ou benzoyle, mais également le radical pentanoyle, hexanoyle, acryloyle, crotonoyle ou carbamoyle,
- le terme radical acyloxy désigne par exemple un radical dans lequel le radical acyle a les valeurs indiquées ci-dessus et désigne de préférence un radical formyloxy, acétyloxy, propionyloxy, butyryloxy ou benzoyloxy,
- le terme radical cycloalkyle désigne de préférence les radicaux cyclopropyle, cyclobutyle et tout particulièrement les radicaux cyclopentyle et cyclohexyle,
- le terme radical aryle désigne les radicaux insaturés, monocycliques ou constitués de cycles condensés, carbocycliques ou hétérocycliques, étant entendu que les radicaux hétérocycliques peuvent renfermer un ou plusieurs hétéroatomes identiques ou différents choisis parmi les atomes d'oxygène, d'azote ou de soufre.

Comme exemples de tel radical aryle, on peut citer les radicaux phényle, naphtyle, thiényle tel que 2-thiényle et 3-thiényle, furyle tel que 2-furyle, pyridyle tel que 2-pyridyle et 3-pyridyle, pyrimidyle, pyrrolyle, thiazolyle isothiazolyle, pyrazolyle, triazolyle, tétrazolyle, thiadiazolyle, thiatriazolyle, oxazolyle, oxadiazolyle, 3- ou 4-isoxazolyle ; benzothiényle tel que 3-benzothiényle, benzofuryle, benzopyrrolyle, benzimidazolyle, benzoxazolyle, thionaphtyle, indolyle, indolinyle ou purinyle, pyrrolidinyle, pipéridino, morpholino, pipérazinyle, ces radicaux pouvant être substitués par un ou plusieurs radicaux tels que définis ci-dessus comme par exemple dans méthylpipérazinyle, fluorométhylpipérazinyle, éthylpipérazinyle, propylpipérazinyle, phénylpipérazinyle ou benzylpipérazinyle ;
- le terme arylalkyle désigne des radicaux dans lesquels respectivement les radicaux alkyle et aryle peuvent prendre les valeurs définies ci-dessus pour ces radicaux ; comme exemples de tels radicaux arylalkyle on peut citer les radicaux benzyle, diphénylméthyle, triphénylméthyle, naphtylméthyle, indénylméthyle, thiénylméthyle tel que 2-thiénylméthyle, furylméthyle tel que furfuryle, pyridylméthyle, pyrimidylméthyle ou pyrrolylméthyle, étant entendu que dans la liste non exhaustive d'exemples de radicaux telle que citée ci-dessus, le radical alkyle peut être représenté tout aussi également par les radicaux éthyle, propyle ou butyle tel que, par exemple, dans le radical phényléthyle ;
- le terme radical haloalkyle désigne de préférence les radicaux dans lesquels le radical alkyle est tel que défini ci-dessus et est substitué par un ou plusieurs atomes d'halogène tel que défini ci-dessus comme par exemple dans bromoéthyle, trifluorométhyle, trifluoroéthyle ou encore pentafluoroéthyle,
- le terme radical alkylthio désigne de préférence les radicaux dans lesquels le radical alkyle est tel que défini ci-dessus comme par exemple dans méthylthio ou éthylthio,
- le terme radical haloalkylthio désigne de préférence les radicaux dans lesquels le radical alkyle est tel que défini ci-dessus et est substitué par un ou plusieurs atomes d'halogène tel que défini ci-dessus comme par exemple dans bromoéthylthio, trifluorométhylthio, trifluoroéthylthio ou encore pentafluoroéthylthio,
- le terme radical haloalcoxy désigne de préférence les radicaux dans lesquels le radical alcoxy est tel que défini ci-dessus et est substitué par un ou plusieurs atomes d'halogène tel que définis ci-dessus comme par exemple dans bromoéthoxy, trifluorométhoxy, trifluoroéthoxy ou encore pentafluoroéthoxy,
- le terme radical aryloxy désigne de préférence les radicaux dans lesquels le radical aryle est tel que défini ci-dessus comme par exemple dans phénoxy,
- le terme radical arylthio désigne de préférence les radicaux dans lesquels le radical aryle représente les radicaux tels que définis ci-dessus comme par exemple dans phénylthio,
- le terme radical aryle substitué par un radical alkylthio représente par exemple le radical benzylthio ou phénéthylthio.

Dans tous les radicaux que peuvent représenter R₁, R₂, R₃ et R₄, tels que définis ci-dessus, les atomes de soufre peuvent ne pas être oxydés comme dans les radicaux alkylthio, arylthio, cycloalkylthio tel que par exemple cyclohexylthio ou au contraire être oxydés pour donner les radicaux alkylsulfinyle, cycloalkylsulfinyle, arylsulfinyle, alkylsulfonyle, cycloalkylsulfonyle ou arylsulfonyle :
- les termes radical alkylthio, alkylsulfinyle et alkylsulfonyle désignent les radicaux dans lesquels le radical alkyle linéaire ou ramifié peut représenter, par exemple, les valeurs indiquées ci-dessus pour le radical alkyle ; ces radicaux représentent ainsi de préférence les radicaux méthylthio, hydroxyméthylthio, éthylthio, aminoéthylthio, méthylsulfinyle, éthylsulfinyle, méthylsulfonyle, éthylsulfonyle mais peut aussi représenter un radical propylthio, isopropylthio, butylthio, sec-butylthio, tert-butylthio, isopentylthio ou isohexylthio ou ces radicaux dans lesquels le radical thio est oxydé en radical sulfinyle ou sulfonyle,
- le terme radical arylthio, arylsulfinyle et arylsulfonyle désigne les radicaux dans lesquels le radical aryle peut représenter, par exemple, les valeurs indiquées ci-dessus pour le radical aryle comme, par exemple, dans phénylthio, pyridylthio ou pyrimidylthio, imidazolylthio, N-méthylimidazolylthio ou ces radicaux dans lesquels le radical thio est oxydé en radical sulfinyle ou sulfonyle tel que par exemple dans phénylsulfinyle ou phénylsulfonyle.

Parmi les substituants des radicaux alkylthio, alcoxy, arylthio et aryloxy, éventuellement oxydés, on peut citer par exemple les radicaux hydroxyle, alcoxy, carboxy libre, salifié ou estérifié, acyle, acyloxy, alkyle, phényle, les atomes d'halogène.

Comme exemples de radicaux alkyle substitués par un radical aryle, on peut citer, par exemple, les radicaux benzyle, diphénylméthyle, triphénylméthyle, naphtylméthyle, indénylméthyle, thiénylméthyle tel que 2-thiényl méthyle, furylméthyle tel que furfuryle, pyridylméthyle, pyrimidylméthyle ou pyrrolylméthyle, étant entendu que dans la liste non exhaustive d'exemples de radicaux telle que citée ci-dessus, le radical alkyle peut être représenté tout aussi également par les radicaux éthyle, propyle ou butyle tel que, par exemple, dans le radical phénéthyle.

Comme exemples de radicaux alkényle substitués par un radical aryle, on peut citer, par exemple, les exemples donnés ci-dessus de radicaux arylalkyle dans lesquels le radical alkyle est remplacé par un radical alkényle tel que par exemple dans les radicaux phénylvinyle ou phénylallyle, étant entendu que dans ces radicaux le radical phényle peut être remplacé tout aussi également par un radical naphtyl, pyridyle ou encore par exemple l'un des radicaux aryles tels que définis ci-dessus.

Les radicaux arylalkyle tels que définis ci-dessus, désignent de préférence des radicaux alkylphényle tels que benzyle, phénéthyle, et également les radicaux phénylpropyle et phénylbutyl.

Les radicaux carbamoyle et amino que peuvent représenter ou porter l'un ou plusieurs des éventuels substituants des radicaux définis dans les produits de formule (I) et dans ce qui suit, désignent des radicaux dans lesquels à l'atome d'azote sont liés deux radicaux, identiques ou différents, choisis parmi l'atome d'hydrogène pour donner le radical amino ; les radicaux alkyle tels que définis ci-dessus pour donner les radicaux monoalkyl- ou dialkylamino dans lesquels les radicaux alkyles linéaires ou ramifiés renferment de 1 à 6 atomes de carbone ; les radicaux phényle, benzyle, phénéthyle ou naphtyle, tous ces radicaux étant éventuellement substitués ainsi qu'il est indiqué ci-dessus et ci-après.

Lorsque R₆ et R₇ d'une part, R₈ et R₉ d'autre part ou R₁₄ et R₁₅ encore d'autre part, tels que définis ci-dessus, forment ensemble avec l'atome d'azote auquel ils sont liés un hétérocycle, il s'agit, par exemple, d'un cycle pyrrolyle, imidazolyle, indolyle, indolinyle, purinyle, pyrrolidinyle, pipéridino, morpholino, pipérazinyle, imidazolidinyle, pyrazolidinyle, thiomorpholinyle, azépine ; ces radicaux peuvent être éventuellement substitués par les substituants déjà mentionnés précédemment et en particulier par un ou plusieurs radicaux choisis parmi les atomes de chlore et de fluor,les radicaux méthyle, éthyle, isopropyle, tert-butyle, méthoxy, éthoxy, propoxy, benzoyle, méthoxycarbonyle, éthoxycarbonyle, comme par exemple dans méthylpipérazinyle, éthylpipérazinyle, propylpipérazinyle, phénylpipérazinyle ou benzylpipérazinyle : dans ces deux derniers radicaux, les radicaux phényle et benzyle peuvent être substitués comme indiqué précédemment dans les radicaux aryle, arylalkyle et arylalkényle, comme par exemple dans chlorophényle ou trifluorophényle.

L'hétérocycle que peuvent former R₆ et R₇ d'une part, R₈ et R₉ d'autre part ou R₁₄ et R₁₅ encore d'autre part respectivement avec l'atome d'azote auquel ils sont liés représente de préférence un hétérocycle saturé.

De plus, dans les produits de formule (I), les radicaux carbamoyle ou amino sont tels que les radicaux portés par l'atome d'azote, identiques ou différents, peuvent représenter des chaînes aliphatiques ou cyclisées ou peuvent former avec l'atome d'azote auquel ils sont liés un hétérocycle, ainsi qu'il a été défini ci-dessus pour R₆, R₇, R₈, R₉, R₁₄ et R₁₅.

Les radicaux carbamoyle substitué et amino substitué désignent respectivement les radicaux dans lesquels l'atome d'azote peut être substitué par un ou deux radicaux choisis parmi les radicaux tels que définis précédemment notamment le ou les radicaux alkyle choisis parmi les radicaux alkyle tels que définis ci-dessus comme par exemple pour monoalkylamino dans méthylamino ou éthylamino ou isopropylamino ou par exemple pour dialkylamino dans diméthylamino, diéthylamino ou encore méthyléthylamino, ces radicaux alkyles étant éventuellement substitués ainsi qu'il est indiqué ci-dessus, comme par exemple les radicaux méthoxyméthyle, méthoxyéthyle, éthoxyéthyle.

A titre d'exemple et de façon non exhaustive, le terme radical carbamoyle désigne les radicaux carbamoyle substitué sur l'atome d'azote par un ou deux radicaux alkyle éventuellement substitués ainsi qu'il est défini ci-dessus, pour former notamment un groupe N-monoalkyl carbamoyle tel que N-méthylcarbamoyle, N-éthylcarbamoyle ou un groupe N,N-dialkyl carbamoyle, tel que N,N-diméthylcarbamoyle, N,N-diéthylcarbamoyle ; un groupe N-(hydroxyalkyl) carbamoyle, tel que -(hydroxyméthyl) carbamoyle, N-(hydroxyéthyl) carbamoyle, phénylcarbamoyle ; pyridylcarbamoyle ; benzylcarbamoyle ; N-méthyl N-phénylcarbamoyle ; pyridylméthylcarbamoyle. Par ailleurs, parmi les radicaux alkyles substitués, on peut citer également les radicaux alkyles substitués par un radical carbamoyle tel que défini ci-dessus, pour former un groupe carbamoylalkyle tel que carbamoylméthyle ou carbamoyléthyle.

Le radical amino peut être un radical alcoxycarbonylamino, ce radical étant alors de préférence le radical tertbutyloxycarbonylamino ou le radical benzyloxycarbonylamino.

Les radicaux amino et carbamoyle peuvent encore notamment être substitués par un ou deux acides aminés choisis parmi les 20 acides aminés naturels tels que notamment la proline ou par exemple la glycine, l'alanine, la leucine, l'isoleucine, la valine ou la phénylalanine ou l'un des autres acides aminés naturels connus de l'homme de métier.

Selon que m₁ représente la valeur 0, 1, 2, 3 ou 4 le radical -(CH₂)ₘ- représente une simple liaison, le radical méthylène, le radical éthylène, propylène, isopropylène ou butylène.

Le ou les radicaux carboxy des produits de formule (I) peuvent être salifiés ou estérifiés par les groupements divers connus de l'homme du métier parmi lesquels on peut citer, par exemple :
- parmi les composés de salification, des bases minérales telles que, par exemple, un équivalent de sodium, de potassium, de lithium, de calcium, de magnésium ou d'ammonium ou des bases organiques telles que, par exemple, la méthylamine, la propylamine, la triméthylamine, la diéthylamine, la triéthylamine, la N,N-diméthyléthanolamine, le tris (hydroxyméthyl) amino méthane, l'éthanolamine, la pyridine, la picoline, la dicyclohexylamine, la morpholine, la benzylamine, la procaïne, la lysine, l'arginine, l'histidine, la N-méthylglucamine,
- parmi les composés d'estérification, les radicaux alkyle pour former des groupes alcoxy carbonyle tel que, par exemple, méthoxycarbonyle, éthoxycarbonyle, tert-butoxycarbonyle ou benzyloxycarbonyle, ces radicaux alkyles pouvant être substitués par des radicaux choisis par exemple parmi les atomes d'halogène, les radicaux hydroxyle, alcoxy, acyle, acyloxy, alkylthio, amino ou aryle comme, par exemple, dans les groupements chlorométhyle, hydroxypropyle, méthoxyméthyle, propionyloxyméthyle, méthylthiométhyle, diméthylaminoéthyle, benzyle ou phénéthyle.

Les sels d'addition avec les acides minéraux ou organiques des produits de formule (I) peuvent être, par exemple, les sels formés avec les acides chlorhydrique, bromhydrique, iodhydrique, nitrique, sulfurique, phosphorique, propionique, acétique, formique, benzoïque, maléique, fumarique, succinique, tartrique, citrique, oxalique, glyoxylique, aspartique, ascorbique, les acides alcoylmonosulfoniques tels que par exemple l'acide méthanesulfonique, l'acide éthanesulfonique, l'acide propanesulfonique, les acides alcoyldisulfoniques tels que par exemple l'acide méthanedisulfonique, l'acide alpha, bêta-éthanedisulfonique, les acides arylmonosulfoniques tels que l'acide benzènesulfonique et les acides aryldisulfoniques.

Lorsque R₂ et R₃ représentent tous deux un groupement soufré, R₂ et R₃ étant identiques ou différents, dans les produits préférés de l'invention ces groupements soufrés n'ont pas nécessairement le même degré d'oxydation.

R₂ et R₃ peuvent ainsi notamment représenter les radicaux alkylthio, substitués par un ou plusieurs atomes d'halogène tels que chlore et fluor, pour donner par exemple les radicaux : -S-CF₃ ; -S-CHF₂ ; -S-CH₂F ; -S-CF₂-CHF₂ ; -S-CF₂-CF₃.

L'invention a notamment pour objet l'utilisation telle que définie ci-dessus, des produits de formule (I) telle que définie ci-dessus et répondant à la formule (I_{A}) : 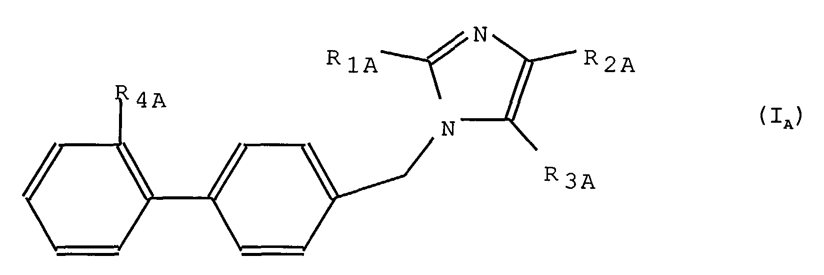 dans laquelle R_{1A} représente un radical alkyle ou alkylthio linéaire ou ramifié, renfermant au plus 6 atomes de carbone,
R_{2A} représente
**a)** les radicaux 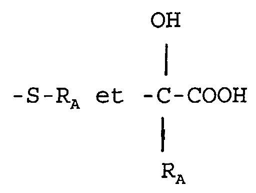 dans lesquels R_{A} représente un radical alkyle ou alkényle, linéaire ou ramifié, renfermant au plus 8 atomes de carbone, cycloalkyle ou phényle, ces radicaux étant éventuellement substitués par un ou plusieurs radicaux choisis parmi les atomes d'halogènes et plus particulièrement le fluor,
**b)** un atome d'halogène,
**c)** un radical 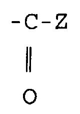
dans lequel Z représente un radical carboxy libre, salifié ou estérifié.
R_{3A} représente
- un radical carboxy libre, salifié, estérifié ou amidifié,
- un radical formyle et acétyle éventuellement substitué par un radical phényle, benzyle, phénéthyle ou tétrazolyle,
- un atome d'halogène,
- un radical alkyle, alkényle ou alkylthio linéaire ou ramifié, renfermant au plus 8 atomes de carbone, éventuellement substitué par un ou plusieurs radicaux choisis parmi les radicaux hydroxyle, alcoxy, acyle, phényle, carboxy libre, salifié, estérifié ou amidifié, carbamoyle et amino éventuellement substitués sur l'atome d'azote par un ou deux radicaux choisis parmi les radicaux alkyle renfermant au plus 6 atomes de carbone, acyle, alkylsulfonyle, phénylsulfonyle, phényle, phénylalkyle, ces radicaux étant éventuellement substitués par un ou plusieurs radicaux choisis parmi les atomes d'halogène, le radical hydroxyle et les radicaux alcoxy linéaire ou ramifié renfermant au plus 4 atomes de carbone,
R_{4A} représente SO₂-W_{A}-R_{16A}, dans lequel W_{A} représente le radical -NH-, NH-CO-, -NH-CO-O-, 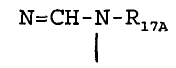 ou -NH-CO-NH- et R_{16A} et R_{17A} représentent un atome d'hydrogène, un radical alkyle ou alkényle, linéaire ou ramifié renfermant au plus 4 atomes de carbone, ou un radical aryle, ces radicaux étant éventuellement substitués par un ou plusieurs radicaux choisis parmi les atomes d'halogène, les radicaux hydroxyle, alcoxy renfermant au plus 4 atomes de carbone, nitro, cyano, amino, mono et dialkylamino, carboxy libre, salifié ou estérifié, cyclohexyle, cyclohexényle, pyridyle, thiényle et phényle, ces radicaux étant éventuellement substitués par un atome d'halogène ou un radical hydroxyle ou alcoxy renfermant au plus 4 atomes de carbone, étant entendu que les atomes de soufre dans les produits de formule (I_{A}) peuvent éventuellement être oxydés sous forme de sulfone ou de sulfoxyde, lesdits produits de formule (I_{A}) étant sous toutes les formes isomères racémiques, énantiomères et diastéréoisomères possibles ainsi que les sels d'addition avec les acides minéraux et organiques ou avec les bases minérales et organiques desdits produits de formule (I_{A}).

Le radical SO₂-W_{A}-R_{16A} que représente R_{4A} peut notamment représenter les radicaux suivants : SO₂NH₂, -SO₂-N=CH-N(CH₃)₂, SO₂-NH-CO-CF₃,

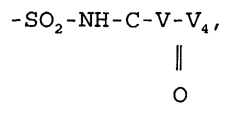

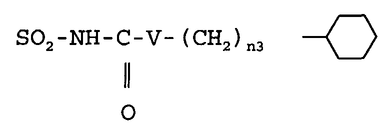

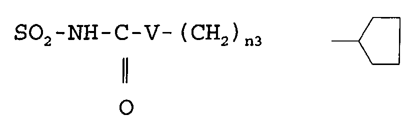

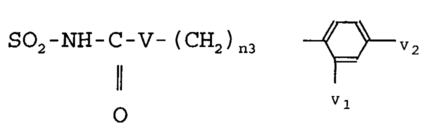

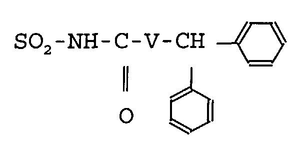

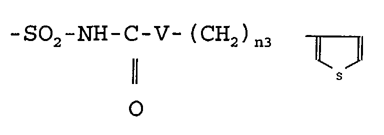

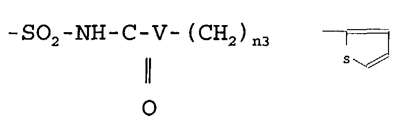

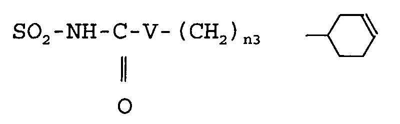

-SO₂-NH-CO-V-CH₂-CH=CH₂

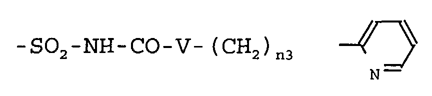

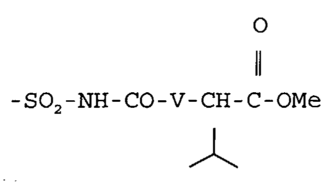

avec n3 représente un entier de 0 à 3, V représente -NH-, -O- ou une simple liaison, V₁ et V₂ identiques ou différents représentent un atome d'hydrogène, un atome d'halogène notamment chlore et fluor et un radical alcoxy notamment méthoxy, V₄ représente un atome d'hydrogène, un radical alkyle tel que notamment méthyle, éthyle, propyle et butyle.

L'invention a particulièrement pour objet l'utilisation telle que définie ci-dessus des produits de formule (I) suivants, dénomms respectivement P1 à P13 :
- Acide 2-butyl 1-[(2'-(((((cyclohexylméthyl) amino) carbonyl) amino) sulfonyl) (1,1'-biphényl) 4-yl) méthyl] 4-(méthylthio) 1H-imidazole 5-carboxylique,
- Acide 2-butyl 4-(méthylthio) 1-[(2'-(((((phénylméthyl) amino) carbonyl) amino) sulfonyl) (1,1'-biphényl) 4-yl) méthyl] 1H-imidazole 5-carboxylique,
- Acide 2-butyl 4-(méthylthio) 1-[(2'-(((((1-β-phénylpropyl) amino) carbonyl) amino) sulfonyl) (1,1'-biphényl) 4-yl) méthyl] 1H-imidazole 5-carboxylique,
- Acide 2-butyl 4-(méthylthio) 1-[(2'-(((((1-(4-phénylbutyl)) amino) carbonyl) amino) sulfonyl) (1,1'-biphényl) 4-yl) méthyl] 1H-imidazole 5-carboxylique,
- Acide 2-butyl 1-[(2'-((((((4-méthoxy) phénylméthyl) amino) carbonyl) amino) sulfonyl) (1,1'-biphényl) 4-yl) méthyl] 4-méthylthio 1H-imidazole 5-carboxylique,
- Acide 2-butyl 1-[(2'-((((((4-fluoro) phénylméthyl) amino) carbonyl) amino) sulfonyl) (1,1'-biphényl) 4-yl) méthyl] 4-méthylthio 1H-imidazole 5-carboxylique,
- Acide 2-butyl 1-[(2'-(((((1-(1-carboxy 2-phényl) éthyl) amino) carbonyl) amino) sulfonyl) (1,1'-biphényl) 4-yl) méthyl] 4-méthylthio 1H-imidazole 5-carboxylique,
- Acide 2-butyl 4-(méthylthio) 1-[(2'-((((2-thiényl) méthoxycarbonyl) amino) sulfonyl) (1,1'-biphényl) 4-yl) méthyl] 1H-imidazole 5-carboxylique,
- Acide 2-butyl 4-(méthylthio) 1-[(2'-((((3-thiényl) méthoxycarbonyl) amino) sulfonyl) (1,1'-biphényl) 4-yl) méthyl] 1H-imidazole 5-carboxylique,
- Acide 2-butyl 1-[(2'-((((cyclohexen 4-yl) méthoxycarbonyl) amino) sulfonyl) (1,1'-biphényl) 4-yl) méthyl] 4-méthylthio 1H-imidazole 5-carboxylique,
- Acide 2-butyl 1-[(2'-((((cyclohexyl) méthoxycarbonyl) amino) sulfonyl) (1,1'-biphényl) 4-yl) méthyl] 4-méthylthio 1H-imidazole 5-carboxylique,
- Acide 2-butyl 4-(méthylthio) 1-[(2'-(((phénylméthoxycarbonyl) amino) sulfonyl) (1,1'-biphényl) 4-yl) méthyl] 1H-imidazole 5-carboxylique,
- ((4'-((2-butyl 5-formyl 4-méthoxy 1H-imidazol 1-yl) méthyl) (1,1'-biphényl) 2-yl) sulfonyl) carbamate de (phénylméthyle).

Les produits indiqués ci-dessus peuvent notamment être préparés ainsi qu'il est indiqué dans la demande de brevet européen EP 0503162.

Parmi les produits de formules (I) et (I_{A}) certains produits de formule (I_{B}) sont nouveaux.

L'invention a aussi particulièrement pour objet les produits de formule (I_{B}) : 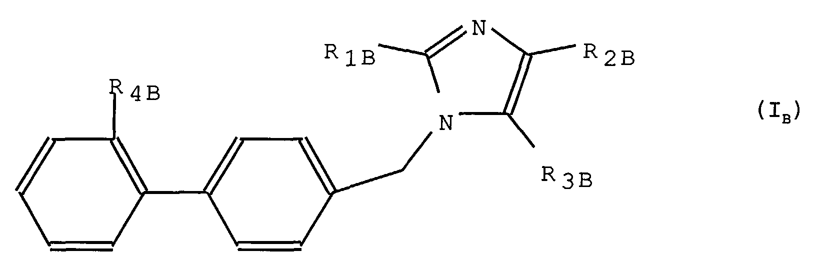 dans laquelle
R_{1B} représente un radical alkyle, linéaire ou ramifié renfermant au plus 4 atomes de carbone,
R_{2B} représente un radical alkylthio, linéaire ou ramifié renfermant au plus 4 atomes de carbone, éventuellement substitué par un ou plusieurs atomes de fluor,
R_{3B} représente
- un radical carboxy libre, salifié, estérifié ou amidifié,
- un radical acyle, carbamoyle,
- un radical alkyle et alkényle renfermant au plus 6 atomes de carbone substitués par un ou plusieurs radicaux choisis parmi les atomes d'halogène, les radicaux hydroxyle, alcoxy, carboxy, libre, salifié, estérifie ou amidifié, phényle et carbamoyle, éventuellement substitué par un radical phényle ou benzyle,
R_{4B} représente le radical -SO₂-NH₂, -SO₂-N=CH-N(CH₃)₂, 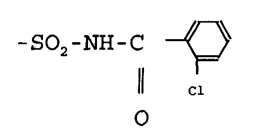 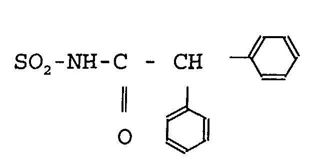 -SO₂-NH-CO₂Et, -SO₂-NH-CO₂Pr, -SO₂-NH-CO₂Bu, -SO₂-NH-CO₂H, 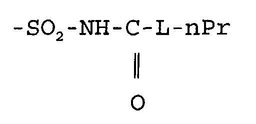 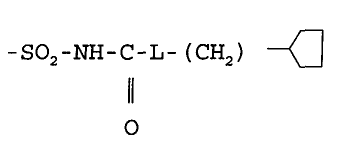 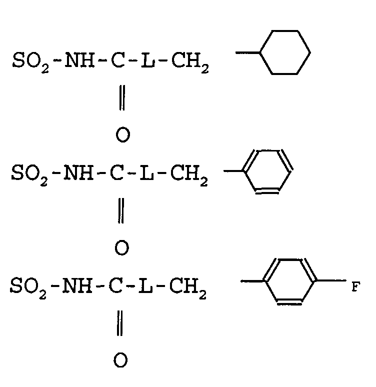 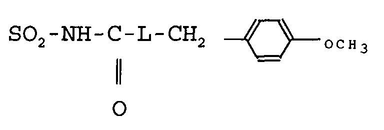 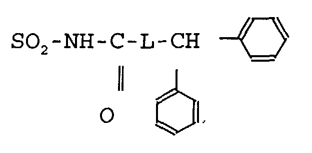 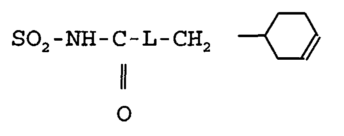 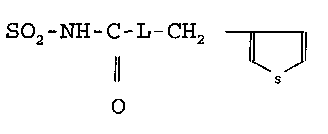 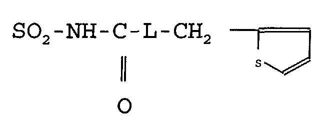 avec L représente -O- ou -NH- ,
étant entendu que les atomes de soufre dans les produits de formule (I_{B}) peuvent éventuellement être oxydés sous forme de sulfone ou de sulfoxyde, lesdits produits de formule (I_{B}) étant sous toutes les formes isomères racémiques, énantiomères et diastéréoisomères possibles ainsi que les sels d'addition avec les acides minéraux et organiques ou avec les bases minérales et organiques desdits produits de formule (I_{B}).

L'invention a tout particulièrement pour objet les produits de formule (I_{B}) telle que définie ci-dessus, répondant aux formules suivantes :
- Acide 2-butyl α-hydroxy α-méthyl 4-(méthylthio) 1-[(2'((((phénylméthoxy) carbonyl) amino) sulfonyl) (1,1'-biphényl) 4-yl) méthyl] 1H-imidazole 5-acétique,
- Acide 2-butyl α-hydroxy α-méthyl 4-(méthylthio) 1-[(2'((((propylamino) carbonyl) amino) sulfonyl) (1,1'-biphényl) 4-yl) méthyl] 1H-imidazole 5-acétique,
- Acide 2-butyl α-hydroxy α-méthyl 4-(méthylthio) 1-[(2'((((phénylméthyl) amino) carbonyl) amino) sulfonyl) (1,l1biphényl) 4-yl) méthyl] 1H-imidazole 5-acétique,
- Acide 2-butyl 1-[(2'-(((((cyclohexylméthyl) amino) carbonyl) amino) sulfonyl) (1,1'-biphényl) 4-yl) méthyl] α-hydroxy α-méthyl 4-(méthylthio) 1H-imidazole 5-acétique,
- Acide 1-[(2'-(((((phénylméthyl) amino) sulfonyl) (1,1'biphényl) 4-yl) méthyl] α-hydroxy 4-(méthylthio) 2-propyl 1H-imidazole 5-acétique,
- 1-[(2'-(((((cyclohexylméthyl) amino) carbonyl) amino) sulfonyl) (1,1'-biphényl) 4-yl) méthyl] 4-(méthylthio) 2-propyl 1H-imidazole 5-carboxylate de sodium,
- Acide 4-(méthylthio) 1-[(2'-(((((phénylméthyl) amino) carbonyl) amino) sulfonyl) (1,1'-biphényl) 4-yl) méthyl] 2-propyl 1H-imidazole 5-carboxylique,
- α-butyl α-hydroxy 4-(méthylthio) 1-[(2'-(((((phénylméthyl) amino) carbonyl) amino) sulfonyl) (1,1'-biphényl) 4-yl) méthyl] 2-propyl 1H-imidazole 5-acétate de sodium,
- Acide α-hydroxy 4-(méthylthio) α-phényl 1-((2'-(((((phénylméthyl) amino) carbonyl) amino) sulfonyl) (1,1'-biphényl) 4-yl) méthyl) 2-propyl 1H-imidazole 5-acétique,
- Acide 1-((2'-(((((cyclohexylméthyl) amino) carbonyl) amino) sulfonyl) (1,1'-biphényl) 4-yl) méthyl) 4-(difluorométhyl) thio) 2-propyl 1H-imidazole 5-carboxylique,
- Acide 4-((difluorométhyl) thio) 1-((2'-(((((phénylméthyl) amino) carbonyl) amino) sulfonyl) 1,1'-biphényl) 4-yl) méthyl) 2-propyl 1H-imidazole 5-carboxylique,
- Acide 4-((difluorométhyl) thio) 2-propyl 1-((2'-(((((2-thiénylméthyl) amino) carbonyl) amino) sulfonyl) (1,l'biphényl) 4-yl) méthyl) 1H-imidazole 5-carboxylique.

L'invention a également pour objet un procédé de préparation des produits de formule (I), telle que définie ci-dessus, caractérisé en ce que :
soit l'on soumet un composé de formule (II): 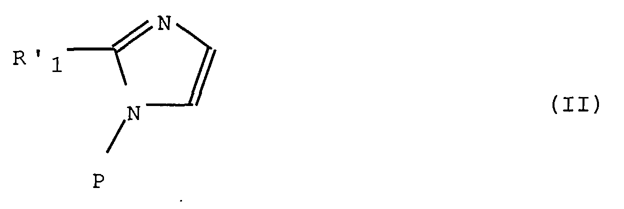 dans laquelle R'₁ a la signification indiquée ci-dessus pour R₁, dans laquelle les éventuelles fonctions réactives sont éventuellement protégées par des groupements protecteurs et P représente un groupement protecteur de l'atome d'azote, à une réaction d'halogénation pour obtenir un composé de formule (III) : 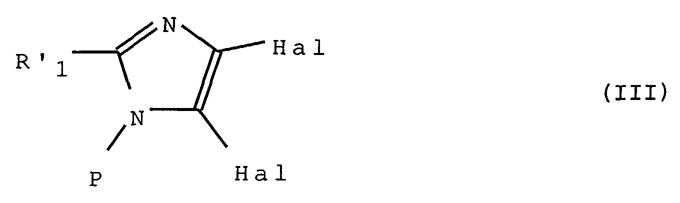 dans laquelle R'₁ et P ont les significations indiquées ci-dessus et Hal représente un atome d'halogène, que l'on soumet à une réaction d'échange halogène-métal sur l'un des atomes d'halogène puis à une réaction avec un composé de formule (IVₐ), (IV_{b}), (IV_{c}), (IV_{d}) ou (IVₑ) :
(-S-R')₂ (IVₐ) ou MeSO₂SR' (IV_{b}) ou 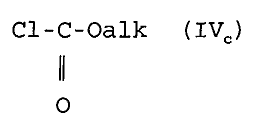 ou 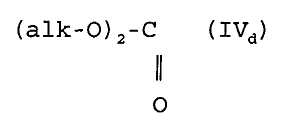 ou 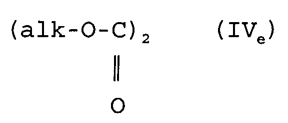
dans lesquelles R' a la signification indiquée ci-dessus pour R, dans laquelle les éventuelles fonctions réactives sont éventuellement protégées par des groupements protecteurs, et alk représente un radical alkyle renfermant au plus 4 atomes de carbone,
pour obtenir le composé de formule (V) : 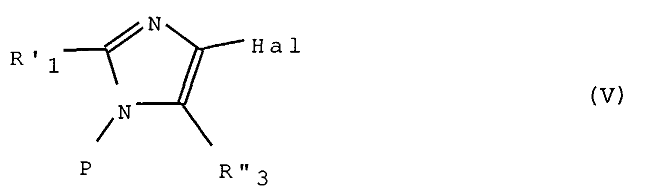 dans laquelle R'₁, P et Hal ont les significations indiquées ci-dessus et R"₃ représente S-R' ou K-O-alk tels que définis ci-dessus avec K représente le radical 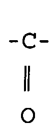 ou 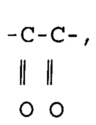 composé de formule (V) que l'on peut soumettre à une réaction d'échange halogène-métal sur l'atome d'halogène puis à une réaction avec un composé de formule (IV'ₐ), (IV'_{b}), (IV'_{c}), (IV'_{d}) ou (IV'ₑ)
(-S-R")₂ (IV'ₐ) ou MeSO₂SR" (IV'_{b}) 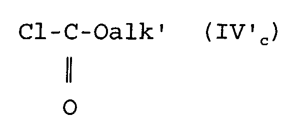 ou 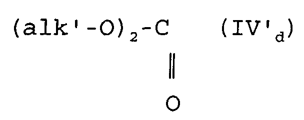 ou 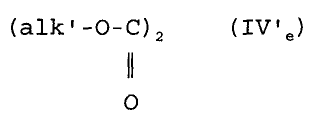 dans lesquelles R" identique ou différent de R' a la signification indiquée ci-dessus pour R, dans lesquelles les éventuelles fonctions réactives sont éventuellement protégées par des groupements protecteurs et alk' identique ou différent de alk, représente un radical alkyle renfermant au plus 4 atomes de carbone, pour obtenir le composé de formule (VII) : 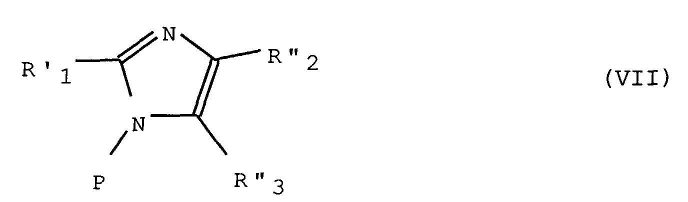 dans laquelle R'₁ et P ont les significations indiquées ci-dessus, et R"₂ et R"₃ identiques ou différents représentent -S-R', -S-R", -K-Oalk ou -K-Oalk' tels que définis ci-dessus dans lesquelles R', R", alk, alk' et K ont les significations indiquées ci-dessus,
   produit de formule (VII) dont on libère la fonction amine bloquée par P tel que défini ci-dessus, puis fait réagir avec un composé de formule (VIII) : 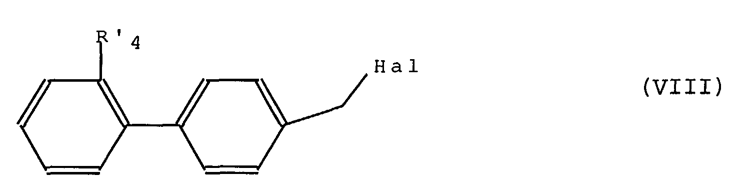
dans laquelle R'₄ a la signification indiquée ci-dessus pour R₄, dans laquelle les éventuelles fonctions réactives sont éventuellement protégées par des groupements protecteurs et Hal représente un atome d'halogène pour obtenir un produit de formule (I₁) 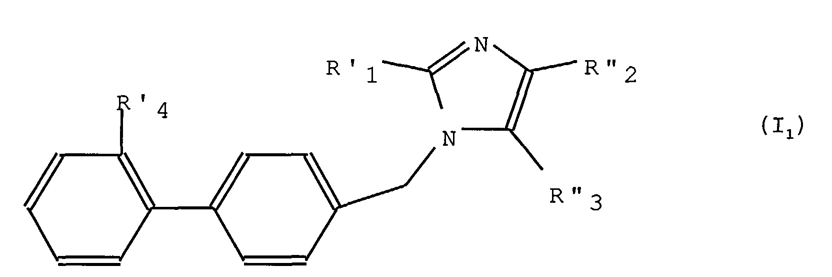 dans laquelle R'₁, R"₂, R"₃ et R'₄ ont les significations indiquées ci-dessus, soit l'on soumet un composé de formule (IX) : 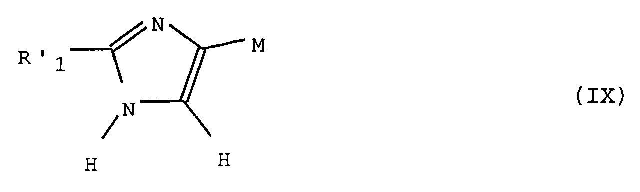 dans laquelle R'₁ a la signification indiquée ci-dessus et M représente un atome d'hydrogène ou le radical R'₂ qui a la signification indiquée ci-dessus pour R₂, dans laquelle les éventuelles fonctions réactives sont éventuellement protégées par des groupements protecteurs,
à une réaction avec le composé de formule (VIII) telle que définie ci-dessus, pour obtenir un produit de formule (X) : 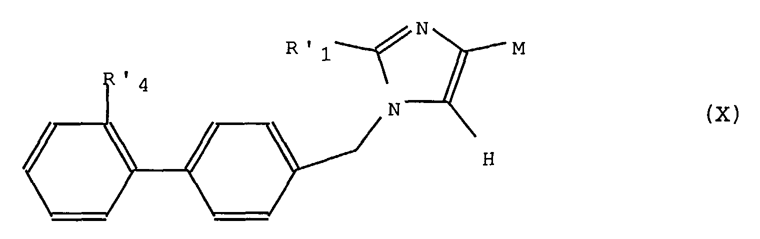 dans laquelle R'₁, M et R'₄ ont les significations indiquées ci-dessus,
produits de formule (X) que lorsque M représente R'₂ tel que défini ci-dessus, l'on soumet à une réaction d'halogénation pour obtenir le produit de formule (XI) : 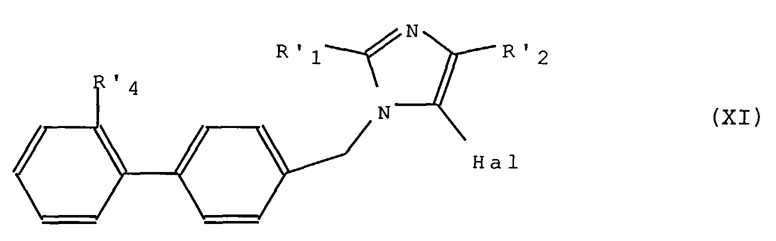 dans laquelle R'₁, R'₂, R'₄ et Hal ont les significations indiquées ci-dessus, que l'on soumet à une réaction d'échange halogène-métal puis à une réaction avec un composé de formule (XII) : 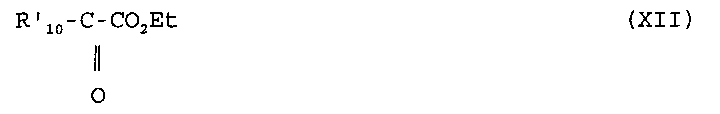 dans laquelle R'₁₀ a la signification indiquée ci-dessus pour R₁₀, dans laquelle les éventuelles fonctions réactives sont éventuellement protégées par des groupements protecteurs, pour obtenir un produit de formule (I₂) : 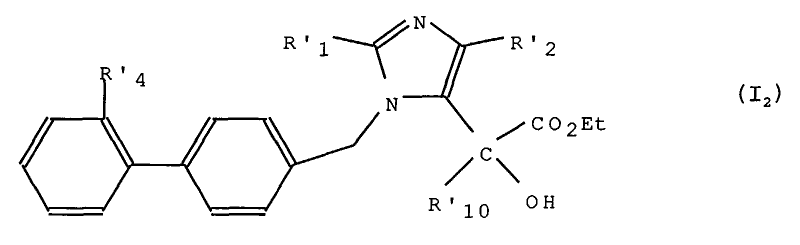 dans laquelle R'₁, R'₂, R'₄ et R'₁₀ ont les significations indiquées ci-dessus,
ou bien l'on soumet le produit de formule (I₂) à une réaction de saponification pour obtenir le produit de formule (I₄) : 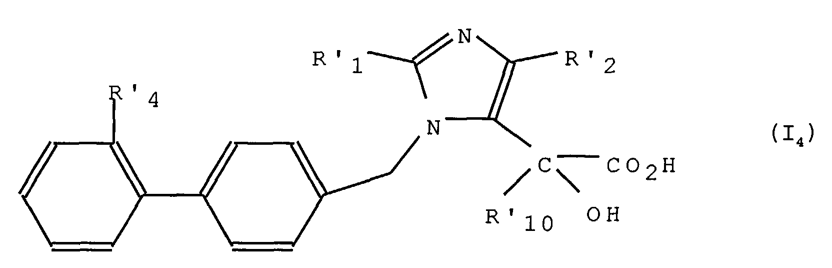 dans laquelle R'₁, R'₂, R'₄ et R'₁₀ ont les significations indiquées ci-dessus,
produit de formule (X) que lorsque M représente un atome d'hydrogène, l'on peut soumettre à une réaction d'halogénation pour obbtenir le produit de formule (XIV) : 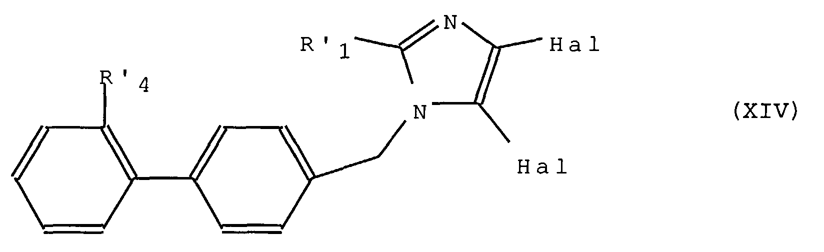 dans laquelle R'₁, R'₄ et Hal ont les significations indiquées ci-dessus, que l'on peut soumettre à une réaction d'échange halogène métal puis à l'action d'un composé de formule (IVₐ), (IV_{b}), (IV_{c}), (IV_{d}) ou (IVₑ) telle que définie ci-dessus pour obtenir le produit de formule (I₇) : 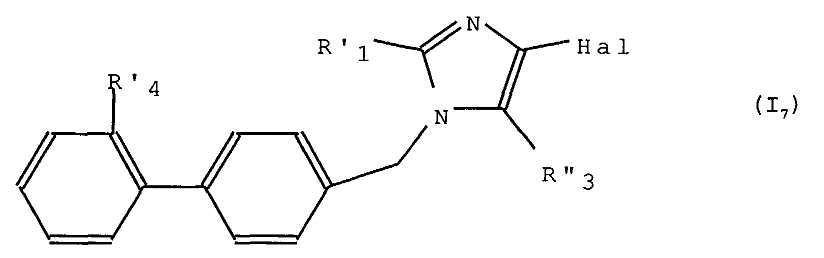 dans laquelle R'₁, R'₄, Hal et R"₃ ont les significations indiquées ci-dessus, produit de formule (I₇) que l'on peut soumettre à une réaction d'échange halogène métal puis à l'action d'un composé de formule (IV'ₐ), (IV'_{b}), (IV'_{c}), (IV'_{d}) ou (IV'ₑ), telle que définie ci-dessus, pour obtenir un produit de formule (I₈) : 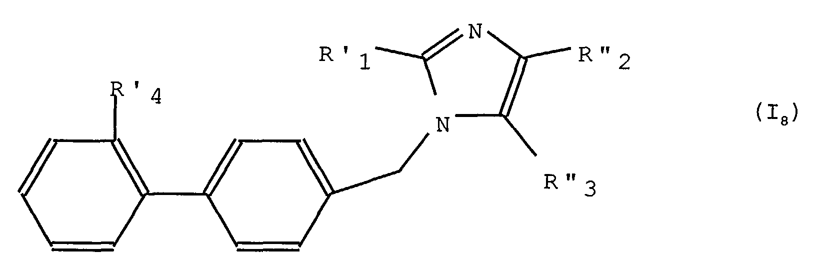 dans laquelle R'₁, R'₄, R"₂ et R"₃ ont les significations indiquées ci-dessus,
soit l'on soumet un composé de formule (XX) : 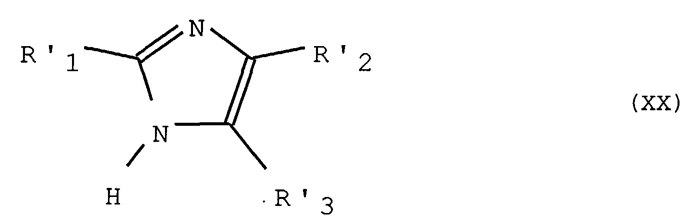 dans laquelle R'₁ et R'₂ ont les significations indiquées ci-dessus et R'₃ a la signification indiquée ci-dessus pour R₃ dans laquelle les éventuelles fonctions réactives sont éventuellement protégées par des groupements protecteurs, à une réaction avec le composé de formule (VIII) telle que définie ci-dessus, pour obtenir un produit de formule (I') : 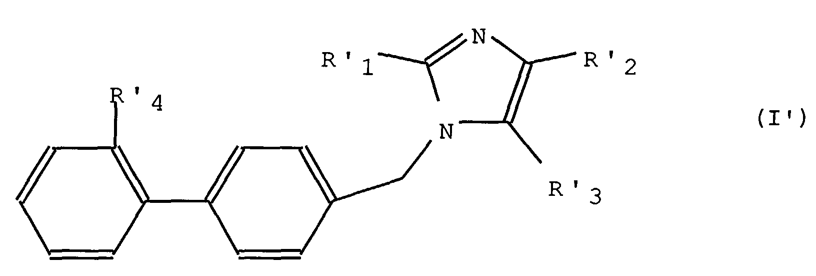 dans laquelle R'₁, R'₂, R'₃ et R'₄ ont les significations indiquées ci-dessus, produits de formules (I₁), (I₂), (I₃), (I₄), (I₅), (I₆), (I₇), (I₈) et (I') qui peuvent être des produits de formule (I) et que, pour obtenir des ou d'autres produits de formule (I), l'on peut soumettre, si désiré et si nécessaire, à l'une ou plusieurs des réactions de transformations suivantes, dans un ordre quelconque :
a) estérification de fonction acide,
b) saponification de fonction ester en fonction acide,
c) transformation de fonction ester en fonction acyle,
d) transformation de la fonction cyano en fonction acide,
e) transformation de fonction acide en fonction amide, puis éventuellement en fonction thioamide,
f) réduction de la fonction carboxy en fonction alcool,
g) transformation de fonction alcoxy en fonction hydroxyle,
   ou encore de fonction hydroxyle en fonction alcoxy,
h) oxydation de fonction alcool en fonction aldéhyde, acide
   ou cétone,
i) transformation du radical formyle en radical carbamoyle,
j) transformation du radical carbamoyle en radical nitrile,
k) transformation de radical nitrile en tétrazolyle,
l) oxydation de groupement alkylthio ou arylthio en sulfoxyde ou sulfone correspondant,
m) transformation de fonction sulfure, sulfoxyde ou sulfone en fonction sulfoximine correspondante,
n) transformation de fonction oxo en fonction thioxo,
o) déshydratation d'un radical hydroxylalkyle en radical alkényle,
p) transformation de fonction acide en fonction 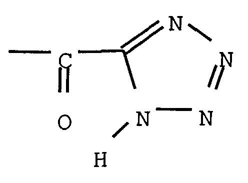
q) transformation de la fonction β-céto-sulfoxyde en fonction α-céto thio ester,
r) transformation d'un carbamate en urée et notamment d'un sulfonylcarbamate en sulfonylurée,
s) élimination des groupements protecteurs que peuvent porter les fonctions réactives protégées,
t) salification par un acide minéral ou organique ou par une base pour obtenir le sel correspondant,
u) dédoublement des formes racémiques en produits dédoublés, lesdits produits de formule (I) ainsi obtenus étant sous toutes les formes isomères possibles racémiques, énantiomères et diastéréoisomères.

Dans des conditions préférentielles de mise en oeuvre de l'invention, la réaction d'halogénation des composés de formule (II) et (X) telles que définies ci-dessus respectivement en composés de formule (III) et (XI) ou (XIV) telles que définies ci-dessus, peut être réalisée dans les conditions usuelles de l'homme du métier et notamment par bromation à l'aide de N-bromosuccinimide dans le dichlorométhane ou encore de brome dans l'acide acétique.

L'obtention du composé de formule (V) correspondant peut être réalisée par réaction du composé de formule (III) telle que définie ci-dessus avec un composé organo métallique tel que le n-butyllithium dans un solvant tel que le tétrahydrofuranne à une température d'environ -78°C suivie de l'action d'un composé de formule (IVₐ), (IV_{b}), (IV_{c}), (IV_{d}) ou (IVₑ).

La réaction du produit de formule (V) telle que définie ci-dessus avec le composé de formule (IV'ₐ), (IV'_{b}), (IV'_{c}) (IV'_{d}) ou (IV'ₑ), telle que définie ci-dessus, pour obtenir un composé de formule (VII) telle que définie ci-dessus peut être réalisée d'une manière identique en utilisant le n-butyllithium comme agent de métallation.

La fonction amine du composé de formule (VII) telle que définie ci-dessus, protégée par P tel que défini ci-dessus, peut être libérée dans les conditions usuelles connues de l'homme du métier et notamment lorsque P représente le radical -CH₂-O-(CH₂)₂-Si(CH₃)₃, par action de l'acide trifluoroacétique ou encore en présence d'ion fluorure.

Dans le produit de formule (VIII), Hal représente de préférence un atome de brome mais peut également représenter un atome de chlore ou d'iode.

La réaction du produit de formule (VIII) sur le produit de formule (VII) ou (IX) ou (XX), peut être réalisée dans un solvant tel que par exemple le diméthylformamide ou encore le diméthylacétamide, le tétrahydrofuranne, le diméthoxyéthane ou le diméthylsulfoxyde au reflux du solvant ou à la température ambiante, de préférence sous agitation ; la réaction est réalisée de préférence en présence d'une base telle que par exemple l'hydrure de sodium ou de potassium ou encore du carbonate de sodium ou de potassium, du méthylate ou éthylate ou tert-butylate de sodium ou de potassium.

L'obtention du composé de formule (I₂) telle que définie ci-dessus est réalisée par action, sur le dérivé magnésien du composé de formule (XI), du composé de formule (XII) telle que définie ci-dessus dans un solvant tel que par exemple le tétrahydrofuranne ou le toluène.

Le dérivé magnésien du composé de formule (XI) est réalisée par action du composé de formule (XI) telle que définie ci-dessus dans laquelle Hal peut par exemple représenter un atome de brome, avec un composé magnésien comme par exemple le chlorure d'isopropyle magnésium, dans un solvant tel que par exemple le toluène.

La réaction de saponification du produit de formule (I₂) telle que définie ci-dessus, en produit de formule (I₄) telle que définie ci-dessus, peut être réalisée selon les méthodes usuelles connues de l'homme du métier, telles que par exemple en présence de soude ou de potasse ou encore de carbonate de cesium, dans un solvant tel que le méthanol ou l'éthanol le dioxane ou le diméthoxyéthane.

La réaction de transformation du produit de formule (XIV) telle que définie ci-dessus, en produit de formule (I₇) telle que définie ci-dessus puis en produit de formule (I₈), telle que définie ci-dessus, peut être réalisée dans les mêmes conditions que celles définies pour l'obtention des produits de formules (V) et (VII) telles que définies ci-dessus, à partir du produit de formule (III) telle que définie ci-dessus.

Selon les valeurs de R'₁, R'₂, R"₂, R'₃, R"₃ et R'₄, les produits de formules (I₁), (I₂), (I₄), (I₇), (I₈) et (I') constituent ou non des produits de formule (I) et peuvent donner des produits de formule (I), ou être transformés en d'autres produits de formule (I) en étant soumis à une ou plusieurs des réactions a) à u) indiquées ci-dessus.

Ainsi les diverses fonctions réactives que peuvent porter certains composés des réactions définies ci-dessus peuvent, si nécessaire, être protégées : il s'agit par exemple des radicaux hydroxyle, acyle, carboxy libres ou encore amino et monoalkylamino qui peuvent être protégés par les groupements protecteurs appropriés.

La liste suivante, non exhaustive, d'exemples de protection de fonctions réactives peut être citée :
- les groupements hydroxyle peuvent être protégés par exemple par les radicaux alkyle tels que tert-butyle, triméthylsilyle, tert-butyldiméthylsilyle, méthoxyméthyle, tétrahydropyrannyle, benzyle ou acétyle,
- les groupements amino peuvent être protégés par exemple par les radicaux acétyle, trityle, benzyle, tert-butoxycarbonyle, phtalimido ou d'autres radicaux connus dans la chimie des peptides,
- les groupements acyles tel que le groupement formyle peuvent être protégés par exemple sous forme de cétals ou de thiocétals cycliques ou non cycliques tels que le diméthyl ou diéthylcétal ou l'éthylène dioxycétal, ou le diéthylthiocétal ou l'éthylènedithiocétal,
- les fonctions acide des produits décrits ci-dessus peuvent être, si désiré, amidifiées par une amine primaire ou secondaire par exemple dans du chlorure de méthylène en présence, par exemple, de chlorhydrate de 1-éthyl-3-(diméthylaminopropyl) carbodiimide à la température ambiante :
- les fonctions acide peuvent être protégées par exemple sous forme d'esters formés avec les esters facilement clivables tels que les esters benzyliques ou ter butyliques ou des esters connus dans la chimie des peptides.

Les réactions auxquelles les produits de formules (I₁), (I₂), (I₄), (I₇), (I₈) et (I'), telles que définies ci-dessus peuvent être soumis, si désiré ou si nécessaire, peuvent être réalisées, par exemple, comme indiqué ci-après.
a) Les produits décrits ci-dessus peuvent, si désiré, faire l'objet, sur les éventuelles fonctions carboxy, de réactions d'estérification qui peuvent être réalisées selon les méthodes usuelles connues de l'homme du métier.
b) Les éventuelles transformations de fonctions ester en fonction acide des produits décrits ci-dessus peuvent être, si désiré, réalisées dans les conditions usuelles connues de l'homme du métier notamment par hydrolyse acide ou alcaline par exemple par de la soude ou de la potasse en milieu alcoolique tel que, par exemple, dans du méthanol ou encore par de l'acide chlorhydrique ou sulfurique.
c) La réaction d'addition sur la fonction ester 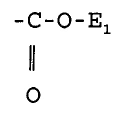 dans laquelle E₁ peut représenter un radical alkyle ou aryle éventuellement substitué et éventuellement protégé en fonction acyle 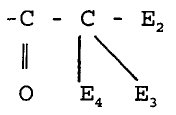 peut être réalisée notamment par action de l'anion carboné 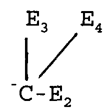 dans lequel E₂, E₃ et E₄, identiques ou différents sont choisis parmi l'atome d'hydrogène, les radicaux alkyl, alkylthioaryle, alkylsulfoxyde, arylsulfoxyde, alkylsulfone, arylsulfone, acyle, carboxy libre, salifié, estérifié ou amidifié, les radicaux alkyle, alkylthio et aryle étant éventuellement substitués et éventuellement protégés ainsi qu'il est indiqué ci-dessus.
   Une telle réaction est réalisée notamment ainsi qu'il est décrit dans la partie expérimentale, ou selon les méthodes usuelles connues de l'homme du métier.
d) Les éventuelles fonctions cyano des produits décrits ci-dessus peuvent être, si désiré, transformées en fonction acide dans les conditions usuelles connues de l'homme du métier par exemple par une double hydrolyse réalisée en milieu acide tel que par exemple dans un mélange d'acide sulfurique, d'acide acétique glacial et d'eau, ces trois composés étant de préférence en proportions égales, ou encore dans un mélange de soude, d'éthanol et d'eau au reflux.
e) La réaction de transformation de fonction acide en fonction amide peut notamment être réalisée par formation d'abord d'un chlorure d'acide selon les conditions usuelles connues de l'homme du métier et par exemple par action de SOCl₂ puis amidification comme indiqué ci-dessus, ou encore par amidification directe de l'acide ci-dessus.
   Notamment, on peut obtenir le radical 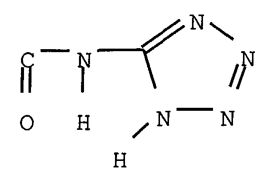 en transformant la fonction acide en chlorure d'acide, notamment par action de SOCl₂ dans un solvant tel que par exemple le toluène, ou le benzène,
   puis en faisant agir l'amine 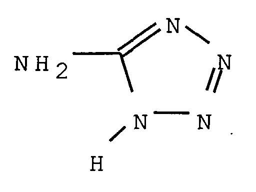
   L'amide ainsi obtenu peut alors si désiré, être transformé en thioamide par action notamment du réactif de LAWESSON dans le toluène,
f) Les éventuelles fonctions carboxy libre ou estérifié des produits décrits ci-dessus peuvent être, si désiré, réduites en fonction alcool par les méthodes connues de l'homme de métier : les éventuelles fonctions carboxy estérifié peuvent être, si désiré, réduites en fonction alcool par les méthodes connues de l'homme du métier et notamment par de l'hydrure de lithium et d'aluminium dans un solvant tel que par exemple le tétrahydrofuranne ou encore le dioxane ou l'éther éthylique.
   Les éventuelles fonctions carboxy libre des produits décrits ci-dessus peuvent être, si désiré, réduites en fonction alcool notamment par de l'hydrure de bore.
g) Les éventuelles fonctions alcoxy telles que notamment méthoxy des produits décrits ci-dessus peuvent être, si désiré, transformées en fonction hydroxyle dans les conditions usuelles connues de l'homme du métier par exemple par du tribromure de bore dans un solvant tel que par exemple le chlorure de méthylène, par du bromhydrate ou chlorhydrate de pyridine ou encore par de l'acide bromhydrique ou chlorhydrique dans de l'eau ou de l'acide trifluoro acétique au reflux.
h) Les éventuelles fonctions alcool des produits décrits ci-dessus peuvent être, si désiré, transformées en fonction aldéhyde ou acide par oxydation dans les conditions usuelles connues de l'homme du métier telles que par exemple par action de l'oxyde de manganèse pour obtenir les aldéhydes ou du réactif de Jones pour accéder aux acides.
i) j) Les réactions de transformation de radical formyle en radical carbamoyle et de radical carbamoyle en radical nitrile, sont réalisées notamment pour R₃ et R₄ selon les conditions usuelles connues de l'homme du métier, telles que par exemple passage par le céto nitrile et déplacement par une amine (Chem. Comm. 1971, p. 733).
k) Les éventuelles fonctions nitrile des produits décrits ci-dessus peuvent être, si désiré, transformées en tétrazolyle dans les conditions usuelles connues de l'homme du métier telles que par exemple par cycloaddition d'un azidure métallique tel que par exemple l'azidure de sodium ou un azidure de trialkylétain sur la fonction nitrile ainsi qu'il est indiqué dans la méthode décrite dans l'article référencé comme suit :
   J. Organometallic Chemistry., 33, 337 (1971) KOZIMA S.& coll.
l) Les éventuels groupements alkylthio ou arylthio des produits décrits ci-dessus peuvent être, si désiré, transformés en les fonctions sulfoxyde ou sulfone correspondantes dans les conditions usuelles connues de l'homme du métier telles que par exemple par les peracides comme par exemple l'acide peracétique ou l'acide métachloroperbenzoïque ou encore par l'ozone, l'oxone, le périodate de sodium dans un solvant tel que par exemple le chlorure de méthylène ou le dioxanne à la température ambiante.
   L'obtention de la fonction sulfoxyde peut être favorisée par un mélange équimolaire du produit renfermant un groupement alkylthio ou arylthio et du réactif tel que notamment un peracide.
   L'obtention de la fonction sulfone peut être favorisée par un mélange du produit renfermant un groupement alkylthio ou arylthio avec un excés du réactif tel que notamment un peracide.
m) Les éventuelles fonctions sulfure, sulfoxyde ou sulfone des produits décrits ci-dessus peuvent être, si désiré, transformées en les fonctions sulfoximine correspondantes dans les conditions usuelles connues de l'homme du métier : des exemples non exhaustifs de préparation de produits renfermant une fonction sulfoximine sont décrites ci-dessous.
   Ainsi par exemple pour la préparation de composés tels que les N-(arylsulfonyl) sulfoximines et par exemple dans le cas où le groupement aryle est un radical toluène, la sulfoximine peut être obtenue par action de l'azoture de paratoluènesulfonyle sur le sulfoxyde correspondant soit -S(O)CH₃ de préférence en présence de cuivre ainsi qu'il est indiqué, par exemple, dans la référence suivante : J. A. C. S., 95, pp. 4287 (1973) JOHNSON C.R. & coll.
   Une autre méthode également utilisée consiste à traiter la N-tosylsulfilimine, elle-même préparée à partir du sulfure par action, par exemple, de la chloramine "T", par un agent oxydant comme par exemple, l'hypochlorite de sodium dans des conditions de transfert de phase ainsi qu'il est indiqué, par exemple, dans la référence suivante : J. Org. Chem., 49, pp. 2282 (1984) AKUTAGAWA K.& coll.
n) la réaction de transformation de fonction oxo en fonction thioxo peut être réalisée notamment par le réactif de LAWESSON dans les conditions définies ci-dessus.
o) La réaction de déshydratation d'un radical hydroxylalkyle en radical alkényle, peut notamment être réalisée au moyen d'un acide tel que l'acide chlorhydrique concentré ou l'acide sulfurique dans un alcool ou le dioxanne.
p) La réaction de transformation de fonction acide en fonction tétrazolylcarboxy peut être réalisée par exemple par transformation préalable de la fonction acide en chlorure d'acide ainsi qu'il est indiqué ci-dessus, puis par action de cyanure cuivreux, selon les conditions usuelles connues de l'homme du métier sur le chlorure d'acide ainsi obtenu, on obtient ainsi le radical 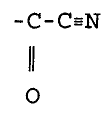 que l'on peut transformer en radical 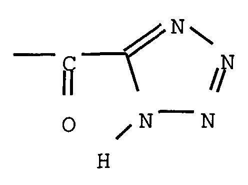 par exemple par action du composé Sn(Bu)₃N₃ dans le toluène,
q) la réaction de transformation de la fonction β céto sulfoxyde en fonction α céto thioester, peut être réalisée par bromation en α du cétosulfoxyde par exemple par action du N-bromosuccinimide dans par exemple le chlorure de méthylène puis par une réaction de PUMMERER réalisée dans un mélange d'acide trifluoroacétique et de chlorure de méthylène ou encore un mélange d'acide sulfurique et de dioxanne.
   Notamment, ainsi qu'il est défini ci-dessus en c) et q), on peut réaliser le schéma réactionnel suivant : 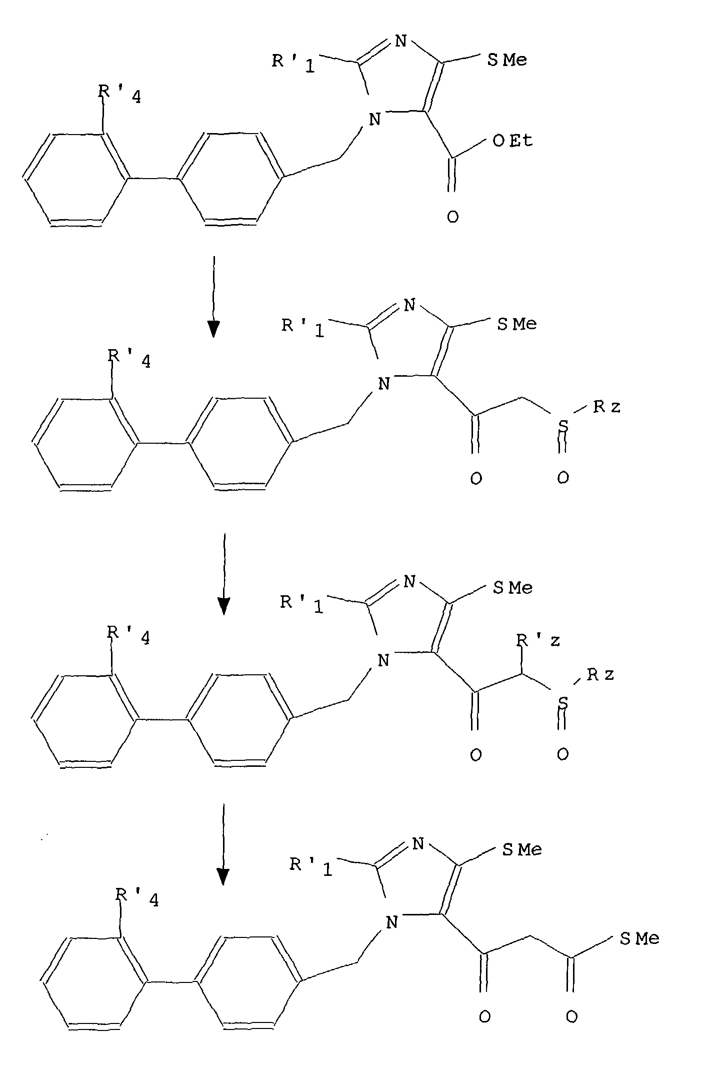 composés dans lesquels R'₁ et R'₄ ont les significations indiquées ci-dessus, et Rz et R'z identiques ou différents représentent un radical alkyle ou aryle éventuellement substitués ainsi qu'il est indiqué ci-dessus.
   Une illustration de ce schéma réactionnel est donnée ci-après dans la partie expérimentale à la préparation 4.
r) la réaction de transformation d'un carbamate en urée et notamment d'un sulfonylcarbamate en sulfonylurée, peut être réalisée par exemple au reflux d'un solvant comme par exemple le toluène en présence de l'amine adéquate.
s) L'élimination de groupements protecteurs tels que par exemple ceux indiqués ci-dessus peut être effectuée dans les conditions usuelles connues de l'homme de métier notamment par une hydrolyse acide effectuée avec un acide tel que l'acide chlorhydrique, benzène sulfonique ou para-toluène sulfonique, formique ou trifluoroacétique ou encore par une hydrogénation catalytique.
   Le groupement phtalimido peut être éliminé par l'hydrazine.
   On trouvera une liste de différents groupements protecteurs utilisables par exemple dans le brevet BF 2 499 995.
t) Les produits décrits ci-dessus peuvent, si désiré, faire l'objet de réactions de salification par exemple par un acide minéral ou organique ou par une base minérale ou organique selon les méthodes usuelles connues de l'homme du métier.
u) Les éventuelles formes optiquement actives des produits décrits ci-dessus peuvent être préparées par dédoublement des racémiques selon les méthodes usuelles connues de l'homme du métier.

Des illustrations de telles réactions définies ci-dessus sont données dans la préparation des exemples décrits ci-après.

Certains produits de départ de formules (II), (IX) et (XX) sont connus et peuvent être préparés par exemple comme indiqué dans le brevet européen EP 168 950.

D'autres produits de départ de formules (II), (IX) et (XX) peuvent en particulier être préparés comme indiqué dans le brevet européen EP 0465368 ou dans les préparations 1 à 5 décrites ci-après.

Certains produits de départ de formules (II), (IX) et (XX) sont commerciaux tels que par exemple :
les produits de formule (II) suivants :
   - le 2-phénylimidazole
   - le 2-méthoxyméthylimidazole
   - le 2-propylimidazole
   - le 2-isopropylimidazole
   - le 2-éthylimidazole
   - le 2-méthylimidazole
les produits de formule (IX) suivants :
   - le 4-méthyl 2-phénylimidazole
   - le 2,4-diméthylimidazole
   - le 2-éthyl 4-méthylimidazole.

Des exemples de produits commerciaux de formule (XX) sont donnés dans les brevets EP 0465368 ou EP 0503162.

On peut encore notamment préparer certains produits de formules (II), (IX) et (XX) à partir d'autres produits de formule (II) ou (IX) par exemple en les soumettant à une ou plusieurs des réactions décrites aux points a) à u) ci-dessus réalisées comme indiquées ci-dessus.

Certains produits de formules (IX) et (XX) peuvent également être obtenus par monohalogénation du produit de formule (II) tel que défini ci-dessus en produit de formule ^{(P}1^{) :} 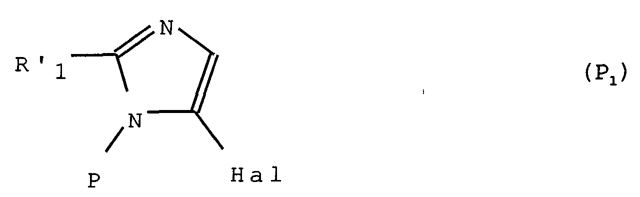 dans lequel R'₁ et P ont les significations indiquées ci-dessus pour le produit de formule (II), produit de formule (P₁) que l'on peut faire réagir après échange suivant la réaction halogène métal connue de l'homme du métier avec l'électrophile adapté, suivant les méthodes connues de l'homme du métier et notamment ainsi qu'il a été décrit ci-dessus pour passer du produit de formule (III) au produit de formule (V). Par le même processus, certains produits de formules (IX) et (XX) peuvent également être obtenus à partir du produit de formule (III) telle que définie ci-dessus. On peut encore noter que le produit de formule : 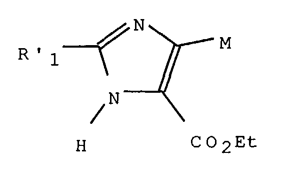 dans laquelle R'₁ et M ont les significations indiquées ci-dessus, décrit dans EP 0465368, peut être soumis à une réaction de saponification thermique puis à une décarboxylation pour obtenir un produit de formule (IX) telle que définie ci-dessus.

Une telle illustration est donnée dans la partie expérimentale décrite ci-après.

Les produits de formule (III) dans lesquels R'₁ représente un radical alkylthio peuvent être obtenus soit à partir du produit de formule (II) tel que défini ci-dessus ainsi qu'il est décrit ci-dessus, soit à partir de produits commerciaux tels que notamment le 2,4,5-tribromoimidazole ou le 4,5-dibromo 2-phénylimidazole, ainsi qu'il a été décrit ci-dessus pour passer du produit de formule (III) au produit de formule (V).

Les composés de départ de formule (VIII) peuvent être disponibles dans le commerce ou peuvent être préparés selon les méthodes usuelles connues de l'homme du métier.

Les produits de départ de formules (IVₐ), (IV_{b}), (IV_{c}), (IV_{d}), (IVₑ), (XII) et (XV) sont commerciaux notamment les produits de formule (IVₐ) comme le sec-butyl disulfide, l'éthyl disulfide, l'isopropyl disulfide, le méthyl disulfide, le benzyl disulfide, le phényl disulfide, le propyl disulfide,
le produit de formule (IV_{b}) comme le méthyl méthanethiosulfonate,
les produits de formule (IV_{c}) comme le méthyl chloroformate, le benzyl chloroformate, l'isobutyl chloroformate, l'éthyl chloroformate, le N-propyl chloroformate,
les produits de formule (IV_{d}) comme le diméthyl carbonate, le diéthyl carbonate,
les produits de formule (IVₑ) comme le di-tert-butyl oxalate, le diéthyl oxalate, le diméthyl oxalate,
les produits de formule (XII) comme l'éthyl thiophène 2-glyoxylate, l'éthyl 3-méthyl 2-oxobutyrate, l'éthyl phényl glyoxylate, le méthyl pyruvate, le méthyl benzoylformate, les produits de formule (XV) comme le méthyl isocyanate, le 2-carbométhoxyphényl isocyanate, le benzyl isocyanate, le cyclohexyl isocyanate, le N-propyl isocyanate, l'allyl isocyanate, le phényl isocyanate.

Un procédé de préparation de certains produits de formule (VIII) est notamment décrit dans le brevet européen EP 0465368.

Des exemples de préparation de composés de formule (VIII) sont également décrits dans la littérature et des exemples en sont donnés notamment dans le brevet US 4,880,804 ou par exemple dans la référence Chemistry and Industry 7 september 1987 HOWARD and COLQUHOUN pp. 612-617.

Les composés de formule (I_{B}) tels que définis ci-dessus ainsi que leurs sels d'addition avec les acides présentent d'intéressantes propriétés pharmacologiques.

Les produits sont doués de propriétés antagonistes pour le récepteur à l'angiotensine II et sont ainsi notamment inhibiteurs des effets de l'angiotensine II, en particulier de l'effet vasoconstricteur et également de l'effet trophique au niveau des myocytes.

Ces propriétés justifient leur application en thérapeutique et l'invention a également pour objet à titre de médicaments, les produits de formule (I_{B}) telle que définie ci-dessus, lesdits produits de formule (I_{B}) étant sous toutes les formes isoméres possibles racémiques ou optiquement actives, ainsi que les sels d'addition avec les acides minéraux ou organiques pharmaceutiquement acceptables desdits produits de formule (I_{B}).

L'invention a plus particulièrement pour objet, à titre de médicaments, les produits décrits ci-aprés dans les exemples et notamment les produits de formule (I_{B}) suivants :
- Acide 2-butyl α-hydroxy α-méthyl 4-(méthylthio) 1-[(2'((((phénylméthoxy) carbonyl) amino) sulfonyl) (1,1'-biphényl) 4-yl) méthyl] 1H-imidazole 5-acétique,
- Acide 2-butyl α-hydroxy α-méthyl 4-(méthylthio) 1-[(2'((((propylamino) carbonyl) amino) sulfonyl) (1,1'-biphényl) 4-yl) méthyl] 1H-imidazole 5-acétique,
- Acide 2-butyl α-hydroxy α-méthyl 4-(méthylthio) 1-[(2'((((phénylméthyl) amino) carbonyl) amino) sulfonyl) (1,l'biphényl) 4-yl) méthyl] 1H-imidazole 5-acétique,
- Acide 2-butyl 1-[(2'-(((((cyclohexylméthyl) amino) carbonyl) amino) sulfonyl) (1,1'-biphényl) 4-yl) méthyl] α-hydroxy α-méthyl 4-(méthylthio) 1H-imidazole 5-acétique,
- Acide 1-[(2'-(((((phénylméthyl) amino) sulfonyl) (1,1'-biphényl) 4-yl) méthyl] α-hydroxy 4-(méthylthio) 2-propyl 1H-imidazole 5-acétique,
- 1-[(2'-(((((cyclohexylméthyl) amino) carbonyl) amino) sulfonyl) (1,1'-biphényl) 4-yl) méthyl] 4-(méthylthio) 2-propyl 1H-imidazole 5-carboxylate de sodium,
- Acide 4-(méthylthio) 1-[(2'-(((((phénylméthyl) amino) carbonyl) amino) sulfonyl) (1,1'-biphényl) 4-yl) méthyl] 2-propyl 1H-imidazole 5-carboxylique,
- α-butyl α-hydroxy 4-(méthylthio) 1-[(2'-(((((phénylméthyl) amino) carbonyl) amino) sulfonyl) (1,1'-biphényl) 4-yl) méthyl] 2-propyl 1H-imidazole 5-acétate de sodium,
- Acide α-hydroxy 4-(méthylthio) α-phényl 1-((2'-(((((phénylméthyl) amino) carbonyl) amino) sulfonyl) (1,1'-biphényl) 4-yl) méthyl) 2-propyl 1H-imidazole 5-acétique,
- Acide 1-((2'-(((((cyclohexylméthyl) amino) carbonyl) amino) sulfonyl) (1,1'-biphényl) 4-yl) méthyl) 4-(difluorométhyl) thio) 2-propyl 1H-imidazole 5-carboxylique,
- Acide 4-((difluorométhyl) thio) 1-((2'-(((((phénylméthyl) amino) carbonyl) amino) sulfonyl) 1,1'-biphényl) 4-yl) méthyl) 2-propyl 1H-imidazole 5-carboxylique,
- Acide 4-((difluorométhyl) thio) 2-propyl 1-((2'-(((((2-thiénylméthyl) amino) carbonyl) amino) sulfonyl) (1,1'- biphényl) 4-yl) méthyl) 1H-imidazole 5-carboxylique, ainsi que leurs sels pharmaceutiquement acceptables.

Les médicaments, objet de l'invention, peuvent être utilisés dans le traitement des affections cardiovasculaires associées à une altération de la vasomotricité ou de la volémie : infarctus du myocarde et ses conséquences, insuffisance cardiaque, insuffisance rénale, angine de poitrine, hyperaldostéronisme, hypertension artérielle et ses conséquences. Ces médicaments, objet de l'invention, pourraient également être utilisés pour le traitement du glaucome, de l'athérosclérose et de différents types de spasmes viscéraux, ainsi qu'à titre de substances protectrices neuronales ou encore dans la prévention des resténoses post-angioplastie.

Notamment les médicaments, objet de l'invention peuvent être utilisés pour leurs effets anti-hypertrophique et anti- fibrotique aux niveaux cardiaque et vasculaire. Tout particu- lièrement, ils peuvent être utilisés pour le traitement et la prévention des désordres cardiovasculaires associés au diabète.

Ils peuvent également être utilisés dans le traitement de certains désordres gastro-intestinaux, gynécologiques et en particulier pour un effet relaxant au niveau de l'utérus.

Les médicaments, objet de l'invention peuvent également être utilisés dans le traitement des troubles de la mémoire et des fonctions cognitives, ainsi que de l'anxiété.

L'invention s'étend aux compositions pharmaceutiques contenant à titre de principe actif l'un au moins des médicaments tels que définis ci-dessus.

Les produits de formule (I_{B}) de la présente invention ont donc une affinité non seulement pour le récepteur AT₁ mais également pour le récepteur AT₂ de l'Angiotensine II.

L'invention a donc particulièrement pour objet l'utili- sation des produits de formule (I_{B}) telle que définie ci-dessus, pour la préparation de compositions pharmaceutiques destinées au traitement d'affections résultant d'une stimulation anormale des récepteurs AT₁ et/ou AT₂ de l'Angiotensine II.

L'avantage que présentent de tels produits de formules (I), (I_{A}) et (I_{B}) qui possèdent une affinité à la fois pour le récepteur AT₁ et pour le récepteur AT₂ est par le blocage simultané de ces deux récepteurs, d'augmenter l'efficacité c'est-à-dire les effets protecteurs, résultant de l'adminis- tration de tels produits vis-àvis des organes touchés notamment le coeur, les vaisseaux et les reins et également d'élargir les indications notamment pour les organes non cardiovasculaires tels que l'appareil urogénital.

Les taux circulants de l'Angiotensine II étant augmentés par un phénomène de rétro-contrôle lors du blocage du récepteur AT₁, le blocage simultané du récepteur AT₂ permettrait une meilleure efficacité à long terme dans le traitement notamment de l'hypertension artérielle et la prévention des complications en particulier les hypertrophies cardiaques et vasculaires ainsi que le développement de fibroses au niveau des organes cibles.

On peut également souligner l'amélioration des propriétés cognitives des produits montrant une affinité à la fois pour les récepteurs AT₁ et AT₂.

L'invention a ainsi plus particulièrement pour objet l'utilisation des produits de formules (I), (I_{A}) et (I_{B}) telles que définies ci-dessus, pour la préparation de compositions pharmaceutiques destinées au traitement de l'hyper- tension artérielle, de l'insuffisance cardiaque et des resténoses post-angioplastie.

L'invention a tout particulièrement pour objet l'utili- sation des produits de formules (I), (I_{A}) et (I_{B}) telles que définies ci-dessus, pour la préparation de compositions pharmaceutiques destinées au traitement de l'insuffisance rénale.

L'invention a également pour objet l'utilisation des produits de formules (I), (I_{A}) et (I_{B}) telles que définies ci-dessus, pour la préparation de compositions pharmaceutiques destinées au traitement et à la prévention des désordres cardiovasculaires associés au diabète.

Les compositions pharmaceutiques indiquées ci-dessus peuvent être administrées par voie buccale, rectale, par voie parentérale ou par voie locale en application topique sur la peau et les muqueuses ou par injection par voie intraveineuse ou intramusculaire.

Ces compositions peuvent être solides ou liquides et se présenter sous toutes les formes pharmaceutiques couramment utilisées en médecine humaine comme, par exemple, les comprimés simples ou dragéifiés, les gélules, les granulés, les suppositoires, les préparations injectables, les pommades, les crèmes, les gels et les préparations en aérosols ; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie usuelle, variable selon le produit utilisé, le sujet traité et l'affection en cause, peut être, par exemple, de 1 à 100 mg par jour chez l'adulte, par voie orale.

Les exemples suivants illustrent l'invention sans toutefois la limiter.

### PREPARATION 1 : 2-butyl α-hydroxy α-méthyl 1-[(2'-((((diméthylamino) méthylène) amino) sulfonyl) (1,1'-biphényl) 4-yl) méthyl] 4-(méthylthio) 1H-imidazole 5-acétate d'éthyle

### Stade A : 4'-[(2-butyl 4-(méthylthio) 1H-imidazol 1-yl) méthyl] N-[(diméthylamino) méthylène] (1,1'-biphényl) 2-sulfonamide

### a) 2-butyl 4-(méthylthio) 1H-imidazole

On introduit 760 mg de 2-n-butyl 4-méthylthio imidazole 5-carboxylate d'éthyle, préparé ainsi qu'il est décrit dans la demande de brevet européen EP 0465368, dans 15 cm³ de NaOH(2N). On porte à reflux et agite durant 24 heures. Après refroidissement, on dilue par 50 cm³ H₂O, extrait par 3 x 20 cm³ CH₂Cl₂, lave par 20 cm³ H₂O et sèche. On obtient 535 mg de produit attendu.
F = 64°C.

| Spectre IR (CHCl₃) | |
|---|---|
| Absence de C=0 | |
| =C-NH | 3452 cm⁻¹ |
| Système conjugué | 1596 - 1502 cm⁻¹ |

### b) 4'-[(2-butyl 4-(méthylthio) 1H-imidazol 1-yl) méthyl] N-[(diméthylamino) méthylène] (1,1'-biphényl) 2-sulfonamide

On dissout 5 g de 2-butyl 4-(méthylthio) 1H-imidazole dans 120 cm³ de THF. Puis on ajoute lentement à la solution orangée obtenue 1,55 g d'hydrure de sodium à 50 % en dispersion dans l'huile. La température s'élève jusqu'à 25° C. On agite 30 mn à cette température puis on introduit 14 g de 4'bromométhyl N-[(diméthylamino) méthylène] (1,1'-biphényl) 2-sulfonamide. On agite à température ambiante jusqu'à fin d'évolution, soit pendant environ 3 heures. On reprend à l'eau, extrait à l'acétate d'éthyle, sépare par chromatographie sur silice en éluant à l'acétate d'éthyle puis empâte à l'éther iso, filtre et sèche. On obtient ainsi 9,35 g du produit attendu (cristaux incolores). PF = 148° C.

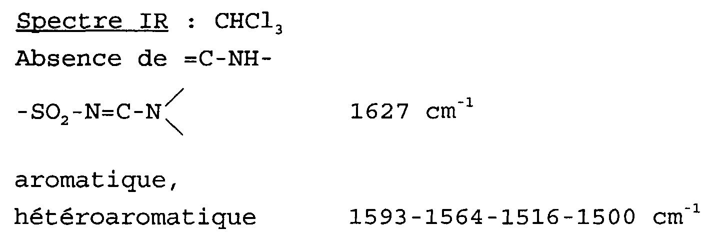

### Stade B : 4'-[(5-bromo 2-butyl 4-(méthylthio) 1H-imidazol 1-yl) méthyl] N-[(diméthylamino) méthylène] (1,1'-biphényl) 2-sulfonamide

On dissout 10,4 g du produit obtenu au stade A, ci-dessus, dans 450 cm³ de CH₂Cl₂ et ajoute 3,9 g de N-bromosuccinimide.

On agite environ 15 mn à température ambiante, lave à l'eau et à l'eau salée, décante, sèche, filtre et chasse le solvant sous vide à 50°C.

On empâte à l'éther iso, filtre et sèche. On obtient 11,8 g de produit attendu (cristaux incolores) F = 158°C.

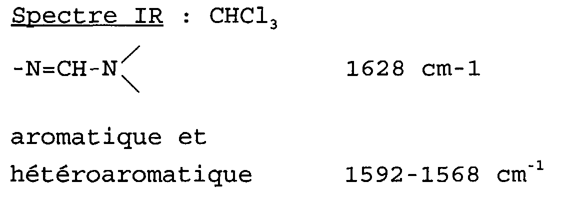

**Microanalyse :**

| | Br |
|---|---|
| % calculé | 14,54 |
| % trouvé | 14,4-14,7 |

### Stade C : 2-butyl α-hydroxy α-méthyl 1-[(2'-((((diméthylamino) méthylène) amino) sulfonyl) (1,1'-biphényl) 4-yl) méthyl] 4-(méthylthio) 1H-imidazole 5-acétate d'éthyle

On dissout 11,8 g du produit obtenu au stade B ci-dessus dans 160 cm³ de THF. Puis on ajoute sans dépasser 25°C, 17,5 cm³ d'une solution 1M de chlorure d'isopropyl magnésium en solution dans l'éther. Après environ 30 mn d'agitation à température ambiante, on ajoute lentement 4 cm³ de pyruvate d'éthyle. On agite environ 1 heure à température ambiante, ajoute 1 cm³ de pyruvate d'éthyle et poursuit l'agitation pendant encore environ 1 heure.

On reprend par 200 cm³ de NH₄Cl en solution à 10 % et extrait à l'acétate d'éthyle. On sèche, concentre jusqu'à ~ 200 cm³, filtre et sèche les cristaux obtenus.

On obtient 6 g de produit attendu (cristaux incolores).
F = 208-210°C.

| Spectre IR : CHCl₃ | |
|---|---|
| OH complexe | ∼3530 cm⁻¹ |
| C=O | 1722 cm⁻¹ |
| C=N | 1626 cm⁻¹ |
| Hétérocycle + aromatique | 1565, 1518 cm⁻¹ |

| Spectre UV : | |
|---|---|
| 1) Dans EtOH | |
| infl. 274 nm | ∈= 3100 |
| infl. 231 nm | ∈ = 23000 |

| 2) Dans EtOH - HCl N/10 | |
|---|---|
| infl. 228 nm | ∈= 30000 |
| infl. 273 nm | ∈ = 3400 |

### PREPARATION 2 : 2-méthyl 4-(méthylthio) 1-[(2'-((((diméthylamino) méthylène) amino) sulfonyl) (1,1'-biphényl) 4-yl) méthyl] α-oxo 1H-imidazole 5-acétate d'éthyle

### Stade A : 4,5-dibromo 2-méthyl 1-[(2-(triméthylsilyl) éthoxy) méthyl] 1H-imidazole

Le produit est obtenu par 2 réactions consécutives.

### 1) Etape de protection de la 2-méthyl imidazole

On dissout 6,8 g de 2-méthyl imidazole dans 250 cm³ de THF et on ajoute par petites fractions 4 g d'hydrure de sodium à 50 % dans l'huile. La réaction étant exothermique, on maintient la température du milieu à température ambiante durant environ 30 mn.

On ajoute alors goutte à goutte dans le milieu réactionnel 17,5 cm³ de chlorure de SEM. Après environ 20 mn d'agitation à 20°C, on hydrolyse l'excès d'hydrure de sodium par addition de THF à 20 % H₂O.

La solution obtenue est amenée à sec, reprise par de l'acétate d'éthyle et lavée par de l'eau. La phase organique ainsi recueillie est séchée. On obtient une huile jaune qui est purifiée par chromatographie sur silice avec pour éluant : CH₂Cl₂-méthanol (90-10).

On obtient ainsi 14 g de produit protégé.

### 2) Bromation

Le produit protégé obtenu ci-dessus est dissout dans 250 cm³ de CH₂Cl₂. On ajoute ensuite à cette solution 25 g de N-bromo succinimide par petites fractions.

L'agitation est maintenue environ 30 mn à température ambiante. La phase organique est lavée par une solution de bicarbonate de sodium, puis abondamment à l'eau. Après séchage et évaporation, on récupère 13,4 g de produit attendu (huile jaune homogène).

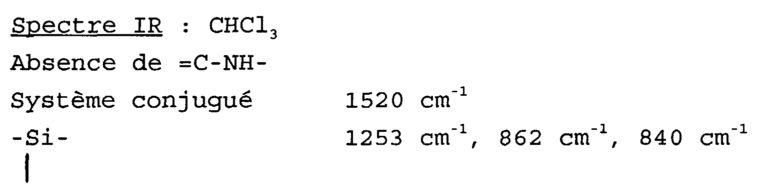

### Stade B : 2-méthyl 1-[(2-(triméthylsilyl) éthoxy) méthyl] 5-(méthylthio) α-oxo 1H-imidazole 4-acétate d'éthyle

On dissout, sous atmosphère anhydre, 3 g du produit obtenu au stade A ci-dessus, dans 20 cm³ de THF anhydre. Cette solution est alors refroidie à -78°C et on y ajoute, tout en maintenant la température à -78°C, 5,6 cm³ de n-butyl lithium 1,5 molaire dans l'hexane. L'agitation est maintenue à -78°C pendant environ 10 mn.

On introduit ensuite 0,76 cm³ de diméthyl disulfure, puis laisse remonter lentement la température jusqu'à 20°C et agite pendant environ 30 mn.

Le milieu réactionnel est de nouveau refroidi à -78°C, et on y ajoute comme précédemment 5,6 cm³ de n-butyl lithium 1,5 M dans l'hexane. Après environ 10 mn d'agitation à -78°C, on introduit en une fois 5,5 cm³ d'oxalate de diéthyle. L'agitation est maintenue à température ambiante pendant environ 30 mn. Le milieu réactionnel est alors versé sur de l'eau glacée. On extrait par de l'acétate d'éthyle, lave la phase organique par une solution de bicarbonate de sodium puis par de l'eau et sèche. On récupère une huile brune, que l'on purifie par chromatographie sur silice avec pour éluant : acétate d'éthyle-cyclohexane (50-50). On obtient 1,63 g de produit attendu (huile).

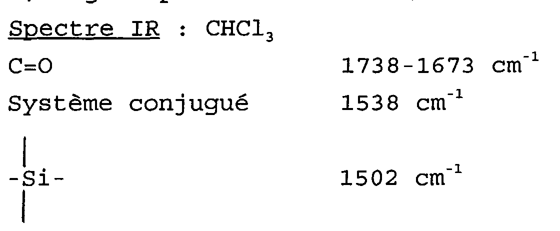

### Stade C : 2-méthyl 5-(méthylthio) α-oxo 1H-imidazole 4-acétate d'éthyle

On dissout 1,6 g du produit obtenu au stade B ci-dessus dans 30 cm³ de CH₂Cl₂, et ajoute 10 cm³ d'acide trifluoroacétique. Le milieu réactionnel est alors porté au reflux pendant environ 10 h. La solution est ensuite amenée à sec, et le résidu repris par de l'eau. On alcaline la phase aqueuse par addition de bicarbonate de sodium, extrait par de l'acétate d'éthyle, lave à l'eau puis sèche et récupère 920 mg de produit attendu (huile jaune) utilisé tel quel dans la suite de la synthèse.

| Spectre IR : CHCl₃ | |
|---|---|
| =C-NH | 3415 cm⁻¹ |
| C=O | 1716-1633 cm⁻¹ |
| Système conjugué | 1530-1502 cm⁻¹ |

### Stade D : 2-méthyl 4-(méthylthio) 1-[(2'-((((diméthylamino) méthylène) amino) sulfonyl) (1,1'-biphényl) 4-yl) méthyl] α-oxo 1H-imidazole 5-acétate d'éthyle

On dissout 880 mg du produit obtenu au stade C ci-dessus dans 10 cm³ de DMF/anhydre et ajoute successivement 800 mg de carbonate de potassium puis 2,2 g de 4'-bromométhyl N-[(diméthylamino) méthylène] (1,1'-biphényl) 2-sulfonamide. L'agita- tion est maintenue pendant environ 3 H à température ambiante. La suspension jaune ainsi obtenue est versée sur de l'eau. On extrait par de l'acétate d'éthyle, lave à l'eau puis sèche et récupère une résine jaune que l'on purifie sur silice avec pour éluant l'acétate d'éthyle.

On obtient ainsi 1,17 g de produit attendu.

| Spectre IR : CHCl₃ | |
|---|---|
| Absence de =C-NH | |
| C=O ester | 1735 cm⁻¹ |
| Autre C=O | 1629 cm⁻¹ (F) |
| C=N | |
| Aromatique | 1570 cm⁻¹ |
| hétéroatome | 1516 cm⁻¹ |

### PREPARATION 3 : 1-[(2'-(aminosulfonyl) (1,1'-biphényl) 4-yl) méthyl] 4-(méthylthio) α-oxo 2-propyl 1H-imidazole 5-acétate d'éthyle

### Stade A : 4,5-dibromo 2-n-propyl 1-[(2-(triméthylsilyl) éthoxy) méthyl] 1H-imidazole

On procède comme au stade A de la préparation 2, en remplaçant 2-méthyl 1H-imidazole par le 2-propyl 1H-imidazole.

Le produit est obtenu par 2 réactions consécutives.

### 1) Etape de protection de la 2-propyl imidazole

On dissout 30 g de 2-propyl imidazole dans 500 cm³ de THF et on ajoute par petites fractions 12,5 g d'hydrure de sodium à 50 % dans l'huile.

On ajoute alors dans le milieu réactionnel 53 cm³ de chlorure de SEM, on hydrolyse par du THF à 20 % H₂O.

On obtient ainsi 57,3 g de produit protégé.

### 2) Bromation

Le produit protégé obtenu ci-dessus est dissous dans 500 cm³ de CH₂Cl₂. On ajoute 93,5 g de N-bromo succinimide et obtient 93,7 g de produit attendu.

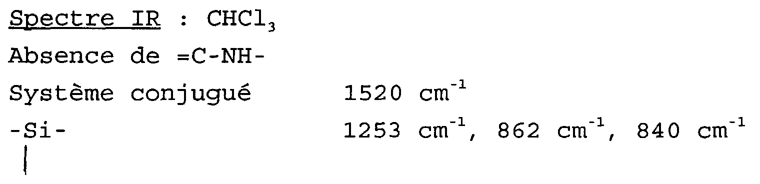

### Stade B : 2-n-propyl 1-[(2-(triméthylsilyl) éthoxy) méthyl 5-(méthylthio) α-oxo 1H-imidazole 4-acétate d'héthyle

On introduit, sous atmosphère anhydre, 36,7 g du produit obtenu au stade A ci-dessus, dans 200 cm³ de THF, on ajoute à -78°C 63,7cm³ de n-butyl lithium 1,5 Molaire dans l'hexane puis 8,62 cm³ de diméthyl disulfure, on laisse remonter à température ambiante. Puis on ajoute comme précédemment à -78°C 63,7 cm³ de n-butyl lithium 1,5 Molaire dans l'hexane suivi de 55 cm³ d'oxalate d'éthyle. On obtient 11,75 g de produit attendu.

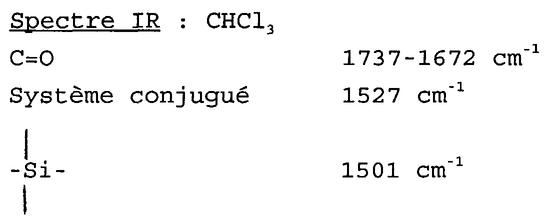

### Stade C : 2-n-propyl 5-(méthylthio) α-oxo 1H-imidazole 4-acétate d'éthyle

On procède comme au stade C de la préparation 2 à partir de 11 g du produit obtenu au stade B ci-dessus dans 200 cm³ de CH₂Cl₂, et 40 cm³ d'acide trifluoroacétique. On obtient 7,15 g de produit attendu.

| Spectre IR : CHCl₃ | |
|---|---|
| =C-NH | 3413 cm⁻¹ |
| C=O | 1714-1633 cm⁻¹ |
| Système conjugué | 1524-1492 cm⁻¹ |

### Stade D : 2-n-propyl 4-(méthylthio) 1-[(2'-((((diméthylamino) méthylène) amino) sulfonyl) (1,1'-biphényl) 4-yl) méthyl] α-oxo 1H-imidazole 5-acétate d'éthyle

On procède comme au stade D de la préparation 2 à partir de 7 g du produit obtenu au stade C ci-dessus dans 100 cm³ de DMF et 7,5 g de carbonate de potassium et 15,6 g de 4'-(bromométhyl) N-[(diméthylamino) méthylène] (1,1'-biphényl) 2-sulfonamide. On obtient ainsi 7,83 g de produit attendu.

| Spectre IR : CHCl₃ | |
|---|---|
| Absence de =C-NH | |
| C=O ester | 1735 cm⁻¹ |
| Autre C=O | 1630 cm⁻¹ (F) |
| C=N | |

### Stade E : 1-[(2'-(aminosulfonyl) (1,1'-biphényl) 4-yl) méthyl] 2-n-propyl 4-(méthylthio) α-oxo 1H-imidazole 5-acétate d'éthyle

On procède comme au stade A de l'exemple 2 à partir de 7,8 g du produit obtenu au stade D ci-dessus, dans 100 cm³ d'éthanol et 30 cm³ de HCl concentré et obtient ainsi 3,6 g de produit attendu.

| Spectre IR CHCl₃ | |
|---|---|
| -NH₂ | 3443-3343 cm⁻¹ |
| C=O | 1734-1627 cm⁻¹ |
| Aromatique | 1593 cm⁻¹ |
| Hétéroatome | 1565 cm⁻¹ |
| NH₂ def. | 1542 cm⁻¹ |

### PREPARATION 4 : 1-[(2'-(aminosulfonyl) (1,1'-biphényl) 4-yl) méthyl] 2-butyl 4-(méthylthio) α-oxo 1H-imidazole 5-éthane thioate de S-méthyle

### Stade A : 4'-[(2-butyl 5-((méthylsulfinyl) acétyl) 4-(méthylthio) 1H-imidazol 1-yl) méthyl] (1,1'-biphényl) 2-sulfonamide

On prépare d'abord l'anion du DMSO en introduisant 3,36 g d'hydrure de sodium à 50 % dans l'huile. Le NaH est débarrassé de son huile par 3 lavages successifs au pentane. Puis on sèche et on ajoute 70 cm³ de diméthylsulfoxyde anhydre et le mélange est porté à 75°C, pendant environ 1 H. On baisse alors la température à 0°C et ajoute à l'anion du DMSO ainsi formé 70 cm³ de THF anhydre et 9,5 g de 2-butyl 1-[(2'((((diméthylamino) méthylène) amino) sulfonyl) (1,l'-biphényl) 4-yl) méthyl] 4-(méthylthio) 1H-imidazole 5-carboxylate d'éthyle, préparé ainsi qu'il est indiqué dans la demande de brevet européen EP 0503162, dissous dans 70 cm³ de THF anhydre. Puis on laisse remonter à température ambiante et agite pendant environ 1/2 H. Le mélange réactionnel est alors versé dans 400 cm³ H₂O.

La solution est acidifiée jusqu'à pH 2 avec HCl 2N. On extrait par 4 x 200 cm³ de chlorure de méthylène et lave la phase organique par 4 x 100 cm³ d'H₂O distillée. On sèche la phase organique, filtre et évapore.

On purifie sur silice avec pour éluant CH₂Cl₂-méthanol (9-1) et récupère 7,5 g de produit attendu.

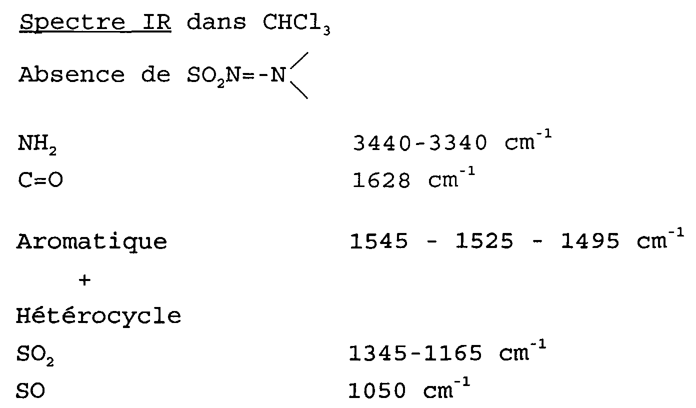

### Stade B : 4'-[[5-[bromo (méthylsulfinyl) acétyl] 2-butyl 4-(méthylthio) 1H-imidazol 1-yl] méthyl] (1,1'-biphényl) 2-sulfonamide

On introduit 1 g du produit obtenu au stade A, ci-dessus et 530 mg de K₂CO₃. Puis on ajoute 10 cm³ de CH₂Cl₂ anhydre, porte la température du milieu à 0°C et ajoute goutte à goutte 342 mg de N-bromo succinimide, dissout dans le minimum de CH₂Cl₂ anhydre. On ajoute alors 100 cm³ de CH₂Cl₂ et lave la phase organique par 3 x 200 cm³ d'eau distillée, et 1 x 100 cm³ de NaCl saturée. On sèche, filtre et concentre à sec.

On obtient 1,09 g de produit attendu.

| Spectre IR dans CHCl₃ | |
|---|---|
| NH₂ | 3440-3344 cm⁻¹ |
| >=O | 1634 cm⁻¹ |
| Système conj. | 1542 - 1520 cm⁻¹ |
| + Aromatique | |
| + NH₂ | |

### Stade C : 1-[(2'- (aminosulfonyl) (1,1'-biphényl) 4-yl) méthyl] 2-butyl 4-(méthylthio) α-oxo 2-propyl 1H-imidazole 5-éthane thioate de S-méthyle

On dissout 6,8 g du produit obtenu au stade B ci-dessus, dans 60 cm³ d'un mélange 25 cm³ TFA-75 cm³ CH₂Cl₂ et porte à reflux de chlorure de méthylène, pendant environ 5 heures. On traite la réaction en neutralisant par une solution de NaHCO₃ saturée jusqu'à pH 5-6, extrait par 2 x 200 cm³ d'acétate d'éthyle, lave par 1 x 100 cm³ de NaCl saturée, sèche, filtre et concentre à sec. On purifie sur silice, avec pour éluant ACOEt-cyclohexane (5-5). On récupère ainsi 2,7 g de produit attendu.

| Spectre IR dans CHCl₃ | |
|---|---|
| NH₂ | 3445-3350 cm⁻¹ |
| >=O | 1670-1614 cm⁻¹ |
| Aromatique | 1542 cm⁻¹ - 1518 cm⁻¹ |
| + hétéroaromatique | |
| + NH₂ dif. | |

### PREPARATION 5 : 1-[(2'-(aminosulfonyl) (1,1'-biphényl) 4-yl) méthyl] 4-(méthylthio) 2-propyl 1H-imidazole 5-carboxylate d'éthyle

### Stade A : cyano-[(1-oxobutyl) amino] acétate d'éthyle

On mélange 5 g d'éthyl (hydroxyimino) cyanoacétate, 40 cm³ de tétrahydrofuranne, 11,5 cm³ d'anhydride butyrique et 2,5 g de platine et agite en atmosphère d'hydrogène jusqu'à saturation. On filtre, rince par 5 x 15 cm³ d'éther éthylique, évapore l'éther, ajoute peu à peu 200 cm³ d'essence G, essore, lave avec 3 x 10 cm³ d'essence G et sèche à environ 75°C. On concentre à ∼ 10 cm³, ajoute 50 cm³ d'essence G, laisse cristalliser 30 mn à température ambiante, essore, lave avec 3 x 3 cm³ d'essence G et sèche à environ 75°C. On obtient 5,73 g de produit. F = 110°C.
Recristallisation pour analyses :

On dissout 540 mg du produit obtenu dans 50 cm³ d'éther isopropylique à reflux, filtre, concentre, laisse environ 1 h au repos à température ambiante, essore, lave à l'éther isopropylique et sèche. On obtient 440 mg de produit attendu.
F = 110°C.

| Microanalyse pour C₉H₁₄N₂O₃ = 198,22 | | | | |
|---|---|---|---|---|
| | C | H | N | O |
| % calculé | 54,53 | 7,12 | 14,13 | 24,22 |
| % trouvé | 54,5 | 7,2 | 14,0 | |

| Spectre IR CHCl₃ | |
|---|---|
| =C-NH | ~ 3430 cm⁻¹ |
| -C≡N | ~ 2245 cm⁻¹ |
| C=O | 1758 cm⁻¹ ester |
| | 1692 cm⁻¹ amide |
| Amide II | 1492 cm⁻¹ |

### Stade B : 3-amino 2-[(1-oxobutyl) amino] 3-(méthylthio) 2-propenoate d'éthyle

A une solution de 20 g de nitrile obtenu au stade A ci-dessus, dans 400 ml d'éthanol on ajoute 1,4 ml de triéthylamine, on refroidit à environ -10°C et introduit environ 22 g de méthylmercaptan par barbotage. On agite environ 72 heures à 0°C. On élimine l'excès de méthanethiol chasse l'éthanol, empâte à l'essence G, filtre et sèche. On obtient 24,3 g de produit attendu (cristaux incolores).
F_{K115} = 120-124°C

| Microanalyse pour C₁₀H₁₈N₂O₃S = 246,33 | | | | | |
|---|---|---|---|---|---|
| | C | H | N | S | O |
| % calculé | 48,76 | 7,37 | 11,37 | 13,02 | 19,49 |
| % trouvé | 48,6 | 7,5 | 11,4 | 12,6 | - |

| Spectre IR CHCl₃ | |
|---|---|
| =C-NH₂ | 3500, 3412 cm⁻¹ |
| =C-NH | 3365, 3275 cm⁻¹ |
| C=O complexe | 1665 cm⁻¹ |
| C=C et NH₂ déf. | 1592 cm⁻¹ |
| Amide II | 1488 cm⁻¹ |

| Spectre UV dans EtOH | |
|---|---|
| Max. 220 nm | ∈ = 5500 |
| Max. 291-292 | ∈ = 19400 |

### Stade C : 4-(méthylthio) 2-propyl 1H-imidazole 5-carboxylate d'éthyle

A 20,1 g de pentachlorure de phosphore dans 300 cm³ de chlorure de méthylène, refroidis à environ -70°C on ajoute une solution de 12,9 g de 4-diméthylaminopyridine dans 90 cm³ de chlorure de méthylène.

On maintient encore environ 15 mn à environ -70°C puis on introduit une solution de 12 g du produit obtenu au stade B ci-dessus dans 120 cm³ de chlorure de méthylène. On laisse revenir à température ambiante et maintient sous agitation pendant environ 22 heures.

On verse le mélange réactionnel dans 2,5 1 d'eau + glace et neutralise par addition d'environ 60 g de bicarbonate de sodium. On agite encore environ 30 mn, on décante et on extrait avec 500 cm³ de CH₂Cl₂. On lave à l'eau salée, sèche et chasse le solvant à environ 50°C. On purifie par chromatographie sur silice avec pour éluants CH₂Cl₂-ACOEt (90-10) puis CH₂Cl₂-ACOEt (80-20). On chasse les solvants à environ 50°C, empâte à l'essence G, filtre et sèche. On obtient 7,4 g de produit attendu (cristaux incolores). F_{K95} = 85°C.

| Microanalyse | | | | | |
|---|---|---|---|---|---|
| Concordance pour C₁₀H₁₆N₂O₂S = 228,32 | | | | | |
| | C | H | N | S | O |
| % calculé | 52,61 | 7,06 | 12,27 | 14,04 | 14,02 |
| % trouvé | 52,7 | 7,3 | 12,2 | 14,0 | |

| Spectre IR CHCl₃ | |
|---|---|
| =C-NH | 3440 - 3260 cm⁻¹ |
| C=O Complexe max. | ∼ 1672 cm⁻¹ |
| Hétérocycle | 1542 - 1498 cm⁻¹ |

| Spectre UV dans EtOH | |
|---|---|
| Max. 213-214 nm | ∈ = 14500 |
| Infl. 229 nm | ∈ = 7200 |
| Max. 286 nm | ∈ = 12200 |

| Spectre UV dans EtOH/HCl N/10 | |
|---|---|
| Max. 238 nm | ∈ = 6800 |
| Max. 277 nm | ∈ = 9600 |
| par retour basique --> max. 296 nm. | |

### Stade D : 1-[(2'-((((diméthylamino) méthylène) amino) sulfonyl) (1,1'-biphényl) 4-yl) méthyl] 4-(méthylthio) 2-propyl 1H-imidazole 5-carboxylate d'éthyle

On dissout 8,1 g du produit obtenu au stade C ci-dessus, dans 80 cm³ de diméthylformamide et ajoute 16,1 g de 4'-(bromométhyl) N-[(diméthylamino) méthylène] (1,1'-biphényl) 2-sulfonamide et 4,9 g de carbonate de potassium. On agite à température ambiante pendant environ 24 heures. On chasse le DMF à 50°C, empâte à l'eau, filtre, lave à l'eau et sèche à environ 60°C.

On empâte dans 240 cm³ d'ACOEt, sous agitation pendant environ 30 mn à environ 50°C, ajoute 160 cm³ d'hexane, filtre et sèche. On obtient 14 g de produit attendu (cristaux incolores). F_{K163} : 182°C.

| Spectre IR CHCl₃ | |
|---|---|
| Absence de =C-NH | |
| C=O | 1690 cm⁻¹ |
| C=N | 1628 cm⁻¹ |
| Hétérocycle | 1504 - 1565 cm⁻¹ |

| Spectre UV dans EtOH | |
|---|---|
| Infl. 230 nm | ∈ = 32000 |
| Max. 287 nm | ∈ = 15500 |

### Stade E : 1-[(2'-(aminosulfonyl) (1,1'-biphényl) 4-yl) méthyl] 4-(méthylthio) 2-propyl 1H-imidazole 5-carboxylate d'éthyle

On mélange 11,2 g du produit obtenu au stade D, ci-dessus avec 200 cm³ d'éthanol et 100 cm³ d'acide chlorhydrique concentré. On chauffe au reflux pendant environ 2 heures. On évapore l'éthanol, dilue par addition de 400 cm³ d'eau, alcalinise sous agitation par addition de lessive de soude et extrait à l'acétate d'éthyle. On lave à l'eau et à l'eau salée, sèche, filtre et chasse le solvant à environ 50°C.

On purifie par chromatographie sur silice avec pour éluant ACOEt 60 Hexane 40. On obtient 9,3 g de produit attendu (cristaux incolores) . F_{K135} = 130-132°C.

| Spectre IR CHCl₃ | |
|---|---|
| Absence de produit de départ | |
| C=O | 1689 cm⁻¹ |
| NH₂ | 3444 - 3340 cm⁻¹ |
| Système conjugué | |
| + aromatique | 1618 - 1590 - 1560 - 1540 - 1508 cm⁻¹ |
| NH₂ déf. | |

| Spectre UV dans EtOH | |
|---|---|
| Infl. 210 nm | ∈ = 45000 |
| Infl. 234 nm | ∈ = 17000 |
| Max. 286 nm | ∈= 15000 |

### EXEMPLE 1 : 1-[(2'-(aminosulfonyl) (1,1'-biphényl) 4-yl) méthyl] 2-butyl α-hydroxy α-méthyl 4-(méthylthio) 1H-imidazole 5-acétate d'éthyle

On introduit 1,24 g du produit obtenu à la préparation 1, 30 cm³ d'éthanol à 95 et 10 cm³ d'HCl concentré.

On chauffe au reflux jusqu'à transformation totale, la solution obtenue soit pendant environ 2 heures.

On refroidit, dilue à l'eau, alcalinise par addition de NaOHc, sature la phase aqueuse par K₂CO₃, extrait à l'AcOEt, puis purifie par chromatographie sur silice avec comme solvant AcOEt. On obtient 930 mg de produit attendu (résine jaune paille).

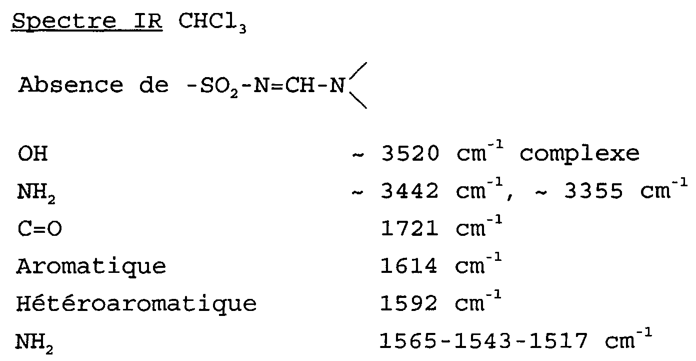

### EXEMPLE 2 : 2-butyl 1-[(2'-(((éthoxycarbonyl) amino) sulfonyl) (1,1'-biphényl) 4-yl) méthyl] α-méthylène 4-(méthylthio) 1H-imidazole 5-acétate d'éthyle

### Stade A : 1-[(2'-(aminosulfonyl) (1,1'-biphényl) 4-yl) méthyl] 2-butyl α-méthylène 4-(méthylthio) 1H-imidazole 5-acétate d'éthyle

On introduit 5,7 g du produit obtenu à la préparation I, dans 300 cm³ de dioxane et ajoute 30 cm³ d'acide sulfurique concentré. On chauffe au reflux pendant environ 5 heures. On reprend à l'eau et à l'AcOEt, alcalinise à la soude concentrée, sature par addition de K₂CO₃ et poursuit l'extraction à l'AcOEt. On purifie sur silice avec pour éluant AcOEt-CH₂Cl₂ (60-40) et obtient 1,33 g de produit attendu.

### Stade B : 2-butyl 1-[(2'-(((éthoxycarbonyl) amino) sulfonyl) (1,1'-biphényl) 4-yl) méthyl] α-méthylène 4-(méthylthio) 1H-imidazole 5-acétate d'éthyle

On mélange 1 g du produit obtenu au stade A ci-dessus, dans 15 cm³ de diméthoxyéthane, 550 mg de K₂CO₃ et 0,4 cm³ de chloroformiate d'éthyle. On chauffe au reflux pendant environ 1 heure, dilue à l'eau et extrait à l'AcOEt. On purifie sur silice avec pour éluant AcOEt-hexane (60-40), puis élue par CHCl₃ à 2 % de MeOH et récupère 720 mg de produit attendu.

### EXEMPLE 3 : acide 2-butyl α-méthylène 4-(méthylthio) 1-((2'(((éthoxycarbonyl) amino) sulfonyl) (1,1'-biphényl) 4-yl) méthyl) 1H-imidazole 5-acétique

On agite à température ambiante pendant 6 heures 650 mg du produit de l'exemple 2, dans 10 cm³ de dioxanne avec 5 cm³ de NaOH 2N. On dilue à l'eau, extrait une fraction neutre à l'AcOEt, acidifie par addition d'HClc et extrait à l'AcOEt. On purifie d'abord sur silice avec pour éluant CHCl₃-MeOH (80-20), chasse les solvants, empâte à l'éther iso, filtre et sèche à température ambiante. On dissout 200 mg de l'acide obtenu dans 10 cm³ de NaOH 2N et dilue par 10 cm³ d'eau.

On filtre, lave la phase aqueuse à l'éther, puis acidifie par addition d'HClc. On extrait à l'AcOEt, lave à l'eau salée, sèche, filtre et chasse le solvant. On empâte à l'essence G, filtre et sèche à température ambiante. On obtient 118 mg de produit attendu.

| Microanalyse concordance pour C₂₇H₃₁N₃O₆S₂ = 557,69 | | | | |
|---|---|---|---|---|
| | C | H | N | S |
| % calculé | 58,15 | 5,60 | 7,53 | 11,50 |
| % trouvé | 57,8 | 5,7 | 7,7 | 11,9 |

### EXEMPLE 4 : Acide 1-[(2'-(aminosulfonyl) (1,1'-biphényl) 4-yl) méthyl] 2-butyl 4-(méthylthio) α-méthylène 1H-imidazole 5-acétique

### Stade A : Acide 1-[(2'- (aminosulfonyl) (1,1'-biphényl) 4-yl) méthyl] 2-butyl α-hydroxy α-méthyl 4-(méthylthio) 1H-imidazole 5-acétique

On introduit 5,9 g du produit obtenu à la préparation I dans 300 cm³ de dioxanne et ajoute 30 cm³ d'acide sulfurique dilué au demi. On chauffe à environ 100°C et agite pendant environ 3 heures. On évapore le dioxanne, dilue à l'eau et extrait par CHCl₃ à 20 % de MeOH. On purifie sur silice avec pour éluant CHCl₃-MeOH (80-20) et récupère 4,5 g de produit attendu.

### Stade B : Acide 1-[(2'-(aminosulfonyl) (1,1'-biphényl) 4-yl) méthyl] 2-butyl 4-(méthylthio) α-méthylène 1H-imidazole 5-acétique

On introduit 1 g du produit obtenu au stade A ci-dessus dans 50 cm³ de dioxanne et ajoute 5 cm³ d'acide sulfurique concentré. On chauffe pendant environ 5 heures, dilue à l'eau, sature par NaCl et extrait par CHCl₃ à 20 % de MeOH.

On purifie sur silice avec pour éluant CHCl₃-MeOH (80-20) et obtient 595 mg de produit attendu.

### EXEMPLE 5 : Acide 2-butyl α-méthylène 4-(méthylthio) 1-((2'((((propylamino) carbonyl) amino) sulfonyl) (1,1'-biphényl) 4-yl) méthyl) 1H-imidazole 5-acétique (sel de sodium)

A un mélange de 545 mg du produit de l'exemple 4, 310 mg de K₂CO₃ dans 20 cm³ d'acétone, chauffé au reflux on ajoute 0,12 cm³ d'isocyanate de propyle. Puis poursuit le reflux pendant environ 3 heures.

On chasse une partie de l'acétone, dilue à l'eau, sature par addition de NaCl après acidification par HClc et extrait par CHCl₃ à 20 % de MeOH. On purifie sur silice avec pour éluant CHCl₃-MeOH (80-20) puis dissout dans 20 cm³ de NaOH 2N et acidifie sous agitation par addition d'HClc jusqu'à pH 1. On agite à température ambiante pendant environ 3 heures, filtre, lave à l'eau jusqu'à neutralité et sèche. On obtient 190 mg de produit attendu.

| Microanalyse concordance pour C₂₃H₃₃N₄NaO₅S₂ = 592,72 | | | | |
|---|---|---|---|---|
| | C | H | N | S |
| % calculé | 56,74 | 5,61 | 9,45 | 10,82 |
| % trouvé | 56,6 | 5,7 | 9,7 | 11,1 |

### EXEMPLE 6 : 2-butyl α-hydroxy α-méthyl 4-(méthylthio) 1-[(2'(((phénylméthoxy) carbonyl) amino) sulfonyl) (1,1'-biphényl) 4-yl) méthyl] 1H-imidazole 5-acétate d'éthyle

A une solution de 265 mg du produit de l'exemple 1, dans 4 cm³ de diméthoxy éthane on ajoute 138 mg de K₂CO₃ et 0,08 cm³ de chloroformiate de benzyle. On chauffe 30 mn au reflux, dilue à l'eau, extait à l'AcOEt et purifie sur silice avec pour éluant CHCl₃-MeOH (95-5). On récupère 180 mg de produit attendu.

### EXEMPLE 7 : Acide 2-butyl α-hydroxy α-méthyl 4-(méthylthio) 1-[(2'-((((phénylméthoxy) carbonyl) amino) sulfonyl) (1,1'biphényl) 4-yl) méthyl] 1H-imidazole 5-acétique

On introduit 311 mg du produit de l'exemple 6, dans 10 cm³ de MeOH, ajoute 10 cm³ de NaOH 2N et agite environ 2 heures à température ambiante. On extrait à l'éther, la phase aqueuse puis l'acidifie à pH 1 par addition d'HClc et extrait par CHCl₃ à 20 % de MeOH, puis évapore. On redissout dans 10 cm³ de soude 2N et 15 cm³ d'eau, filtre, acidifie par addition d'HClc, filtre, lave à l'eau et à l'essence G et sèche. On obtient 216 mg de produit attendu. F = 140-150°C.

| Spectre IR : Nujol | |
|---|---|
| Absorption complexe région OH/NH | |
| C=O | 1744 cm⁻¹ complexe |
| Aromatique | 1620 cm⁻¹ |
| Hétéroaromatique | 1591 cm⁻¹ |
| Amide | 1500 cm⁻¹ |

| Microanalyse concordance pour C₃₂H₃₅N₃O₇S₂ = 637,78 | | | | | |
|---|---|---|---|---|---|
| | C | H | N | S | O |
| % calculé | 60,26 | 5,53 | 6,59 | 10,05 | 17,56 |
| % trouvé | 59,95 | 5,3 | 6,6 | 10,4 | |

### EXEMPLE 8 : 2-butyl α-hydroxy α-méthyl 4-(méthylthio) 1-[(2'((((propylamino) carbonyl) amino) sulfonyl) (1,1'-biphényl) 4-yl) méthyl] 1H-imidazole 5-acétate d'éthyle

On introduit 1,3 g du produit de l'exemple 1 dans 40 cm³ d'acétone et ajoute 680 mg de carbonate de potassium. On chauffe au reflux, ajoute 0,25 cm³ d'isocyanate de propyle. Et on poursuit le reflux pendant environ 2 heures, refroidit, filtre, lave à l'acétone et chasse le solvant. On purifie sur silice avec pour éluant CHCl₃-MeOH (98-2). On empâte à l'éther iso, filtre et sèche à température ambiante. On obtient 170 mg de produit attendu.

| Spectre IR : CHCl₃ | |
|---|---|
| OH | 3510 cm⁻¹ complexe |
| =C-NH | 3403 cm⁻¹ + associé |
| >=O | 1707 cm⁻¹ |
| Amide II | 1615 cm⁻¹ |
| Aromatique | 1603 cm⁻¹ |
| Hétéroaromatique | 1539 cm⁻¹ (F). |

### EXEMPLE 9 : Acide 2-butyl α-hydroxy α-méthyl 4-(méthylthio) 1-[(2'-((((propylamino) carbonyl) amino) sulfonyl) (1,1'biphényl) 4-yl) méthyl] 1H-imidazole 5-acétique

A une solution de 582 mg du produit de l'exemple 8 dans 10 cm³ de MeOH on ajoute 5 cm³ de NaOH 2N. On agite à température ambiante pendant environ 5 heures. On chasse le MeOH sous vide, dilue à l'eau et extrait une fraction neutre à l'AcOEt. On filtre la phase aqueuse, acidifie par addition de HClc et extrait par CHCl₃ à 20 % de MeOH puis évapore. On empâte à l'éther iso sous agitation, filtre et sèche à température ambiante. Pour purifier, on dissout 420 mg de produit obtenu dans 50 cm³ de soude N sous agitation. On extrait une fraction neutre à l'éther, filtre la phase aqueuse et acidifie par addition lente d'HClc sous agitation. On agite encore environ 30 mn, filtre, lave à l'eau et à l'essence G et sèche. On obtient 170 mg de produit attendu.

| Spectre IR : | |
|---|---|
| Région OH très complexe | |
| =C-NH | 3400 cm⁻¹ |
| C=O | 1753 - 1705 - 1632 cm⁻¹ |
| Aromatique | 1592 cm⁻¹ |
| Hétéroaromatique | 1551 cm⁻¹ (F). |
| Amide II | 1504 cm⁻¹ |

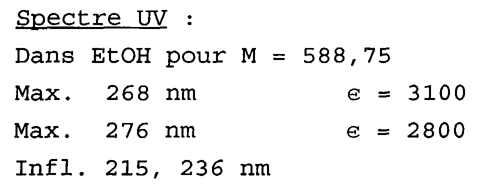

### EXEMPLE 10 : 2-butyl α-hydroxy α-méthyl 4-(méthylthio) 1-[(2'-((((phénylméthyl) amino) carbonyl) amino) sulfonyl) (1,1'-biphényl) 4-yl) méthyl] 1H-imidazole 5-acétate d'éthyle

On introduit 500 mg du produit de l'exemple 1 dans 15 cm³ d'acétone et ajoute 260 mg de carbonate de potassium. On chauffe au reflux et introduit 0,13 cm³ d'isocyanate de benzyle. Puis on poursuit le reflux pendant encore 1 heure. On filtre, chasse le solvant et purifie sur silice avec pour éluant CHCl₃-MeOH (98-2). On récupère 590 mg de produit attendu.

### EXEMPLE 11 : Acide 2-butyl α-hydroxy α-méthyl 4-(méthylthio) 1-[(2'-((((phénylméthyl) amino) carbonyl) amino) sulfonyl) (1,1'-biphényl) 4-yl) méthyl] 1H-imidazole 5-acétique

A une solution de 590 mg du produit de l'exemple 10 dans 10 cm³ de méthanol on ajoute 5 cm³ de soude 2N et agite à température ambiante pendant environ 1 heure. On glace, dilue à l'eau, acidifie par HClc et extrait par CHCl₃ à 20 % de MeOH. Pour purifier, on dissout le produit obtenu dans 20 cm³ de NaOH 2N et 30 cm³ d'eau, extrait à l'éther, filtre la solution aqueuse et acidifie sous agitation par addition d'HCl concentré. On filtre et lave à l'eau, puis sèche. On obtient 444 mg de produit attendu. Fk = 158-160°C.

| Microanalyse concordance pour C₃₂H₃₆N₄O₆S₂ = 636,79 | | | | |
|---|---|---|---|---|
| | C | H | N | S |
| % calculé | 60,36 | 5,70 | 8,80 | 10,07 |
| % trouvé | 60,2 | 5,5 | 8,6 | 10,2 |

### EXEMPLE 12 : 2-butyl 1-[(2'-(((((cyclohexylméthyl) amino) carbonyl) amino) sulfonyl) (1,1'-biphényl) 4-yl) méthyl] α-hydroxy α-méthyl 4-(méthylthio) 1H-imidazole 5-acétate d'éthyle

On introduit 178 mg du produit de l'exemple 1 dans 10 cm³ d'acétone et ajoute 46 mg de carbonate de potassium. On chauffe au reflux puis on introduit 47 mg de cyclohexyméthylisocyanate. On chauffe environ 1 heure au reflux, filtre l'insoluble et chasse le solvant. On purifie sur silice avec pour éluant AcOEt-hexane (60-40), puis CHCl₃-MeOH (95-5).

On récupère 200 mg de produit attendu.

### EXEMPLE 13 : Acide 2-butyl 1-[(2'-(((((cyclohexylméthyl) amino) carbonyl) amino) sulfonyl) (1,1'-biphényl) 4-yl) méthyl] α-hydroxy α-méthyl 4-(méthylthio) 1H-imidazole 5-acétique

On introduit 200 mg du produit de l'exemple 12 dans 2 cm³ d'éthanol ajoute 0,2 cm³ de KOH 6N et agite à température ambiante pendant environ 48 heures. On dilue à l'eau et acidifie par addition d'HCl concentré. On filtre, lave à l'eau et sèche. Pour purifier, on dissout dans 60 cm³ de NaOH 2N et 9 cm³ d'eau. On extrait à l'éther, filtre la phase aqueuse, acidifie par HCl concentré, filtre, lave à l'eau et sèche à température ambiante. On obtient 129 mg de produit attendu.

| Microanalyse pour C₃₂H₄₂N₄O₆S₂ = 642,84 | | | | |
|---|---|---|---|---|
| | C | H | N | S |
| % calculé | 59,79 | 6,59 | 8,72 | 9,98 |
| % trouvé | 59,3 | 6,5 | 8,7 | 9,9 |

### EXEMPLE 14 : 1-[(2'-(aminosulfonyl) (1,1'-biphényl) 4-yl) méthyl] α-hydroxy α-2-diméthyl 4-(méthylthio) 1H-imidazole 5-acétate d'éthyle

### Stade A : α-hydroxy α-2-diméthyl 4-(méthylthio) 1-[(2'((((diméthylamino) méthylène) amino) sulfonyl) (1,1'-biphényl) 4-yl) méthyl] 1H-imidazole 5-acétate d'éthyle

On introduit 1,1 g du produit obtenu à la préparation 2, dans 20 cm³ de THF, à laquelle on ajoute lentement 2,4 cm³ de chlorure de méthyl magnésien, l'agitation est maintenue environ 1 heure à température ambiante. On ajoute lentement au milieu une solution saturée de NH₄Cl, après extraction par de l'acétate d'éthyle, lavage par de l'eau puis séchage, on purifie sur silice avec pour éluant acétate d'éthyle + 5 % d'éthanol et obtient 770 mg de produit attendu.

| Spectre IR : CHCl₃ | |
|---|---|
| OH | 3520 cm⁻¹ |
| C=O | 1725 cm⁻¹ |
| C=N | 1624 cm⁻¹ |
| Aromatique | 1592 - 1564 cm⁻¹ |
| Hétéroaromatique | 1531 - 1517 cm⁻¹ |

### Stade B : 1-[(2'-(aminosulfonyl) (1,1'-biphényl) 4-yl) méthyl] α-hydroxy α-2-diméthyl 4-(méthylthio) 1H-imidazole 5-acétate d'éthyle

On introduit 750 mg du produit obtenu au stade A ci-dessus, dans le mélange éthanol-HCl (20 cm³-5 cm³) et porte au reflux pendant environ 5 heures. Puis on concentre, reprend par de l'eau glacée, et alcalinise par addition de NH₄OH, essore, lave à l'eau puis sèche à température ambiante. On extrait la phase aqueuse par de l'acétate d'éthyle et lave à l'eau, sèche. On récupère au total 540 mg de produit attendu.

| Spectre IR : CHCl₃ | |
|---|---|
| Absence de -N=CH- | |
| NH₂ | 3445-3440 cm⁻¹ |
| OH | 3515 cm⁻¹ |
| C=O | 1722 cm⁻¹ |
| Aromatique | 1591 - 1563 cm⁻¹ |
| Hétéroaromatique | 1530 - 1517 cm⁻¹ |
| NH₂- déf. | |

### EXEMPLE 15 : N-(phénylméthyl) 1-[(2'-(((((phénylméthyl) amino) carbonyl) amino) sulfonyl) (1,1'-biphenyl) 4-yl) méthyl] α-2-diméthyl α-hydroxy 4-(méthylthio) 1H-imidazole 5-acétamide

On introduit 500 mg du produit de l'exemple 14 dans 20 cm³ d'acétone à laquelle on ajoute 282 mg de carbonate de potassium et porte au reflux. On ajoute 0,15 cm³ d'isocyanate de benzyle. Le milieu réactionnel est maintenu sous agitation au reflux pendant environ 3 heures. On essore, lave par de l'acétate d'éthyle puis la solution organique est amenée à sec. On purifie d'abord sur silice avec pour éluant CH₂Cl₂méthanol (95-5) puis empâtage dans 20 cm³ d'éthanol, essore, lave par 2 x 5 cm³ d'éthanol puis sèche. On dissout dans un mélange éthanol 10 cm³, NaOH 2N 10 cm³, puis pendant une nuit à température ambiante. On acidifie le milieu réactionnel par addition d'hydrogéno phosphate de sodium, extrait par de l'acétate d'éthyle, lave à l'eau puis sèche. On purifie d'abord sur silice avec pour éluant CH₂Cl₂-méthanol (90-10), puis par empâtage dans 5 cm³ d'éthanol à chaud, essore et lave par 5 cm³ d'éthanol. On récupère 144 mg de produit attendu (solide blanc). F = 220°C.

| Spectre IR : Nujol | |
|---|---|
| Absorptions complexes région OH/NH | |
| C=O | 1711 - 1645 cm⁻¹ |
| Aromatique | 1592 - 1547 cm⁻¹ |
| Hétéroaromatique | 1518 - 1493 cm⁻¹ |
| Amide II | |

### EXEMPLE 16 : α-hydroxy α-2-diméthyl 4-(méthylthio) 1-[(2"(((((phénylméthyl) amino) carbonyl) amino) sulfonyl) (1,1'biphényl) 4-yl) méthyl] 1H-imidazole 5-acétate d'éthyle

### Stade A : 1-[(2'-(aminosulfonyl) (1,1'-biphényl) 4-yl) méthyl] 2-méthyl 4-(méthylthio) α-oxo 1H-imidazole 5-acétate d'éthyle

On introduit 4,8 g du produit obtenu à la préparation 2, dans le mélange de 50 cm³ d'éthanol et 30 cm³ de HCl concentré. Le milieu réactionnel est porté au reflux pendant environ 5 h. La solution est alors concentrée sous vide puis reprise par de l'eau glacée. On alcalinise par addition de NH₄OHa jusqu'à pH ∼ 8 puis extrait par de l'acétate d'éthyle, lave à l'eau, sèche puis purifie par chromatographie sur silice avec pour éluant l'acétate d'éthyle.

On obtient ainsi 2,4 g de produit attendu.

| Spectre IR CHCl₃ | |
|---|---|
| -NH₂ | 3443-3343 cm⁻¹ |
| C=O | 1734-1627 cm⁻¹ |
| Aromatique | 1593 cm⁻¹ |
| Hétéroatome | 1565 cm⁻¹ |
| NH₂ def. | 1542 cm⁻¹ |

### Stade B : 2-méthyl 4-(méthylthio) 1-[(2'-(((((phénylméthyl) amino) carbonyl) amino) sulfonyl) (1,1'-biphényl) 4-yl) méthyl] α-oxo 1H-imidazole 5-acétate d'éthyle

On introduit 0,8 g du produit obtenu au stade A ci-dessus dans 25 cm³ d'acétone et ajoute 465 mg de carbonate de potassium. Le milieu est alors porté au reflux, et on ajoute goutte à goutte 0,2 cm³ d'isocyanate de benzyle, l'agitation est maintenue dans ces conditions pendant environ 1 h. On évapore à sec, reprend le résidu par de l'eau puis on acidifie par addition d'hydrogénophosphate de sodium, le précipité est essoré, lavé abondamment à l'eau puis séché. On purifie par empâtage dans un mélange isopropanol 10 cm³, éther isopropylique 20 cm³, essore, lave par 2 x 25 cm³ d'éther isopropylique et obtient 480 mg de produit attendu (solide jaune). F = 130°C.

| Spectre IR : CHCl₃ | |
|---|---|
| Absence de SO₂NH₂ | |
| -NH-C= complexe | 3395-3375 cm⁻¹ |
| C=O | 1732-1714-1624 cm⁻¹ épai |
| Aromatique | 1539 cm⁻¹ |
| Hétéroatome | 1497 cm⁻¹ |
| Amide II | |

### Stade C : α-hydroxy α-2-diméthyl 4-(méthylthio) 1-[(2'(((((phénylméthyl) amino) carbonyl) amino) sulfonyl) (1,l'biphényl) 4-yl) méthyl] 1H-imidazole 5-acétate d'éthyle

On introduit 455 mg du produit obtenu au stade B ci-dessus, dans 10 cm³ de THF, à laquelle on ajoute lentement 1,25 cm³ de chlorure de méthyle magnésien, l'agitation est maintenue environ 30 mn à la température ambiante, la solution ainsi obtenue est versée sur de l'eau, on extrait par de l'acétate d'éthyle, la phase organique est lavée à l'eau et séchée. On purifie sur silice avec pour éluant CH₂Cl₂-méthanol (95-5) et obtient 270 mg de produit attendu.
F = 110°C.

| Spectre IR **:** CHCl₃ | |
|---|---|
| -OH | 3515 cm⁻¹ + associé |
| =C-NH | 3395 cm⁻¹ |
| C=O | 1713 cm⁻¹ complexe |
| Aromatique | 1605 - 1592 cm⁻¹ |
| Hétéroatome | 1562 - 1535 cm⁻¹ |
| Amide II | 1498 cm⁻¹ |

### EXEMPLE 17 : Acide α-2-diméthyl α-hydroxy 4-(méthylthio) 1-[(2'-(((((phénylméthyl) amino) carbonyl) amino) sulfonyl) (1,1'-biphényl) 4-yl) méthyl] 1H-imidazole 5-acétique

On introduit 250 mg du produit de l'exemple 16 dans le mélange NaOH-éthanol (5 cm3-5 cm³) et l'agitation est maintenue pendant environ 3 h à température ambiante. Le milieu réactionnel est versé sur de l'eau, puis on acidifie par addition d'HCl 2N et ajoute 20 cm³ d'acétate d'éthyle. Après environ 30 mn d'agitation à environ 0°C, on lave abondamment à l'eau, on empâte dans 2 x 10 cm³ d'éthanol bouillant, puis par 10 cm³ d'éther isopropylique et sèche. On obtient 165 mg de produit attendu (solide blanc). F = 245°C.

| Spectre IR : CHCl₃ | |
|---|---|
| OH/NH | 3350 cm⁻¹ + absorption générale |
| C=O | 1666 cm⁻¹ |
| Région COO⁻ | 1625 cm⁻¹ |
| Aromatique | 1607 - 1580 cm⁻¹ |
| Hétéroatome | 1560 - 1535 cm⁻¹ |
| Amide II | 1497 cm⁻¹ |

### EXEMPLE 18 : α-hydroxy α-2-diméthyl 4-(méthylthio) 1-[(2'((((propylamino) carbonyl) amino) sulfonyl) (1,1'-biphényl) 4-yl) méthyl] 1H-imidazole 5-acétate d'éthyle

### Stade A : 2-méthyl 4-(méthylthio) α-oxo 1-[(2'-((((propylamino) carbonyl) amino) sulfonyl) (1,1'-biphényl) 4-yl) méthyl] 1H-imidazole 5-acétate d'éthyle

On procède comme au stade B de l'exemple 16. On introduit 0,85 g du produit obtenu au stade A de l'exemple 16, dans 15 cm³ d'acétone, à laquelle on ajoute 500 mg de carbonate de potassium. On porte au reflux et ajoute 0,17 cm³ d'isocyanate de n-propyle puis maintient au reflux pendant environ 1 h. La suspension est ensuite concentrée, le résidu obtenu repris par de l'eau puis on acidifie par addition d'acide chlorhydrique 2N jusqu'à pH 2. Puis on essore, lave à l'eau et sèche à température ambiante. On purifie sur silice avec pour éluant CH₂Cl₂ + 3 % méthanol et obtient 380 mg de produit attendu.

| Spectre IR : CHCl₃ | |
|---|---|
| =C-NH | 3402-3378 cm⁻¹ |
| C=O | 1732- 1716 - 1625 cm⁻¹ |
| Région COO⁻ | 1625 cm⁻¹ |
| Aromatique | 1561 cm⁻¹ |
| Amide II | 1540 cm⁻¹ |

### Stade B : α-hydroxy α-2-diméthyl 4-(méthylthio) 1-[(2'((((propylamino) carbonyl) amino) sulfonyl) (1,1'-biphényl) 4-yl) méthyl] 1H-imidazole 5-acétate d'éthyle

On procède comme au stade C de l'exemple 16 en utilisant au départ 350 mg du produit obtenu au stade A ci-dessus, dans 10 cm³ THF et ajoute 1 cm³ de chlorure de méthyl magnésien à une température d'environ 10°C. Puis l'agitation est maintenue à température ambiante pendant environ 1 heure. On ajoute ensuite une solution saturée de NH₄Cl, extrait par de l'acé- tate d'éthyle, lave à l'eau puis sèche. On purifie sur silice avec pour éluant CH₂Cl₂-méthanol (95-5) et obtient 130 mg de produit attendu.

### EXEMPLE 19 : Acide α-2-dimethyl α-hydroxy 4-(méthylthio) 1-[(2'-(((((propylamino) carbonyl) amino) sulfonyl) (1,1'biphényl) 4-yl) méthyl] 1H-imidazole 5-acétique

On procède comme à l'exemple 17 en utilisant 130 mg du produit de l'exemple 18 dans 5 cm³ de NaOH N et l'agitation est maintenue à température ambiante pendant 16 heures.

Le milieu réactionnel est ensuite acidifié par addition d'hydrogéno phosphate de sodium, puis on essore, lave abondamment à l'eau et sèche. On purifie par empâtage dans 5 cm³ d'acétate d'éthyle et obtient 70 mg de produit attendu.
F = 205°C.

| Spectre IR : Nujol | |
|---|---|
| Absorptions complexes | région OH/HN |
| C=O | 1709- 1667 - 1629 cm⁻¹ |
| Aromatique | 1575 - 1559 cm⁻¹ |
| Amide II | 1550 - 1513 cm⁻¹ |

### EXEMPLE 20 : α-hydroxy 4-(méthylthio) 1-[(2'-(((((phénylméthyl) amino) carbonyl) amino) sulfonyl) (1,1'-biphényl) 4-yl) méthyl] 2-propyl 1H-imidazole 5-acétate d'éthyle

### Stade A: 1-[(2'-(((éthoxycarbonyl) amino) sulfonyl) (1,1'biphényl) 4-yl) méthyl] 4-(méthylthio) α-oxo 2-propyl 1H-imidazole 5-acétate d'éthyle

On introduit 3,3 g du produit obtenu à la préparation 3, dans 60 cm³ de diméthoxyéthanol, à laquelle on ajoute successivement 1,81 g de carbonate de potassium puis 1,25 cm³ de chloroformiate d'éthyle. La solution est alors portée au reflux pendant environ 5 heures puis filtre, lave par de l'acétate d'éthyle et la solution organique est amenée à sec. On reprend par du CH₂Cl₂, lave abondamment à l'eau puis sèche. On purifie par chromatographie sur silice avec pour éluant : CH₂Cl₂-méthanol 96-4 et obtient 1,5 g de produit attendu.

| Spectre IR : CHCl₃ | |
|---|---|
| =C-NH | 3384 cm⁻¹ |
| C=O | 1739 - 1626 cm⁻¹ |
| Aromatique | 1564 cm⁻¹ |
| Hétéroatome | 1516 cm⁻¹ |
| Amide II | 1480 cm⁻¹ |

### Stade B : 1-[(2'-(((éthoxycarbonyl) amino) sulfonyl) (1,l'biphényl) 4-yl) méthyl] α-hydroxy 4-(méthylthio) 2-propyl 1H-imidazole 5-acétate d'éthyle

On introduit 1,3 g du produit obtenu au stade A ci-dessus dans 50 cm³ d'éthanol, refroidit à environ -20°C/-25°C et ajoute 90 mg de borohydrure de sodium. L'agitation est maintenue à environ -20°C pendant environ 15 mn. On évapore l'éthanol puis reprend par 100 cm³ d'eau glacée et ajoute 50 cm³ d'acétate d'éthyle, puis évapore la phase organique, essore, lave abondamment puis sèche. On obtient 1,05 g de produit attendu. F = 147°C.

| Spectre IR : CHCl₃ | |
|---|---|
| Absence de cétone conjuguée | |
| OH | 3590 - 3508 cm⁻¹ |
| -NH | 3385 cm⁻¹ |
| + absorption générale | |
| C=O | 1746 cm⁻¹ |
| Aromatique | 1614 - 1590 cm⁻¹ |
| Hétérocycle | 1560 - 1540 cm⁻¹ |
| Amide | 1538 - 1500 cm⁻¹ |

### Stade C : α-hydroxy 4-(méthylthio) 1-[(2'-(((((phénylméthyl) amino) carbonyl) amino) sulfonyl) (1,1'-biphényl) 4-yl) méthyl] 2-propyl 1H-imidazole 5-acétate d'éthyle

On introduit 1 g du produit obtenu au stade B ci-dessus, dans 30 cm³ de toluène, à laquelle on ajoute 1 cm³ de benzylamine, et porte au reflux pendant environ 30 mn. On sèche puis reprend par de l'eau et acidifie par addition d'hydrogéno phosphate de sodium. On extrait par de l'acétate d'éthyle, la phase organique est lavée à l'eau puis séchée. On purifie sur silice avec pour éluant CH₂Cl₂-éthanol (96-4) et obtient 730 mg de produit attendu.

| Spectre IR : CHCl₃ | |
|---|---|
| Absorption OH | 3500 cm⁻¹ |
| NH | 3370 cm⁻¹ complexe |
| C=O | 1714 cm⁻¹ |
| Système conjugué | 1664 - 1592 cm⁻¹ |
| Aromatique | 1538 - 1502 cm⁻¹ |
| Amide II | |

### EXEMPLE 21 : Acide 1-[(2'-(((((phénylméthyl) amino) sulfonyl) (1,1'-biphényl) 4-yl) méthyl] α-hydroxy 4-(méthylthio) 2-propyl 1H-imidazole 5-acétique

On introduit 400 mg du produit de l'exemple 20 dans le mélange éthanol-NaOH (5 cm³-5 cm³) et l'agitation est maintenue environ 3 h à température ambiante. Puis on concentre, reprend par de l'eau et acidifie par addition HCl N, essore, lave abondamment à l'eau puis sèche. Pour purifier, on dissout à chaud dans un mélange acétate d'éthyle 25 cm³ et d'éthanol 25 cm³, filtre, concentre de moitié, puis laisse 1 nuit à température ambiante. Après essorage, lavage du précipité successivement par 20 cm³ d'acétate d'éthyle puis 20 cm³ d'éthanol et séchage, on récupère 136 mg de produit attendu.
F = 225°C.

| Spectre IR : CHCl₃ | |
|---|---|
| Absorption OH/NH | |
| C=O | 1672 - 1640 cm⁻¹ |

### EXEMPLE 22 : 1-[(2'-(((((cyclohexylméthyl) amino) carbonyl) amino) sulfonyl) (1,1'-biphényl) 4-yl) méthyl] 4-(méthylthio) 2-propyl 1H-imidazole 5-carboxylate d'éthyle

On introduit 400 mg du produit obtenu à la préparation 5 dans 5 ml d'acétone anhydre et 236 mg de carbonate de potassium K₂CO₃. On porte au reflux et ajoute 200 µl d'isocyate de cyclohexylméthyle. Après environ 1 h d'agitation à chaud, la solution est refroidie à température ambiante, hydrolysée par une solution NH₃Cl aqueuse saturée puis extraite au CH₂Cl₂. Après séchage et évaporation, on recristallise dans l'éther après dissolution dans le minimum de CH₂Cl₂, puis filtre, sèche et obtient 420 mg de produit attendu (solide blanc).

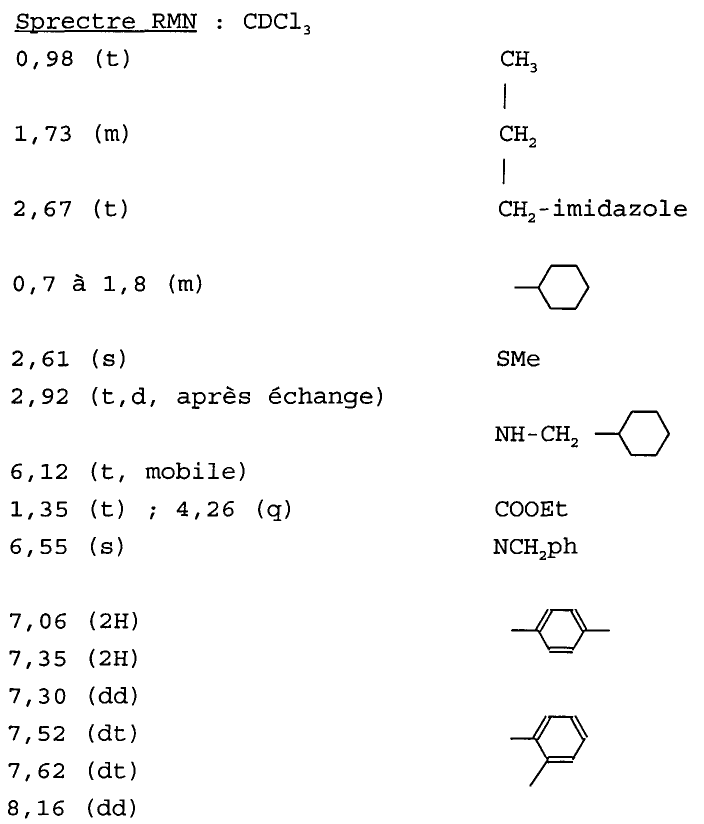

### EXEMPLE 23 : 1-[(2'-(((((cyclohexylméthyl) amino) carbonyl) amino) sulfonyl) (1,1'-biphényl) 4-yl) méthyl] 4-(méthylthio) 2-propyl 1H-imidazole 5-carboxylate de sodium

On introduit 380 mg du produit de l'exemple 22 dans 8 ml d'éthanol et 5 ml de NaOH 2N et laisse sous agitation à température ambiante pendant environ 48 heures. L'éthanol est ensuite évaporé et après ajout de 10 ml d'eau, la phase aqueuse est extraite par de l'acétate d'éthyle, puis filtrée, placée à 0°C et acidifiée lentement jusqu'à pH 2. Après environ 30 mn d'agitatin, le précipité est filtré et séché et on obtient 240 mg de produit attendu.

| Spectre IR : CHCl₃ | |
|---|---|
| Acide d'après région OH | |
| =C-NH | 3390 cm⁻¹ |
| >=O | 1705 - 1680 cm⁻¹ |
| Aromatique | 1606 cm⁻¹ |
| Hétéroatome | 1545 cm⁻¹ |
| Amide II | 1517 cm⁻¹ |

### EXEMPLE 24 : 4-(méthylthio) 1-[(2'-(((((phénylméthyl) amino) carbonyl) amino) sulfonyl) (1,1'-biphényl) 4-yl) méthyl] 2-propyl 1H-imidazole 5-carboxylate d'éthyle

On introduit 2 g du produit obtenu à la préparation 5, dissous dans 25 ml d'acétone anhydre et 1,2 g de carbonate de potassium. On porte au reflux et ajoute 740 µl de benzylisocyanate. Après environ 2 h d'agitation, on refroidit à température ambiante, hydrolyse par une solution aqueuse saturée de NH₄Cl puis extrait au chlorure de méthylène. On sèche, recristallise dans l'éther, filtre et obtient 1,9 g de produit attendu.

### EXEMPLE 25 : acide 4-(méthylthio) 1-[(2'-(((((phénylméthyl) amino) carbonyl) amino) sulfonyl) (1,1'-biphényl) 4-yl) méthyl] 2-propyl 1H-imidazole 5-carboxylique

On introduit 1,8 g du produit obtenu à l'exemple 24, dans 10 ml d'éthanol et 10 ml de soude 2N puis laisse sous agitation à température ambiante pendant environ 36 heures. L'éthanol est ensuite évaporé et, après ajoute de 25 ml d'eau, la phase aqueuse est extraite avec de l'éther, puis filtrée, placée à 0°C et acidifiée lentement jusqu'à pH 1,5 avec HCl 1N. On filtre et sèche et obtient 1,4 g de produit attendu.

| Spectre IR : CHCl₃ | |
|---|---|
| Acide d'après la région OH | |
| =C-NH | 3480 cm⁻¹ |
| >=O | 1706 - 1690 cm⁻¹ complexe |
| Aromatique | 1539 cm⁻¹ |
| Hétéroaromatique | 1521 cm⁻¹ |
| Amide II | 1500 cm⁻¹ |

### EXEMPLE 26 : 4'-((5-acétyl 2-butyl 4-(méthylthio) 1H-imidazole 1-yl) méthyl) N-((propylamino) carbonyl) (1,1'-biphényl) 2-sulfonamide

### Stade A : 4'-((2-butyl 5-((méthylsulfinyl) acétyl) 4-(méthylthio) 1H-imidazol 1-yl) méthyl) N-((propylamino) carbonyl) (1,1'-biphényl) 2-sulfonamide

On introduit 3,52 g de NaH à 50 % dans l'huile, on débarrasse le NaH de son huile par 3 lavages successifs au pentane. Puis on sèche. On ajoute 42 cm³ de DMSO anhydre, on porte le mélange à 75°C, pendant environ 1 heure, puis refroidit le mélange à 0°C et on ajoute à l'anion du DMSO formé 40 cm³ de THF anhydre et goutte à goutte 12 g de 2-butyl H-(méthylthio) 1-[[2'-((((propylamino) carbonyl) amino) sulfonyl) (1,1'-biphényl) 4-yl] méthyl] 1H-imidazole 5-carboxylate d'éthyle, dissous dans 80 cm³ de THF anhydre. On laisse remonter à la température ambiante et laisse sous agitation pendant environ 1 heure. Puis le mélange réactionnel est versé dans 400 cm³ d'eau distillée et acidifié jus- qu'au pH 2, avec de l'acide chlorhydrique 2N. On extrait par 3 x 200 cm³ de chlorure de méthylène et lave la phase organique par 1 x 200 cm³ de solution saturée de NH₄Cl et 2 x 200 cm³ d'eau distillée.

La phase organique est séchée, filtrée et concentrée à sec. On empâte avec de l'éther isopropylique puis purifie sur silice avec pour éluant CH₂Cl₂-méthanol (95-5), empâte dans l'éther iso et obtient 8,93 g de produit attendu (poudre blanche).

| Microanalyse | | | | | |
|---|---|---|---|---|---|
| | C | H | N | S | O |
| % calculé | 55,61 | 5,99 | 9,27 | 15,9 | 13,23 |
| % trouvé | 55,5 | 5,9 | 9,2 | 16,0 | - |

| Spectre IR : CHCl₃ | |
|---|---|
| NH | ~ 3370 cm⁻¹ associé |
| >=O | 1710-1635 cm⁻¹ |

### Stade B : 4'-((5-acétyl 2-butyl 4-(méthylthio) 1H-imidazole 1-yl) méthyl) N-((propylamino) carbonyl) (1,1'-biphényl) 2-sulfonamide

A une solution de 1 g du produit obtenu au stade A ci-dessus dans 35 cm³ d'éthanol, on ajoute 20 cm³ d'une solution aqueuse à 10 % de chlorure d'ammonium et 650 mg de zinc électrocyclique. On agite pendant 24 heures puis filtre, évapore, redissout dans l'acétate d'éthyle, lave à l'eau, sèche et évapore. Le résidu est purifié par passage sur silice en éluant au chlorure de méthylène à 2 % de méthanol. On obtient 711 mg de produit attendu fondant à 176-177°C.

| Microanalyse | | | | |
|---|---|---|---|---|
| | C | H | N | S |
| % calculé | 59,75 | 6,31 | 10,32 | 11,82 |
| % trouvé | 59,6 | 6,3 | 10,1 | 11,7 |

| Spectre IR : CHCl₃ | |
|---|---|
| NH | 3402 et 3368 cm⁻¹ |
| >=O | 1716 et 1632 cm⁻¹ |
| Aromatique | |
| + hétéroaromatique | 1541 - 1493 cm⁻¹ |
| + Amide II | |

### EXEMPLE 27 : 2-butyl 4-(méthylthio) 1-[(2'-(((((diphénylméthyl) amino) carbonyl) amino) sulfonyl) (1,1'-biphényl) 4-yl) méthyl] 1H-imidazole 5-carboxylate d'éthyle

On dissout dans 70 cm³ de toluène, 1,8 g de 2-butyl 1-[(2'-(((éthoxycarbonyl) amino) sulfonyl) (1,1'-biphényl) 4-yl) méthyl] 4-(méthylthio) 1H-imidazole 5-carboxylate d'éthyle préparé ainsi qu'il est indiqué dans EP 0503162 et 1,16 g de diphényl amino méthane. On chauffe environ 14 h à 85°C, refroidit, ajoute de l'AcOEt et lave la phase organique par HCl N, puis à l'eau. On sèche, évapore, cristallise dans l'éther, essore et obtient 0,8 g de produit attendu.
F = 125°C.

| Spectre IR : CHCl₃ | |
|---|---|
| =C-NH | 3366 cm⁻¹ |
| >=O | 1715-1689-1665 cm⁻¹ |

### EXEMPLE 28 : 2-butyl 1-[(2'-(((((diphénylméthyl) amino) carbonyl) amino) sulfonyl) (1,1'-biphényl) 4-yl) méthyl] 4-(méthylthio) 1H-imidazole 5-carboxylate de potassium (disel de potassium)

On introduit 7 ml de EtOH 99 dans 350 mg du produit de l'exemple 27 et ajoute goutte à goutte 0,33 ml de KOH 6N. On agite environ 48 h à température ambiante, essore, lave par 2 x 2 ml EtOH 99. On empâte par 3,5 cm³ EtOH 96, agite environ 45 mn, essore, lave par 2 x 1 cm³ EtOH 96, puis à l'éther. On recristallise par chaud et froid dans 5 cm³ d'EtOH 96 et obtient 170 mg de produit attendu. F > 260°C.

| Spectre IR : Nujol | |
|---|---|
| Absorption complexe région OH/NH | |
| >=O | 1600 cm⁻¹ |
| Aromatique | 1539 cm⁻¹ |
| Hétéroatome | 1517 cm⁻¹ |
| | 1494 cm⁻¹ |

| Microanalyse pour C₃₆H₃₄K₂N₄O₅S₂H₂ | | | | |
|---|---|---|---|---|
| | C | H | N | S |
| % calculé | 56,6 | 4,7 | 7,3 | 8,4 |
| % trouvé | 56,0 | 4,7 | 7,2 | 8,5 |

### EXEMPLE 29 : 2-butyl 4-(méthylthio) 1-[(2'-(((diphénylacétyl) amino) sulfonyl) (1,1'-biphényl) 4-yl) méthyl] 1H-imidazole 5-carboxylate d'éthyle

A une suspension dans 10 ml de toluène de 365 mg de 1-[(2'-(aminosulfonyl) (1,1'-biphényl) 4-yl) méthyl] 2-butyl 4-(méthylthio) 1H-imidazole 5-carboxylate d'éthyle, préparé ainsi qu'il est indiqué dans EP 0503162, on ajoute 225 mg de chlorure de diphényl acétyle et 119 mg de DMAP. On chauffe environ 30 mn à 70°C, refroidit, reprend à l'eau, extrait à l'acétate d'éthyle, sèche et évapore. On cristallise dans 5 cm³ d'acétonitrile puis par chaud et froid dans 7 cm³ d'acétonitrile et obtient 200 mg de produit attendu.
F > 210°C.

| Spectre IR : CHCl₃ | |
|---|---|
| Absence de NH₂ | |
| NH | 3360 cm⁻¹ + associé |
| C=O | 1725 - 1680 cm⁻¹ (ester) |
| Aromatique | 1613 - 1600 cm⁻¹ |
| Hétérocycle | 1560 - 1508 cm⁻¹ |
| Amide II | 1496 - 1483 cm⁻¹ |
| SO₂ | 1346 cm⁻¹ |

### EXEMPLE 30 : Acide 2-butyl 1-[(2'-(((diphénylacétyl) amino) sulfonyl) (1,1'-biphényl) 4-yl) méthyl] 4-(méthylthio) 1H-imidazole 5-carboxylique

On dissout dans 10 ml de MeOH, 500 mg du produit de l'exemple 29 et ajoute 5 ml de NaOH 2N. On agite environ 18 h à température ambiante, évapore le méthanol, reprend à l'eau, extrait à l'acétate d'éthyle, acidifie la phase aqueuse par HCl 2N (pH 1) et extrait par CH₂Cl₂ puis sèche et évapore. On recristallise par chaud et froid dans 50 cm³ d'éthanol et obtient 340 mg de produit attendu. F = 205°C.

| Spectre IR : Nujol | |
|---|---|
| Région OH/NH : max | 3249 cm⁻¹ + absorption générale |
| >=O | 1728 - 1645 cm⁻¹ |
| Aromatique + | 1584 - 1565 cm⁻¹ |
| Hétéroaromatique | 1545 - 1510 - 1495 cm⁻¹ |

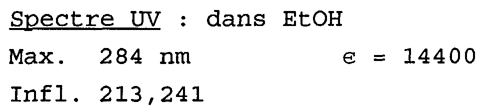

### EXEMPLE 31 : α-butyl α-hydroxy 4-(méthylthio) 1-[(2'(((((phénylméthyl) amino) carbonyl) amino) sulfonyl) (1,1'biphényl) 4-yl) méthyl] 2-propyl 1H-imidazole 5-acétate d'éthyle

### Stade A : 4-(méthylthio) alpha-oxo 1-((2'-(((((phénylméthyl) amino) carbonyl) amino) sulfonyl) (1,1'-biphényl) 4-yl) méthyl) 2-propyl 1H-imidazole 5-acétate d'éthyle

On introduit 3,6 g de produit obtenu à la préparation 3 dans 50 cm³ d'acétone puis en une fois 2 g de carbonate de potassium. Le milieu est alors porté au reflux et on y ajoute goutte à goutte 1 cm³ d'isocyanate de benzyle. Le reflux est maintenu durant 2 h. Puis on verse sur 500 cm³ d'eau glacée, acidifie le milieu par addition d'acide chlorhydrique N, essore, lave abondamment à l'eau, sèche à 50°C. On purifie par empâtage dans 50 cm³ d'éther isopropylique et obtient 4,2 g de produit dont 500 mg sont purifiés par recristallisation dans 25 cm³ d'éthanol, après essorage et séchage, on obtient 300 mg de produit attendu. F = 188°C.

| Spectre IR : CHCl₃ | |
|---|---|
| =C-NH | 3375 cm⁻¹ |
| C=O | 1714 - 1621 cm⁻¹ |
| Système conjugué + | 1537 cm⁻¹ |
| Aromatique + | 1495 cm⁻¹ |
| Amide II + | |

### Stade B : α-butyl α-hydroxy 4-(méthylthio) 1-[(2'(((((phénylméthyl) amino) carbonyl) amino) sulfonyl) (1,l'biphényl) 4-yl) méthyl] 2-propyl 1H-imidazole 5-acétate d'éthyle

On introduit 750 mg du produit obtenu au stade A ci-dessus, dans 50 cm³ de THF anhydre, refroidit à 0°C et ajoute goutte à goutte 2,8 cm³ de chlorure de n-butyl magnésium, l'agitation est maintenue à 0°C pendant 30 mn puis à température ambiante pendant 1 h. Le milieu réactionnel est acidifié par addition d'acide chlorhydique 0,1 N, puis on extrait par de l'acétate d'éthyle. La phase organique est abondamment lavée par de l'eau puis séchée. On récupère une résine jaune. On purifie par 2 chromatographies successives sur silice avec pour éluant CH₂Cl₂ + 2,5 % méthanol et obtient 300 mg de produit attendu.

| Spectre IR : CHCl₃ | |
|---|---|
| OH | 3520 cm⁻¹ |
| NH | 3408 cm⁻¹ |
| C=O | 1714 cm⁻¹ |
| Système conjugué | 1604 cm⁻¹ |
| Aromatique | 1532 cm⁻¹ (complexe, F) |
| Amide II | 1500 cm⁻¹ |

### EXEMPLE 32 : α-butyl α-hydroxy 4-(méthylthio) 1-[(2'(((((phénylméthyl) amino) carbonyl) amino) sulfonyl) (1,1'biphényl) 4-yl) méthyl] 2-propyl 1H-imidazole 5-acétate de sodium

On introduit 260 mg du produit obtenu à l'exemple 31, dans 5 cm³ de soude, 20 cm³ d'éthanol, l'agitation est maintenue pendant 4 h à température ambiante. Le milieu réactionnel est acidifié par addition d'HCl 2N, après extraction par de l'acétate d'éthyle, lavage à l'eau de la phase organique, puis séchage, on purifie par chromatographie sur silice avec pour éluant CH₂Cl₂-méthanol. On purifie à nouveau par HPLc sur silice avec pour éluant H₂O-méthanol (60-40) et obtient 90 mg de produit attendu. F = 210°C.

| Spectre IR : Nujol | |
|---|---|
| Absorption complexe région OH/NH | |
| C=O | 1610 cm⁻¹ (F) |
| Aromatique | 1520 cm⁻¹ |
| Hétéroatome | 1500 cm⁻¹ |
| Amide II | |

### EXEMPLE 33 : 4'-[(5-(1,2-dihydroxyéthyl) 4-(méthylthio) 2-propyl 1H-imidazole 1-yl) méthyl) N-(((phénylméthyl) amino) carbonyl) (1,1'-biphényl) 2-sulfonamide

On dissout 500 mg de produit obtenu au stade A de l'exemple 31 dans 10 cm³ de THF. On refroidit à 0°C et ajoute 2 ml de LibH₄ en solution 2M/THF. Après 3 heures à température ambiante, on ajoute 2 cm³ d'acide acétique, 20 cm³ H₂O et on extrait à l'ACOEt. On sèche et évapore à sec. Après chromatographie sur silice, éluant CH₂Cl₂/MeOH (94/6) et empâtage dans l'éther isopropylique, on récupère 75 mg de produit attendu. F = 140°C.

### EXEMPLE 34 : Acide α-hydroxy 4-(méthylthio) α-phényl 1-((2'(((((phénylméthyl) amino) carbonyl) amino) sulfonyl) (1,1'biphényl) 4-yl) méthyl) 2-propyl 1H-imidazole 5-acétique

On dissout 250 mg de produit obtenu au stade A de l'exemple 31 dans 5 cm³ de THF anhydre. On ajoute goutte à goutte 430 µl de PhMgBr 3M dans l'éther. Après une heure à température ambiante, on hydrolyse avec HCl 2N, extrait à l'ACOEt, sèche et évapore à sec.

Le brut obtenu est dissous dans un mélange de 5 cm³ d'éthanol et 5 cm³ NaOH 2N. Après une nuit à température ambiante, on acidifie avec HCl 1N, on filtre, lave le précipité obtenu à l'eau puis avec 5 cm³ d'ACOEt et 5 cm³ d'éther isopropylique. On obtient 67 mg d'un solide blanc. F = 170°C.

### EXEMPLE 35 : 2-butyl 4-(méthylthio) 1-((2'-((((propylamino) carbonyl) amino) sulfonyl) (1,1'-biphényl) 4-yl) méthyl) 1H imidazole 5-carboxamide

On introduit 600 mg de l'acide 2-butyl H-(méthylthio) 1-[[2'-((((propylamino) carbonyl) amino) sulfonyl) (1,1'-biphényl) 4-yl] méthyl] 1H-imidazole 5-carboxylique, dans 15 ml de toluène et on ajoute 600 µl de chlorure de tionyle SOCl₂. On agite environ 1 h à température ambiante, puis 1 nuit à environ 55°C. Le solvant est ensuite évaporé, repris dans du toluène et à nouveau évaporé. On sèche, reprend dans 15 ml de de dioxanne et ajoute 10 gouttes de NH₄OH (à 20 %). Après environ 1 heure d'agitation, on acidifie à pH 4 avec HCl 1N, puis extrait avec CH₂Cl₂ et sèche. On purifie sur silice avec pour éluant CH₂Cl₂-AcOEt (50-50), puis ACOEt-CH₂Cl₂-MeOH (50-50-5 %) et obtient après évaporation, 350 mg de produit attendu (solide blanc).

| Spectre IR : CHCl₃ | |
|---|---|
| =C-NH₂ | 3383 - 3375 cm⁻¹ |
| =C-NH | 3315 cm⁻¹ |
| >=O | 1710 - 1651 cm⁻¹ |
| Aromatique | 1616 cm⁻¹ |
| Hétéroaromatique | 1584 cm⁻¹ |
| Amide II | 1545 cm⁻¹ |
| NH₂ | 1506 cm⁻¹ |

| Microanalyse : C₂₆H₃₃N₅O₄S₂ M=543 | | | | |
|---|---|---|---|---|
| | C | H | N | S |
| % calculé | 57,4 | 6,12 | 12,87 | 11,79 |
| % trouvé | 57,6 | 6,0 | 12,4 | 11,6 |

Les produits suivants ont été préparés suivant le procédé décrit à l'exemple 23 ou 25 en utilisant au départ les composés appropriés.

### EXEMPLE 36 : Acide 4-bromo 1-((2'-(((((phénylméthyl) amino) carbonyl) amino) sulfonyl) (1,1'-biphényl) 4-yl) méthyl) 2-propyl 1H-imidazole 5-carboxylique

F = 128°C. rf = 0,34 (MeOH-CH₂Cl₂ 20-80).

### EXEMPLE 37 : Acide 4-bromo alpha hydroxy alpha méthyl 1-((2'(((((phénylméthyl) amino) carbonyl) amino) sulfonyl) (1,1'biphenyl) 4-yl) méthyl) 2-propyl 1H-imidazole 5-acétique

F = 153°C. rf = 0,46 (MeOH-CH₂Cl₂ 20-80).

### EXEMPLE 38 : Acide 4-(butylthio) alpha oxo 1-((2' (((((phénylméthyl) amino) carbonyl) amino) sulfonyl) (1,1'biphényl) 4-yl) méthyl) 2-propyl 1H-imidazole 5-acétique

F = 192°C. rf = 0,36 (MeOH-CH₂Cl₂ 20-80).

### EXEMPLE 39 : Acide 4-(butylthio) 1-((2'-(((((phénylméthyl) amino) carbonyl) amino) sulfonyl) (1,1'-biphényl) 4-yl) méthyl) 2-propyl 1H-imidazole 5-carboxylique

F = 197°C.

### EXEMPLE 40 : Acide 1-((2'-(((3-cyclopentyl 1-oxopropyl) amino) sulfonyl (1,1'-biphenyl) 4-yl) méthyl) 4-(méthylthio) 2-propyl 1H-imidazole 5-carboxylique

F ≈ 190°C. rf = 0,4 (CH₂Cl₂-MeOH 95-5).

### EXEMPLE 41 : Acide α-hydroxy α-méthyl 4-(méthylthio) 1-((2'(((((phénylméthyl) amino) carbonyl) amino) sulfonyl (1,1'biphényl) 4-yl) méthyl) 2-propyl 1H-imidazole 5-acétamide

F = 213-215°C. rf = 0,25 (CH₂Cl₂-MeOH 9-1).

### EXEMPLE 42 : Acide 2-butyl 4-(méthylthio) 1-(2'(((phénylacétyl) amino) sulfonyl) (1,1'-biphenyl) 4-yl) méthyl) 1H-imidazole 5-carboxylique

F ≈ 175°C.

### EXEMPLE 43 : Acide 2-butyl α-hydroxy α-méthyl 4-(méthylthio) 1-((2'-(((phénylacétyl) amino) sulfonyl (1,1'-biphenyl) 4-yl) méthyl) 5-imidazolacétique

rf = 0,4 (AcOEt-EtOH-H₂O 70-20-10).

### EXEMPLE 44 : Acide 2-éthyl 4-(méthylthio) 1-((2'-(((((phénylméthyl) amino) carbonyl) amino) sulfonyl) (1,1'-biphényl) 4-yl) méthyl) 1H-imidazole 5-carboxylique

F = 164°C. rf # 0,6 (AcOEt-EtOH-H₂O 70-20-10).

### EXEMPLE 45 : Acide 1-((2'-(((((cyclohexylméthyl) amino) carbonyl) amino) sulfonyl) (1,1'-biphényl 4-yl) méthyl) 4-(difluorométhyl) thio) 2-propyl 1H-imidazole 5-carboxylique

F = 125°C. rf = 0,1 (CH₂Cl₂-MeOH 95-5).

### EXEMPLE 46 : Acide 4-((difluorométhyl) thio) 1-((2'(((((phénylméthyl) amino) carbonyl) amino) sulfonyl) (1,1'biphényl) 4-yl) méthyl) 2-propyl 1H-imidazole 5-carboxylique

F = 150°C. rf = 0,1 (CH₂Cl₂-MeOH 95-5).

### EXEMPLE 47 : Acide 4-((difluorométhyl) thio) 2-propyl 1-(2'(((((2-thièn-2-yl-éthyl) amino) carbonyl) amino) sulfonyl) (1,1'-biphényl) 4-yl) méthyl) 1H-imidazole 5-carboxylique

F = 130°C. rf = 0,1 (CH₂Cl₂-MeOH 95-5).

### EXEMPLE 48 : Acide 4-((difluorométhyl) thio) 1-(2'-(((((2-phényléthyl) amino) carbonyl) amino) sulfonyl) (1,1'-biphényl) 4-yl) méthyl) 2-propyl 1H-imidazole 5-carboxylique

F = 118°C. rf = 0,1 (CH₂Cl₂-MeOH 95-5).

### EXEMPLE 49 : Acide 1-((2'-(((((2-chlorophénylméthyl) amino) carbonyl) amino) sulfonyl) (1,1'-biphenyl) 4-yl) méthyl) 4-((difluorométhyl) thio) 2-propyl 1H-imidazole 5-carboxylique

F = 134°C. rf = 0,1 (CH₂Cl₂-MeOH 95-5).

### EXEMPLE 50 : Acide 4-((difluorométhyl) thio) 2-propyl 1-((2'(((((2-thiénylméthyl) amino) carbonyl) amino) sulfonyl) (1,1'-biphenyl) 4-yl) méthyl) 1H-imidazole 5-carboxylique

F = 142°C. rf = 0,1 (CH₂Cl₂-MeOH 95-5).

### EXEMPLE 51 : Acide 1-((2'-((((((1,3-benzodioxol 5-yl) méthyl) amino) carbonyl) amino) sulfonyl) (1,1'-biphényl 4-yl) méthyl) 4-((difluorométhyl) thio) 2-propyl 1H-imidazole 5-carboxylique

F = 124°C. rf = 0,1 (CH₂Cl₂-MeOH 95-5).

### EXEMPLE 52 : Acide 4-((difluorométhyl) thio) 1-((2'-(((((1-naphtylméthyl) amino) carbonyl) amino) sulfonyl) (1,1'-biphényl) 4-yl) méthyl) 2-propyl 1H-imidazole 5-carboxylique

F = 144°C. rf = 0,1 (CH₂Cl₂-MeOH 95-5).

### EXEMPLE 53 : Acide 4-((difluorométhyl) thio) 1-((2'-(((((3-phénylpropyl) amino) carbonyl) amino) sulfonyl) (1,1'-biphényl) 4-yl) méthyl) 2-propyl 1H-imidazole 5-carboxylique

F = 108°C. rf = 0,1 (CH₂Cl₂-MeOH 95-5).

### EXEMPLE 54 : Acide 1-((2'-((((2-cyclopentyléthyl) carbonyl) amino) sulfonyl) (1,1'-biphényl 4-yl) méthyl) 4-((difluorométhyl) thio) 2-propyl 1H-imidazole 5-carboxylique

F = 118°C. rf = 0,2 (CH₂Cl₂-MeOH 95-5).

### EXEMPLE 55 : Acide 4-((difluorométhyl) thio) 1-((2'-(((((4fluorophénylméthyl) amino) carbonyl) amino) sulfonyl) (1,1'biphényl) 4-yl) méthyl) 2-propyl 1H-imidazole 5-carboxylique

F = 122°C. rf = 0,1 (CH₂Cl₂-MeOH 95-5).

### EXEMPLE 56 : Acide 1-((2'-(((((2-cyclohexen-1-yl-éthyl)

**amino) carbonyl) amino) sulfonyl) (1,1'-biphényl) 4-yl) méthyl) 4-((difluorométhyl) thio) 2-propyl 1H-imidazole 5-carboxylique**

F = 114°C. rf = 0,1 (CH₂Cl₂-MeOH 95-5).

### EXEMPLE 57 : Acide 4-(méthylthio) 1-((2'-(((((1-phényléthyl) amino) carbonyl) amino) sulfonyl) (1,1'-biphényl) 4-yl) méthyl) 2-propyl 1H-imidazole 5-carboxylique

F = 139°C. rf = 0,15 (CH₂Cl₂-MeOH 95-5).

### EXEMPLE 58 : Acide 1-((2'-((((cyclohexylamino) carbonyl) amino) sulfonyl) (1,1'-biphényl 4-yl) méthyl) 4-(méthylthio) 2-propyl 1H-imidazole 5-carboxylique

F = 150°C. rf = 0,1 (CH₂Cl₂-MeOH 95-5).

### EXEMPLE 59 : Acide 2-butyl 4-chloro 1-((2'-(((((phénylméthyl) amino) carbonyl) amino) sulfonyl) (1,1'-biphenyl 4-yl) méthyl) 1H-imidazole 5-carboxylique

F ≈ 135°C.

### EXEMPLE 60 : Acide 2-butyl 4-((difluorométhyl) thio) 1-((2'(((((phénylméthyl) amino) carbonyl) amino) sulfonyl) (1,1'biphényl) 4-yl) méthyl) 1H-imidazole 5-carboxylique

rf = 0,4 (CH₂Cl₂-MeOH 90-10).

### EXEMPLE 61 : Acide 2-butyl 1-((2'(((((cyclohexylméthyl) amino) carbonyl) amino) sulfonyl) (1,1'-biphényl) 4-yl) méthyl) 4-((difluorométhyl) thio) 1H-imidazole 5-carboxylique

F = 130°C.

### EXEMPLE 62 : Acide 4-(méthylthio) 1-((2'-((((phénylamino) carbonyl) amino) sulfonyl) (1,1'-biphenyl) 4-yl) méthyl) 2-propyl 1H-imidazole 5-carboxylique

rf = 0,55 (AcOEt-EtOH-H₂O 70-20-10).

### EXEMPLE 63 : Acide 1-((2'-(((((4-méthylphényl) amino) carbonyl) amino) sulfonyl) (1,1'-biphényl) 4-yl) méthyl) 4-(méthylthio) 2-propyl 1H-imidazole 5-carboxylique

rf = 0,70 (AcOEt-EtOH-H₂O 70-20-10).

### EXEMPLE 64 : Acide 2-éthyl α-hydroxy 4-(méthylthio) α-phényl 1-((2'-(((((phénylméthyl) amino) carbonyl) amino) sulfonyl) (1,1'-biphényl) 4-yl) méthyl) 1H-imidazole 5-acétique

F ≈ 200°C (décomp.). rf # 0,25 (CH₂Cl₂-MeOH 90-10).

### EXEMPLE 65 : Acide 2-butyl β-hydroxy 4-(méthylthio) 1-((2'(((((phénylméthyl) amino) carbonyl) amino) sulfonyl) (1,1'biphényl) 4-yl) méthyl) 1H-imidazole 5-propionique

F ≈ 190°C. rf # 0,1 (CH₂Cl₂-MeOH 90-10).

### EXEMPLE 66 : Acide 1-((2'-(((butoxycarbonyl) amino) sulfonyl) (1,1'-biphényl) 4-yl) méthyl) 4-(méthylthio) 2-propyl 1H-imidazole 5-carboxylique

F = 202-204°C. rf = 0,30 (CHCl₃-MeOH 90-10).

### EXEMPLE 67 : Acide 4-(méthylthio) 2-propyl 1-((2'-(((((2-thiénylméthyl) amino) carbonyl) amino) sulfonyl) (1,1'-biphényl) 4-yl) méthyl) 1H-imidazole 5-carboxylique

F ≈ 140°C. rf = 0,1 (CH₂Cl₂-MeOH 95-5).

### EXEMPLE 68 : Acide 1-((2'-(((((cyclopentylméthyl) amino) carbonyl) amino) sulfonyl) (1,1'-biphényl) 4-yl) méthyl) 4-(méthylthio) 2-propyl 1H-imidazole 5-carboxylique

F ≈ 190°C. rf = 0,4 (CH₂Cl₂-MeOH 90-10).

En opérant de manière identique à l'exemple 28, on a préparé :

### EXEMPLE 69 : 1-((2'-(((((cyclohexylméthyl) amino) carbonyl) amino) sulfonyl) (1,1'-biphényl 4-yl) méthyl) 2-éthyl 4-(méthylthio) 1H-imidazole 5-carboxylate de potassium (sel de potassium)

F > 260°C. rf # 0,33 (CH₂Cl₂-MeOH 90-10).

En opérant comme à l'exemple 5 en utilisant au départ l'acide ou l'ester et l'isocyanate appropriés, on a préparé :

### EXEMPLE 70 : 4'-((5-formyl 4-(méthylthio) 2-propyl 1H-imidazol-1-yl) méthyl) N-(((phénylméthyl) amino) carbonyl) (1,1'biphényl) 2-sulfonamide

F = 180°C. rf = 0,3 (CH₂Cl₂-AcOEt 70-30).

### EXEMPLE 71 : Acide 2-butyl 1-((2'-(((((cyclohexylméthyl) amino) carbonyl) amino) sulfonyl) (1,1'-biphenyl) 4-yl) méthyl) α-hydroxy 4-(méthylthio) α-(trifluorométhyl) 1H-imidazole 5-acétique

rf = 0,45 (AcOEt-EtOH-H₂O 70-20-10).

### EXEMPLE 72 : Acide 2-butyl α-hydroxy 4-(méthylthio) 1-((2'(((((phénylméthyl) amino) carbonyl) amino) sulfonyl) (1,1'biphényl) 4-yl) méthyl) α-(trifluorométhyl) 1H-imidazole 5-acétique

rf = 0,60 (AcOEt-EtOH-H₂O 70-20-10).

### EXEMPLE 73 : Acide 2-butyl 4-(méthylthio) 1-((2'-((((phényl-amino) carbonyl) amino) sulfonyl) (1,1'-biphényl) 4-yl) méthyl) 1H-imidazole 5-carboxylique

F ≈ 165°C. rf # 0,65 (AcOEt-EtOH-H₂O 70-20-10).

### EXEMPLE 74 : Acide 2-butyl α-hydroxy 5-(méthylthio) 1-((2'(((((phénylméthyl) amino) carbonyl) amino) sulfonyl) (1,1'biphényl) 4-yl) méthyl) α-(trifluorométhyl) 1H-imidazole 4-acétique

rf = 0,25 (AcOEt-EtOH-H₂O 70-20-10).

### EXEMPLE 75 : Acide 2-butyl 1-((2'-(((((cyclohexylméthyl) amino) carbonyl) amino) sulfonyl) (1,1'-biphényl) 4-yl) méthyl) 4-(méthylthio) 1H-imidazole 5-carboxylique

F ≈ 175°C. rf # 0,6 (CH₂Cl₂-MeOH 80-20).

### EXEMPLE 76 : 1-((2'-(((((cyclohexylméthyl) amino) carbonyl) amino) sulfonyl) (1,1'-biphényl) 4-yl) méthyl) 4-(méthylthio) 2-propyl 1H-imidazole 5-carboxylate d'éthyle

F ≈ 104°C. rf = 0,30 (CHCl₃-MeOH 80-20).

En opérant comme à l'exemple 26 en utilisant au départ l'ester et le chloroformiate appropriés, on a préparé :

### EXEMPPLE 77 : 1-((2'-(((butoxycarbonyl) amino) sulfonyl) (1,1'-biphényl) 4-yl) méthyl) 4-(méthylthio) 2-propyl 1H-imidazole 5-carboxylate d'éthyle

rf = 0,40 (AcOEt-Hexane 60-40).

En opérant comme à l'exemple 20 stade B au départ du dérivé approprié, on a préparé :

### EXEMPLE 78 : 4'-((2-butyl 5-(α-hydroxy (1H-tétrazolyl) méthyl) 4-(méthylthio) 1H-imidazol-1-yl) méthyl) N-(((phénylméthyl) amino) carbonyl) (1,1'-biphenyl) 2-sulfonamide, dérivé sodé

F = 180°C. rf = 0,2 (CH₂Cl₂-MeOH 80-20).

### EXEMPLE 79 : 3-(2-butyl 1-((2'-((((propylamino) carbonyl) amino) sulfonyl) (1,1'-biphényl) 4-yl) méthyl) 4-(méthylthio) 1H-imidazol-5-yl) 3-hydroxy propanoate d'éthyle F = 135-136°C.

En opérant comme à l'exemple 31 stade B ou à l'exemple 34 en utilisant le magnésien et l'ester appropriés, on a préparé :

### EXEMPLE 80 : α-éthyl α-hydroxy 4-(méthylthio) 1-((2'(((((phénylméthyl) amino) carbonyl) amino) sulfonyl) (1,1'biphényl) 4-yl) méthyl) 2-propyl 1H-imidazole 5-acétate de sodium (sel de sodium)

F = 215°C. rf = 0,35 (CH₂Cl₂-MeOH 90-10).

### EXEMPLE 81 : α-hydroxy α-hexyl 4-(méthylthio) 1-((2'(((((phénylméthyl) amino) carbonyl) amino) sulfonyl) (1,1'biphényl) 4-yl) méthyl) 1H-imidazole 5-acétate de sodium (sel de sodium)

F ≈ 190°C. rf = 0,35 (CH₂Cl₂-MeOH 90-10).

### EXEMPLE 82 : α-hydroxy 4-(méthylthio) α-(phénylméthyl) 1-((2'-(((((phénylméthyl) amino) carbonyl) amino) sulfonyl) (1,1'-biphenyl) 4-yl) méthyl) 2-propyl 1H-imidazole 5-acétate de sodium (sel de sodium)

F = 220°C. rf = 0,3 (CH₂Cl₂-MeOH 90-10).

### EXEMPLE 83 : Acide α-hydroxy α-(1-méthyléthyl) 4-(méthylthio) 1-((2'-(((((phénylméthyl) amino) carbonyl) amino) sulfonyl) (1,1'-biphenyl) 4-yl) méthyl) 2-propyl 1H-imidazole 5-acétique

F = 180°C. rf = 0,36 (CH₂Cl₂-MeOH 90-10).

### EXEMPLE 84 : Acide α-éthényl α-hydroxy 4-(méthylthio) 1-((2'(((((phénylméthyl) amino) carbonyl) amino) sulfonyl) (1,1'biphenyl) 4-yl) méthyl) 2-propyl 1H-imidazole 5-acétique

F = 225°C. rf = 0,15 (CH₂Cl₂-MeOH 90-10).

En chauffant à 100°C le carbamate approprié avec de la cyclopentylméthylamine dans du toluène, on a préparé :

### EXEMPLE 85 : 1-((2'-(((((cyclopentylméthyl) amino) carbonyl) amino) sulfonyl) (1,1'-biphényl) 4-yl) méthyl) 4-(méthylthio) 2-propyl 1H-imidazole 5-carboxylate d'éthyle

F ≈ 163°C. rf = 0,5 (CH₂Cl₂-AcOEt 80-20).

En hydrogénant 2 heures à 50°C sous une pression de 2 bars le dérivé approprié dans du méthanol en présence d'un catalyseur, on a préparé :

### EXEMPLE 86 : Acide 2-butyl α-méthyl 4-(méthylthio) 1-((2'(((((phénylméthyl) amino) carbonyl) amino) sulfonyl) (1,1'biphényl) 4-yl) méthyl) 1H-imidazole 5-acétique

rf = 0,30 (CHCl₃-MeOH 90-10).

### EXEMPLE 87 de composition pharmaceutique.

On a préparé des comprimés répondant à la formule suivante :

| | |
|---|---|
| Produit de l'exemple 15 | 50 mg |
| Excipient pour un comprimé terminé à | 200 mg |
| (détail de l'excipient : lactose, talc, amidon, stéarate de magnésium). | |

### RESULTATS PHARMACOLOGIOUES :

### 1 - Test sur le récepteur AT₁ de l'angiotensine II

On utilise une préparation membranaire fraîche obtenue à partir de foie de rat. Le tissu est broyé au polytron dans un tampon Tris 50 mM pH 7,4, le broyage est suivi de 3 centrifugations à 30 000 g 15' avec reprises intermédiaires des culots dans le tampon Tris pH 7,4.

Les derniers culots sont remis en suspension dans un tampon d'incubation (Tris 20 mM, NaCl 135 mM, KCl 10 mM, glucose 5 mM, MgCl₂ 135 mM, PMSF 0,3 mM, bacitracine 0,1 mM, lysozyme 0,1 %).

On répartit des fractions aliquotées de 1 ml dans des tubes en verre et ajoute de la ¹²⁵ I angiotensine II (25 000 DPM/tube) et le produit à étudier. (Le produit est d'abord testé à 3 x 10⁻⁵M en triple). Lorsque le produit testé déplace de plus de 50 % la radioactivité liée spécifiquement au récepteur, il est testé à nouveau selon une gamme de 7 concentrations afin de déterminer la concentration qui inhibe de 50 % la radioactivité liée spécifiquement au récepteur. On détermine ainsi la concentration inhibitrice 50 %).

La liaison non spécifique est déterminée par addition du produit de l'exemple 94 du brevet européen 0253310, à 10⁻⁵M (en triple). On incube à 25°C pendant 150 minutes, remet au bain-marie à 0°C, 5 minutes, filtre sous vide, rince au tampon Tris pH 7,4 et compte la radioactivité en présence de scintillant solide.

Le résultat est exprimé directement en concentration inhibitrice 50 % (CI₅₀), c'est-à-dire en concentration de produit étudié, exprimée en nM, nécessaire pour déplacer 50 % de la radioactivité spécifique fixée sur le récepteur étudié.

| Résultats : | |
|---|---|
| Produit de l'exemple | Récepteur AT₁ CI₅₀ en nanomoles |
| 7 | 0,7 |
| 9 | 3,4 |
| 11 | 24 |
| 13 | 6,3 |
| 21 | 2,5 |
| 23 | 0,2 |
| 25 | 0,2 |
| 32 | 0,5 |
| 34 | 0,7 |
| 44 | 0,06 |
| 45 | 0,07 |
| 46 | 0,11 |
| 47 | 0,03 |
| 50 | 0,06 |
| 59 | 0,08 |
| 75 | 0,05 |

### 2) Test sur le récepteur AT₂ de l'angiotensine II

On utilise une préparation membranaire fraîche obtenue à partir d'utérus de lapine, prétraitées 4 jours auparavent par 50 µg d'estradiol administré par voie percutanée. Le tissu est broyé au polytron dans un tampon Tris 50 mM, pH 7,4 et le broyage est suivi de 3 centrifugations à 30 000 g 15 minutes, avec reprises intermédiaires des culots dans le tampon Tris pH 7,4.

Les derniers culots sont remis en suspension dans un tampon d'incubation (Tris 20 mM, NaCl 135 mM, KCl 10 mM, glucose 5 mM, MgCl₂, 6H₂O 10 mM, PMSF 0,3 mM, bacitracine 0,1 mM, lysozyme 0,1 %, pH 7,4).

L'homogénat obtenu est mis à préincuber 20 minutes à 25°C en présence de dithiothreitol 10 mM, puis ramené à 0°-4°C.

On répartit des fractions aliquotées de 1 ml dans des tubes en verre et ajoute de la ¹²⁵I angiotensine II (25 000 DPM/tube) et le produit à étudier. Le produit est d'abord testé à 3.10⁻⁵M en triple. Lorsque le produit testé déplace de plus de 50 % la radioactivité liée spécifiquement au récepteur, il est testé à nouveau selon une gamme de 7 concentrations afin de déterminer la concentration qui inhibe de 50 % la radioactivité liée spécifiquement au récepteur. On détermine ainsi la concentration inhibitrice 50 %.

La liaison spécifique est déterminée par addition de EXP 655 (= PD 123-177 de Warner-Lamber) à 10⁻⁵M en triple. On incube à 25°C pendant 150 minutes, remet au bain-marie à 0°C, 5 minutes, filtre sous vide, rince au tampon Tris pH 7,4 et compte la radioactivité en présence de scintillant solide.

Le résultat est exprimé directement en concentration inhibitrice 50 % (CI₅₀), c'est-à-dire en concentration de produit étudié, exprimée en nM, nécessaire pour déplacer 50 % de la radioactivité spécifique fixée sur le récepteur étudié.

| Résultats : | |
|---|---|
| Produit de l'exemple | Récepteur AT₂ CI₅₀ en nanomoles |
| 7 | 5,2 |
| 9 | 8,5 |
| 11 | 1,6 |
| 13 | 1,9 |
| 21 | 6,5 |
| 23 | 2,0 |
| 25 | 5,9 |
| 32 | 0,8 |
| 34 | 2,1 |
| 45 | 1,7 |
| 46 | 0,26 |
| 50 | 7,4 |
| 54 | 2,1 |
| 56 | 5,8 |
| 63 | 3,5 |
| 69 | 1,3 |

On obtient aussi notamment pour les produits P1 à P13 cités ci-dessus les résultats suivants :

| PRODUIT | Sur récepteur AT₁ CI₅₀ | Sur récepteur AT₂ CI₅₀ |
|---|---|---|
| P1 | 0,23 | 17 |
| P2 | 0,10 | 14 |
| P3 | 1,1 | 11 |
| P4 | 0,5 | 47 |
| P5 | 0,3 | 25 |
| P6 | 0,27 | 10 |
| P7 | 0,4 | 49 |
| P8 | 0,14 | 17 |
| P9 | 0,19 | 53 |
| P10 | 0,7 | 31 |
| P11 | 0,14 | 24 |
| P12 | 0,8 | 54 |
| P13 | 5,9 | 63 |

### 3 - Test d'activité antagoniste de l'angiotensine II chez le rat démédullé

Des rats mâles Sprague-Dawley (250 à 350 g) sont anesthésiés par une injection intra-péritonéale de pentobarbital sodique (60 mg/kg). La pression artérielle diastolique est enregistrée grâce à un cathéter (PE50) hépariné introduit dans la carotide gauche de l'animal, et relié à un calculateur de pression (Gould, Pressure Processor) par l'intermé- diaire d'un capteur de pression Gould.

Un cathéter est introduit dans la jugulaire droite de l'animal afin de permettre l'injection des molécules à étudier.

L'animal est placé sous respiration assistée. Une section bilatérale des nerfs vagues est effectuée. Le rat est alors démédullé.

Après une période de stabilisation suffisante, l'étude de l'antagonisme des molécules vis-à-vis de l'angiotensine II (Hypertensine, CIBA) est abordée de la façon suivante :
1 - Trois injections consécutives d'angiotensine II (0,75 microgrammes/kg) espacées de 15 minutes permettent d'obtenir une réponse pressive reproductible et stable.
2 - Tout en gardant une périodicité de 15 minutes pour l'administration d'angiotensine II, les molécules (0,01 à 10 mg/kg) sont injectées 5 minutes avant l'angiotensine II.
   Les effets presseurs de l'angiotensine II en présence de l'antagoniste sont exprimés en pourcentage des effets presseurs de l'angiotensine II administrée seule. La dose inhibitrice à 50 % (DI₅₀) de l'effet étudié est ainsi déterminée.
   Chaque animal est considéré comme son propre témoin.

| Résultats : | | |
|---|---|---|
| Produit de l'exemple | DI₅₀ en mg/kg | |
| | IV | PO |
| 7 | 0,6 | - |
| 23 | 0,3 | 1,54 |
| 25 | 0,22 | 1,45 |
| 30 | 0,93 | - |
| 32 | 0,46 | - |
| 35 | 0,05 | - |
| 45 | 0,22 | 1,1 |
| 46 | 0,26 | 2 |
| 47 | 0,33 | 3 |
| 48 | 0,64 | - |
| 50 | 0,13 | - |
| 62 | 0,52 | - |
| 65 | 0,05 | - |
| 75 | 0,35 | - |

## Revendications

1. Utilisation des produits de formule (I) : 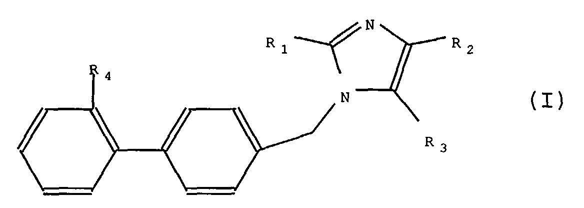 dans laquelle
R₁ représente un radical alkyle, linéaire ou ramifié renfermant au plus 4 atomes de carbone,
R₂ représente un radical alkylthio, linéaire ou ramifié renfermant au plus 4 atomes de carbone, éventuellement substitué par un ou plusieurs atomes de fluor,
R₃ représente
- un radical carboxy libre, salifié par une base minérale ou organique ou estérifié par un radical alkyle renfermant au plus 4 atomes de carbone,
- un radical acyle renfermant au plus 6 atomes de carbone, carbamoyle,
- un radical alkyle et alkényle renfermant au plus 6 atomes de carbone substitués par un ou plusieurs radicaux choisis parmi les atomes d'halogène, les radicaux hydroxyle, alcoxy linéaire ou ramifié renfermant au plus 4 atomes de carbone, carboxy, libre, salifié par une base minérale ou organique ou estérifié par un radical alkyle renfermant au plus 4 atomes de carbone, phényle et carbamoyle, éventuellement substitué par un radical phényle ou benzyle,
R₄ représente le radical -SO₂-NH₂, -SO₂-N=CH-N(CH₃)₂ 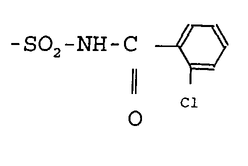 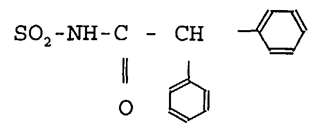
-SO₂-NH-CO₂Et, -SO₂-NH-CO₂Pr, -SO₂-NH-CO₂Bu, -SO₂-NH-CO₂H, 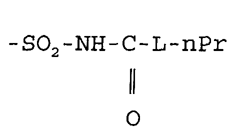 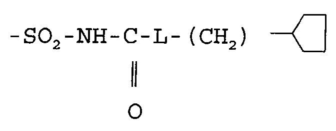 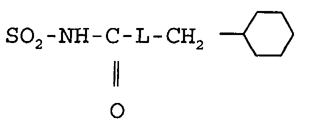 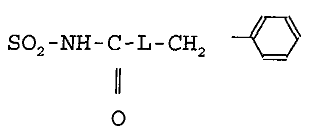 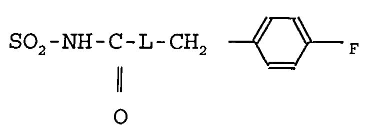 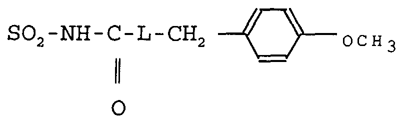 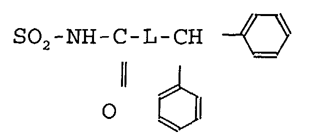 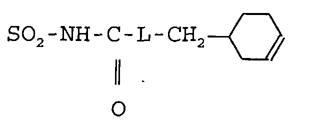 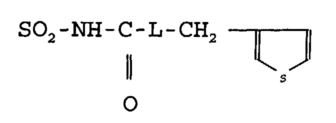 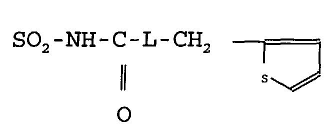
avec L représente -O- ou -NH- ,
étant entendu que les atomes de soufre dans les produits de formule (I) peuvent éventuellement être oxydés sous forme de sulfone ou de sulfoxyde,
répondant aux formules suivantes :
- 4'-((5-acétyl 2-butyl 4-(méthylthio) 1H-imidazole 1-yl) méthyl) N-((propylamino) carbonyl) (1,1'-biphényl) 2-sulfonamide,
- 2-butyl 1-[(2'-(((((diphénylméthyl) amino) carbonyl) amino) sulfonyl) (1,1'-biphényl) 4-yl) méthyl] 4-(méthylthio) 1H-imidazole 5-carboxylate de potassium (disel de potassium),
- Acide 2-butyl 1-[(2'-(((diphénylacétyl) amino) sulfonyl) (1,1'-biphényl) 4-yl) méthyl] 4-(méthylthio) 1H-imidazole 5-carboxylique,
- Acide 4-bromo 1-((2'-(((((phénylméthyl) amino) carbonyl) amino) sulfonyl) (1,1'-biphényl) 4-yl) méthyl) 2-propyl 1H-imidazole 5-carboxylique,
- Acide 4-(butylthio) 1-((2'-(((((phénylméthyl) amino) carbonyl) amino) sulfonyl) (1,1'-biphényl) 4-yl) méthyl) 2-propyl 1H-imidazole 5-carboxylique,
- Acide 2-butyl 4-(méthylthio) 1-(2'-(((phénylacétyl) amino) sulfonyl) (1,1'-biphényl) 4-yl) méthyl) 1H-imidazole 5-carboxylique,
- Acide 2-éthyl 4-(méthylthio) 1-((2'-(((((phénylméthyl) amino) carbonyl) amino) sulfonyl) (1,1'-biphényl) 4-yl) méthyl) 1H-imidazole 5-carboxylique,
- 4'-((5-formyl 4-(méthylthio) 2-propyl 1H-imidazol-1-yl) méthyl) N-(((phénylméthyl) amino) carbonyl) (1,1'-biphényl) 2-sulfonamide,
- α-hydroxy α-hexyl 4-(méthylthio) 1-((2'-(((((phénylméthyl) amino) carbonyl) amino) sulfonyl) (1,1'-biphényl) 4-yl) méthyl) 1H-imidazole 5-acétate de sodium (sel de sodium), lesdits produits de formule (I) étant sous toutes les formes isomères racémiques, énantiomères et diastéréoisomères possibles ainsi que les sels d'addition avec les acides minéraux et organiques ou avec les bases minérales et organiques desdits produits de formule (I) pour la préparation de compositions pharmaceutiques destinées au traitement d'affections résultant d'une stimulation anormale au récepteur AT₁ de l'Angiotensine II.

2. Utilisation des produits tels que définis à la revendication 1, pour la préparation de compositions pharmaceutiques destinées au traitement de l'hypertension artérielle, de l'insuffisance cardiaque et des resténoses post-angioplastie.

3. Utilisation des produits tels que définis à la revendication 1, pour la préparation de compositions pharmaceutiques destinées au traitement de l'insuffisance rénale.

4. Utilisation des produits tels que définis à la revendication 1, pour la préparation de compositions pharmaceutiques destinées au traitement et à la prévention des désordres cardiovasculaires associés au diabète.

5. Les produits répondant aux formules suivantes :
- 4'-((5-acétyl 2-butyl 4-(méthylthio) 1H-imidazole 1-yl) méthyl) N-((propylamino) carbonyl) (1,1'-biphényl) 2-sulfonamide,
- 2-butyl 1-[(2'-(((((diphénylméthyl) amino) carbonyl) amino) sulfonyl) (1,1'-biphényl) 4-yl) méthyl] 4-(méthylthio) 1H-imidazole 5-carboxylate de potassium (disel de potassium),
- Acide 2-butyl 1-[(2'-(((diphénylacétyl) amino) sulfonyl) (1,1'-biphényl) 4-yl) méthyl] 4-(méthylthio) 1H-imidazole 5-carboxylique,
- Acide 4-bromo 1-((2'-(((((phénylméthyl) amino) carbonyl) amino) sulfonyl) (1,1'-biphényl) 4-yl) méthyl) 2-propyl 1H-imidazole 5-carboxylique,
- Acide 4-(butylthio) 1-((2'-(((((phénylméthyl) amino) carbonyl) amino) sulfonyl) (1,1'-biphényl) 4-yl) méthyl) 2-propyl 1H-imidazole 5-carboxylique,
- Acide 2-butyl 4-(méthylthio) 1-(2'-(((phénylacétyl) amino) sulfonyl) (1,1'-biphényl) 4-yl) méthyl) 1H-imidazole 5-carboxylique,
- Acide 2-éthyl 4-(méthylthio) 1-((2'-(((((phénylméthyl) amino) carbonyl) amino) sulfonyl) (1,1'-biphényl) 4-yl) méthyl) 1H-imidazole 5-carboxylique,
- 4'-((5-formyl 4-(méthylthio) 2-propyl 1H-imidazol-1-yl) méthyl) N-(((phénylméthyl) amino) carbonyl) (1,1'-biphényl) 2-sulfonamide,
- α-hydroxy α-hexyl 4-(méthylthio) 1-((2'-(((((phénylméthyl) amino) carbonyl) amino) sulfonyl) (1,1'-biphényl) 4-yl) méthyl) 1H-imidazole 5-acétate de sodium (sel de sodium),

6. Procédé de préparation des produits tels que définis à la revendication 1, **caractérisé en ce que** :
soit l'on soumet un composé de formule (II): 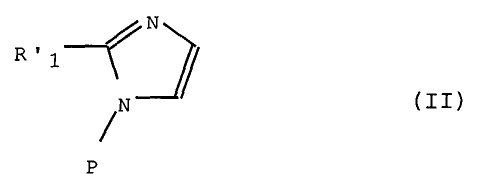 dans laquelle R₁ a la signification indiquée à la revendication 1 et P représente un groupement protecteur de l'atome d'azote,
à une réaction d'halogénation pour obtenir un composé de formule (III) : 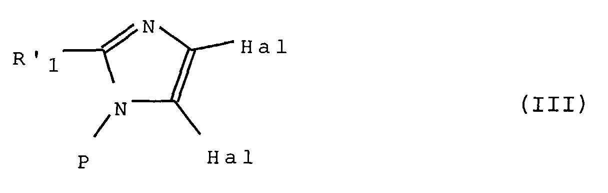 dans laquelle R'₁ et P ont les significations indiquées ci-dessus et Hal représente un atome d'halogène, que l'on soumet à une réaction d'échange halogène-métal sur l'un des atomes d'halogène puis à une réaction avec un composé de formule (IV_{c}) ou (IV_{d}) : 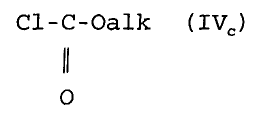 ou 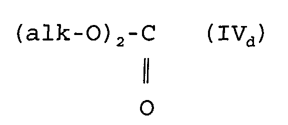 dans lesquelles alk représente un radical alkyle renfermant au plus 4 atomes de carbone,
pour obtenir le composé de formule (V) : 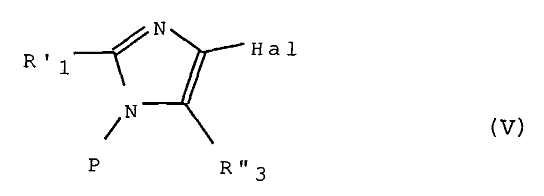 dans laquelle R'₁, P et Hal ont les significations indiquées ci-dessus et R"₃ représente K-O-alk tels que définis ci-dessus avec K représente le radical 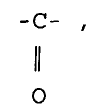 composé de formule (V) que l'on peut soumettre à une réaction d'échange halogène-métal sur l'atome d'halogène puis à une réaction avec un composé de formule (Ivₐ) ou (IV_{b}) :
(-S-R')₂ (IVa)
ou
MeSO₂SR' (IVb)
dans lesquelles R' a la signification de R, dans lesquelles les éventuelles fonctions réactives sont éventuellement protégées par des groupements protecteurs R représentant un radical alkyle renfermant au plus 4 atomes de carbone éventuellement substitué par un ou plusieurs atomes de fluor, pour obtenir le composé de formule (VII) : 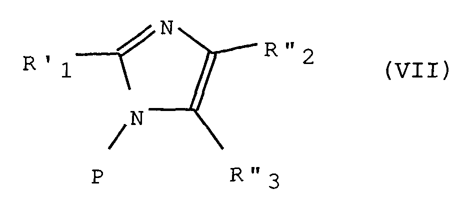 dans laquelle R'₁ et P ont les significations indiquées ci-dessus, R"₂ représente -S-R' et R"₃ représente -K-Oalk tels que définis ci-dessus dans lesquelles R', alk et K ont les significations indiquées ci-dessus,
produit de formule (VII) dont on libère la fonction amine bloquée par P tel que défini ci-dessus, puis fait réagir avec un composé de formule (VIII) : 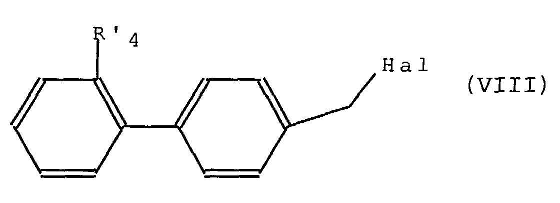 dans laquelle R'₄ a la signification indiquée ci-dessus pour R₄, dans laquelle les éventuelles fonctions réactives sont éventuellement protégées par des groupements protecteurs et Hal représente un atome d'halogène pour obtenir un produit de formule (I₁) : 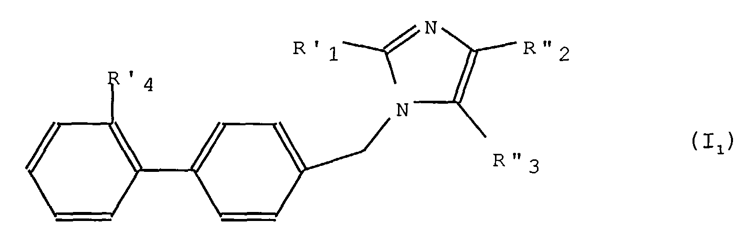 dans laquelle R'₁, R"₂, R"₃ et R'₄ ont les significations indiquées ci-dessus,
soit l'on soumet un composé de formule (IX) : 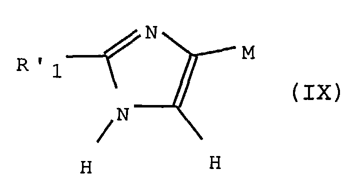 dans laquelle R'₁ a la signification indiquée ci-dessus et M représente un atome d'hydrogène ou le radical R'₂ qui a la signification indiquée ci-dessus pour R₂, dans laquelle les éventuelles fonctions réactives sont éventuellement protégées par des groupements protecteurs,
à une réaction avec le composé de formule (VIII) telle que définie ci-dessus, pour obtenir un produit de formule (X) : 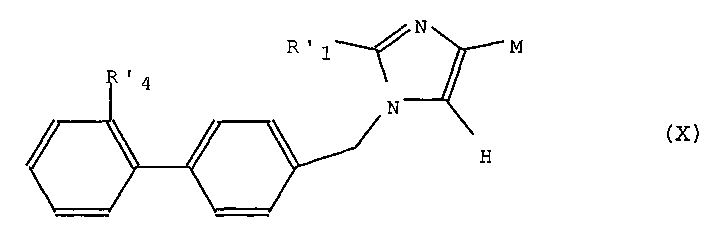 dans laquelle R'₁, M et R'₄ ont les significations indiquées ci-dessus,
produit de formule (X) que lorsque M représente un atome d'hydrogène, l'on peut soumettre à une réaction d'halogé- nation pour obtenir le produit de formule (XIV) : 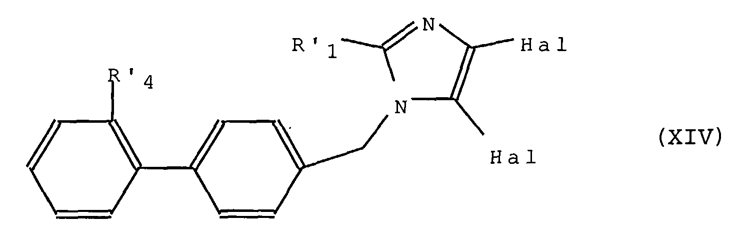 dans laquelle R'₁, R'₄ et Hal ont les significations indiquées ci-dessus, que l'on peut soumettre à une réaction d'échange halogène métal puis à l'action d'un composé de formule (IV_{c}), (IV_{d}) ou (IVₑ) telle que définie ci-dessus pour obtenir le produit de formule (XXI) : 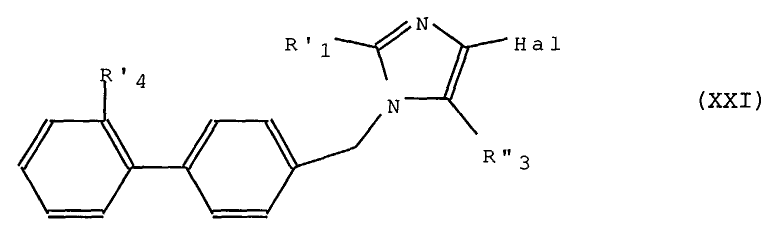 dans laquelle R'₁, R'₄, Hal et R"₃ ont les significations indiquées ci-dessus, produit de formule (XXI) que l'on peut soumettre à une réaction d'échange halogène métal puis à l'action d'un composé de formule (IVₐ) ou (IV_{b}), telle que définie ci-dessus, pour obtenir un produit de formule (I₁) : 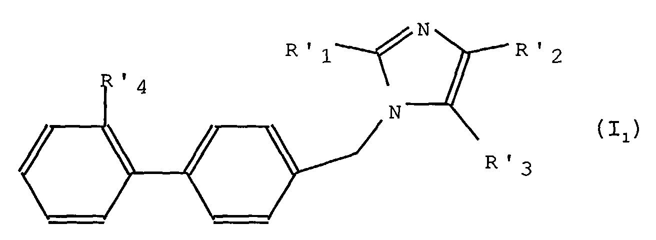 dans laquelle R'₁, R'₄, R"₂ et R"₃ ont les significations indiquées ci-dessus,
soit l'on soumet un composé de formule (XX) : 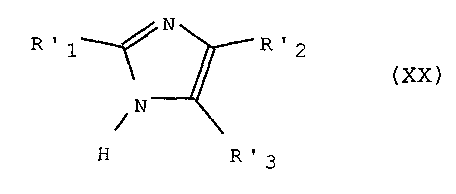 dans laquelle R'₁ et R'₂ ont les significations indiquées ci-dessus et R'₃ a la signification indiquée ci-dessus pour R₃ dans laquelle les éventuelles fonctions réactives sont éventuellement protégées par des groupements protecteurs, à une réaction avec le composé de formule (VIII) telle que définie ci-dessus, pour obtenir un produit de formule (I') : 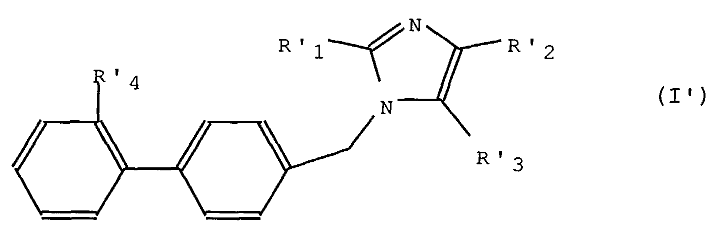 dans laquelle R'₁, R'₂, R'₃ et R'₄ ont les significations indiquées ci-dessus,
produits de formules (I₁)et (I')qui peuvent être des produits de formule (I) et que, pour obtenir des ou d'autres produits de formule (I), l'on peut soumettre, si désiré et si nécessaire, à l'une ou plusieurs des réactions de transformations suivantes, dans un ordre quelconque :
a) estérification de fonction acide,
b) saponification de fonction ester en fonction acide,
c) transformation de fonction ester en fonction acyle,
d) transformation de la fonction cyano en fonction acide,
e) transformation de fonction acide en fonction amide,
f) réduction de la fonction carboxy en fonction alcool,
g) transformation de fonction alcoxy en fonction hydroxyle, ou encore de fonction hydroxyle en fonction alcoxy,
h) oxydation de fonction alcool en fonction aldéhyde ou acide
i) transformation du radical formyle en radical carbamoyle,
j) transformation du radical carbamoyle en radical nitrile,
l) oxydation de groupement alkylthio en sulfoxyde ou sulfone correspondant,
n) transformation de fonction oxo en fonction thioxo,
o) déshydratation d'un radical hydroxylalkyle en radical alkényle,
r) transformation d'un carbamate en urée et notamment d'un sulfonylcarbamate en sulfonylurée,
s) élimination des groupements protecteurs que peuvent porter les fonctions réactives protégées,
t) salification par un acide minéral ou organique ou par une base pour obtenir le sel correspondant,
u) dédoublement des formes racémiques en produits dédoublés, lesdits produits tels que définis à la revendication 1 ainsi obtenus étant sous toutes les formes isomères possibles racémiques, énantiomères et diastéréoisomères.

7. A titre de médicaments, les produits tels que définis à de la revendication 5, ces produits étant sous toutes les formes isomères possibles racémiques, énantiomères et diastéréoisomères, ainsi que les sels d'addition avec les acides minéraux et organiques ou avec les bases minérales et organiques pharmaceutiquement acceptables de ces produits tels que définis à la revendication 1.

8. Les compositions pharmaceutiques contenant à titre de principe actif, l'un au moins des médicaments tels que définis à la revendication 7.
